# EUROPEAN PATENT APPLICATION

(11) **EP 4 265 601 A2**
(43) Date of publication of application: **25.10.2023**
(21) Application number: 21800324.2
(22) Date of filing: 06.07.2021
(51) Int. Cl.: C07D 293/12, A61K 31/41, A61K 31/4439, A61P 31/14

(54) **USE OF BENZISOSELAZOLE DERIVATIVE FOR ANTI-CORONAVIRUS AND CONTROL OF INTERSTITIAL LUNG DISEASE (ILD) RELATED TO CORONAVIRUS**

(30) Priority: 06.05.2020 CN 202010373848
(71) Applicant: Shanghai Yuanxi Medicine Corp., Shanghai 201207 (CN)
(72) Inventor: ZENG, Huihui, Shanghai 201207 (CN); YIN, Hanwei, Shanghai 201207 (CN)
(74) Representative: Gille Hrabal Partnerschaftsgesellschaft mbB Patentanwälte
(86) International application number: PCT/CN2021/104644
(87) International publication number: WO 2021/223780

(57) **Abstract**

The present invention provides the use of the benzisoselazole derivative of formula (I) for preparing an anti-coronavirus drug or a drug for treating diseases caused by coronaviruses. The benzisoselazole derivatives of the present invention can effectively inhibit the activity of 2019-nCoV 3CLpro proteolytic enzyme, thus inhibiting the activation of 2019-nCoV RNA polymerase, inhibiting virus replication, while also effectively treating interstitial lung disease (ILD) caused by coronavirus.

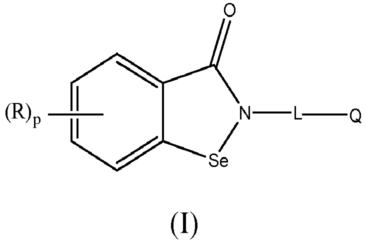

## Description

### TECHNICAL FIELD

The present disclosure belongs to the field of pharmaceuticals, and particularly relates to use of benzisoselenazole derivatives for the manufacturing of anti-coronavirus medicaments.

### BACKGROUND

Coronaviruses belong to the order Nidovirales, family Coronaviridae and genus Coronavirus according to systematic classification. Viruses of the genus Coronavirus are enveloped ones with a linear single-stranded positive-strand RNA genome. They are a large group of viruses widely occurring in nature.

The coronavirus was first isolated from a chicken in 1937. The virus particles are 60-200 nm in diameter, average 100 nm in diameter, are spherical or elliptical in shape, and exhibit polymorphism. The virus has an envelope with spikes on it. The whole virus is reminiscent of the solar coronas. The spikes of different coronaviruses are markedly different.

2019 novel coronavirus (2019-nCoV or SARS-CoV-2, causing coronavirus disease 2019 (COVID-19)) is the seventh known coronavirus to infect humans, after HCoV-229E, HCoV-OC43, HCoV-NL63, HCoV-HKU1, SARS-CoV (causing severe acute respiratory syndrome) and MERS-CoV (causing Middle East respiratory syndrome). 2019-nCoV belongs to the genus of Betacoronavirus. It has an envelope. The particles are circular or elliptical in shape, usually exhibit polymorphism, and are 60-140 nm in diameter. Its genetic characteristics are markedly different from those of SARS-CoV and MERS-CoV. The present research shows that it is more than 85% homologous with Bat SARS-like coronavirus (bat-SL-CoVZC45).

2019-nCoV is an RNA virus. Its proliferation depends on the regeneration of maternal RNA by RNA-dependent RNA polymerase (RdRp). The 2019-nCoV 3CLpro protein is a key protein for 2019-nCoV's key protein RdRp to be in activated form. The 2019-nCoV 3CLpro protein hydrolyzes the virus's pp1a and pp1ab proteins to form a mature product nsp1-16, which is further assembled into active RdRp. At present, 2019-nCoV 3CLpro has been considered as one of the drug targets for combating coronaviruses.

There is an urgent need now for active drugs that are effective against coronaviruses, particularly 2019-nCoV.

### SUMMARY

The present disclosure provides use of benzisoselenazole derivatives for the manufacturing of anti-coronavirus medicaments, wherein the benzisoselenazole derivatives are compounds of formula (I), or stereoisomers, pharmaceutically acceptable salts, solvates, prodrugs or active metabolites thereof, 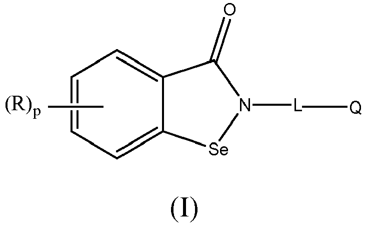 wherein R is selected from hydrogen, cyano, hydroxyl, sulfydryl, halogen, nitro, amino, COOH, SO₃H, C₁₋₁₂ alkyl, C₃₋₁₂ cycloalkyl, C₁₋₁₂ alkoxy, C₁₋₁₂ alkylthio, C₂₋₁₂ alkenyl, C₁₋₁₂ alkylamido, C₁₋₁₂ alkylacyloxy, 3- to 14-membered heterocyclyl, C₆₋₁₄ aryl and 5- to 14-membered heteroaryl, wherein the C₁₋₁₂ alkyl, C₃₋₁₂ cycloalkyl, C₁₋₁₂ alkoxy, C₁₋₁₂ alkylthio, C₁₋₁₂ alkylamido, C₁₋₁₂ alkylacyloxy, 3- to 14-membered heterocyclyl, C₆₋₁₄ aryl and 5- to 14-membered heteroaryl are optionally substituted with one or more substituents selected from the following: cyano, hydroxyl, sulfhydryl, halogen, amino, nitro, oxo, thio, COOH, SO₃H, C₁₋₁₂ alkyl, C₃₋₁₂ cycloalkyl, C₁₋₁₂ alkoxy, C₂₋₁₂ alkenyl, 3- to 14-membered heterocyclyl, C₆₋₁₄ aryl and 5- to 14-membered heteroaryl;
p is an integer from 0 to 4;
L is selected from ^{∗}-(CH₂)ₙ-#, ^{∗}-(CH₂)ₙ-NH-(CH₂)ₙ-#, ^{∗}-(CH₂)ₙ-NR^{N}-(CH₂)ₙ-#, ^{∗}-(CH₂)ₙ-CH(OH)-(CH₂)ₙ-#, ^{∗}-(CH₂)ₙ-NH-C(O)-(CH₂)ₙ-#, ^{∗}-(CH₂)ₙ-C(O)-NH-(CH₂)ₙ-#, ^{∗}-(CH₂)ₙ-O-(CH₂)ₙ-#, ^{∗}-(CH₂)ₙ-O-C(O)-(CH₂)ₙ-#, ^{∗}-(CH₂)ₙ-C(O)-O-(CH₂)ₙ-#, ^{∗}-(CH₂)ₙ-C(O)-(CH₂)ₙ-#, ^{∗}-(CH₂)ₙ-C(O)-C(O)-(CH₂)ₙ-#, ^{∗}-(CH₂)ₙ-S(O)-(CH₂)ₙ-#, ^{∗}-(CH₂)ₙ-S(O)₂-(CH₂)ₙ-#, ^{∗}-(CH₂)ₙ-C₃₋₈ cycloalkylene-(CH₂)ₘ-#, ^{∗}-(CH₂)ₙ-S-S-(CH₂)ₘ-#, ^{∗}-(CHR^{L})(CH₂)ₙ-S-S-(CH₂)ₘ(CHR^{L})-#, ^{∗}-(CH₂)ₙ-(CHR^{L})-S-S-(CHR^{L})-(CH₂)ₘ-#, phenylene, biphenylene, triphenylene, ^{∗}-phenyl-(CH₂)ₙ-#, ^{∗}-(CH₂)ₙ-phenyl-#, ^{∗}-phenyl-(CH₂)ₙ-phenyl-#, ^{∗}-phenyl-(CH₂)ₙ-S(O)₂-# and ^{∗}-(CH₂)ₙ-S(O)₂-phenyl-#;
wherein * represents a linking site for the N atom of the benzisoselenazole moiety, and # represents a linking site for the Q moiety;
n is an integer from 0 to 12, m is an integer from 0 to 12, R^{N} represents C₁₋₁₂ alkyl, and R^{L} represents halogen, hydroxyl, amino, carboxyl or sulfhydryl;
Q is selected from H, C₁₋₁₂ alkyl, C₃₋₁₂ cycloalkyl, C₁₋₁₂ alkoxy, C₁₋₁₂ alkylthio, C₁₋₁₂ alkylamido, C₁₋₁₂ alkylacyloxy, C₂₋₁₂ alkenyl, 3- to 14-membered heterocyclyl, C₆₋₁₄ aryl and 5- to 14-membered heteroaryl, wherein the C₁₋₁₂ alkyl, C₃₋₁₂ cycloalkyl, C₁₋₁₂ alkoxy, C₁₋₁₂ alkylamido, C₁₋₁₂ alkylacyloxy, 3- to 14-membered heterocyclyl, C₆₋₁₄ aryl and 5- to 14-membered heteroaryl are optionally substituted with one or more substituents selected from the following: cyano, hydroxyl, sulfhydryl, halogen, amino, nitro, oxo, thio, COOH, SO₃H, C₁₋₁₂ alkyl, C₃₋₁₂ cycloalkyl, C₁₋₁₂ alkoxy, C₂₋₁₂ alkenyl, 3- to 14-membered heterocyclyl, C₆₋₁₄ aryl and 5- to 14-membered heteroaryl.

According to an embodiment of the present disclosure, in the compounds of formula (I), R is selected from hydrogen, cyano, hydroxyl, sulfhydryl, halogen, nitro, amino, COOH, SO₃H, C₁₋₆ alkyl, C₃₋₇ cycloalkyl, C₁₋₆ alkoxy, C₁₋₆ alkylthio, C₁₋₆ alkylamido, C₁₋₆ alkylacyloxy, C₂₋₆ alkenyl, 3- to 10-membered heterocyclyl, C₆₋₁₀ aryl and 5- to 10-membered heteroaryl, wherein the C₁₋₆ alkyl, C₃₋₇ cycloalkyl, C₁₋₆ alkoxy, C₁₋₆ alkylthio, C₁₋₆ alkylamido, C₁₋₆ alkylacyloxy, C₂₋₆ alkenyl, 3- to 10-membered heterocyclyl, C₆₋₁₀ aryl and 5- to 10-membered heteroaryl are optionally substituted with one or more substituents selected from the following: cyano, hydroxyl, sulfhydryl, halogen, amino, nitro, oxo, thio, COOH, SO₃H, C₁₋₆ alkyl, C₃₋₇ cycloalkyl, C₁₋₆ alkoxy, C₂₋₆ alkenyl, 3-to 10-membered heterocyclyl, C₆₋₁₀ aryl and 5- to 10-membered heteroaryl;
p is an integer from 0 to 4;
L is selected from ^{∗}-(CH₂)ₙ-#, ^{∗}-(CH₂)ₙ-NH-(CH₂)ₙ-#, ^{∗}-(CH₂)ₙ-NR^{N}-(CH₂)ₙ-#, ^{∗}-(CH₂)ₙ-CH(OH)-(CH₂)ₙ-#, ^{∗}-(CH₂)ₙ-NH-C(O)-(CH₂)ₙ-#, ^{∗}-(CH₂)ₙ-C(O)-NH-(CH₂)ₙ-#, ^{∗}-(CH₂)ₙ-O-(CH₂)ₙ-#, ^{∗}-(CH₂)ₙ-O-C(O)-(CH₂)ₙ-#, ^{∗}-(CH₂)ₙ-C(O)-O-(CH₂)ₙ-#, ^{∗}-(CH₂)ₙ-C(O)-(CH₂)ₙ-#, ^{∗}-(CH₂)ₙ-C(O)-C(O)-(CH₂)ₙ-#, ^{∗}-(CH₂)ₙ-S(O)-(CH₂)ₙ-#, ^{∗}-(CH₂)ₙ-S(O)₂-(CH₂)ₙ-#, ^{∗}-(CH₂)ₙ-C₃₋₈ cycloalkylene-(CH₂)ₘ-#, ^{∗}-(CH₂)ₙ-S-S-(CH₂)ₘ-#, ^{∗}-(CHR^{L})(CH₂)ₙ-S-S-(CH₂)ₘ(CHR^{L})-#, ^{∗}-(CH₂)ₙ-(CHR^{L})-S-S-(CHR^{L})-(CH₂)ₘ-#, phenylene, biphenylene, triphenylene, ^{∗}-phenyl-(CH₂)ₙ-#, ^{∗}-(CH₂)ₙ-phenyl-# and ^{∗}-phenyl-(CH₂)ₙ- phenyl-#, ^{∗}-phenyl-(CH₂)ₙ-S(O)₂-# and ^{∗}-(CH₂)ₙ-S(O)₂-phenyl-#, wherein * represents a linking site for the N atom of the benzisoselenazole moiety, and # represents a linking site for the Q moiety; n is an integer from 0 to 12, m is an integer from 0 to 12, R^{N} represents C₁₋₆ alkyl, and R^{L} represents halogen, hydroxyl, amino, carboxyl or sulfhydryl;
Q is selected from H, C₁₋₆ alkyl, C₃₋₇ cycloalkyl, C₁₋₆ alkoxy, C₁₋₆ alkylthio, C₁₋₆ alkylamido, C₁₋₆ alkylacyloxy, C₂₋₆ alkenyl, 3- to 10-membered heterocyclyl, C₆₋₁₀ aryl and 5- to 10-membered heteroaryl, wherein the C₁₋₆ alkyl, C₃₋₇ cycloalkyl, C₁₋₆ alkoxy, C₁₋₆ alkylamido, C₁₋₆ alkylacyloxy, C₂₋₆ alkenyl, 3- to 10-membered heterocyclyl, C₆₋₁₀ aryl and 5- to 10-membered heteroaryl are optionally substituted with one or more substituents selected from the following: cyano, hydroxyl, sulfhydryl, halogen, amino, nitro, oxo, thio, COOH, SO₃H, C₁₋₆ alkyl, C₃₋₇ cycloalkyl, C₁₋₆ alkoxy, C₂₋₆ alkenyl, 3- to 10-membered heterocyclyl, C₆₋₁₀ aryl and 5- to 10-membered heteroaryl.

According to an embodiment of the present disclosure, in the compounds of formula (I), L is selected from ^{∗}-(CH₂)₂-#, ^{∗}-(CH₂)₃-#, ^{∗}-(CH₂)₄-#, ^{∗}-(CH₂)₅-#, ^{∗}-(CH₂)₆-#, ^{∗}-(CH₂)₇-#, ^{∗}-(CH₂)₈-#, ^{∗}-(CH₂)₉-#, ^{∗}-(CH₂)₁₀-#, ^{∗}-(CH₂)₁₁-#, ^{∗}-(CH₂)₁₂-#, cyclopentylene, cyclohexylene, phenylene, biphenylene, triphenylene, ^{∗}-phenyl-(CH₂)ₙ-#, ^{∗}-(CH₂)ₙ-phenyl-#, ^{∗}-phenyl-(CH₂)ₙ-phenyl-#^{∗}, ^{∗}-phenyl-S(O)₂-#, ^{∗}-(CH₂)ₙ-S(O)₂-phenyl-#, ^{∗}-(CH₂)ₙ-NH-(CH₂)ₙ-#, ^{∗}-(CH₂)ₙ-NR^{N}-(CH₂)ₙ-#, ^{∗}-(CH₂)ₙ-CH(OH)-(CH₂)ₙ-#, ^{∗}-(CH₂)ₙ-NH-C(O)-(CH₂)ₙ-#, ^{∗}-(CH₂)ₙ-C(O)-NH-(CH₂)ₙ-#, ^{∗}-(CH₂)ₙ-O-(CH₂)ₙ-#, ^{∗}-(CH₂)ₙ-O-C(O)-(CH₂)ₙ-#, ^{∗}-(CH₂)ₙ-C(O)-O-(CH₂)ₙ-#, ^{∗}-(CH₂)ₙ-C(O)-(CH₂)ₙ-#, ^{∗}-(CH₂)ₙ-C(O)-C(O)-(CH₂)ₙ-#, ^{∗}-(CH₂)ₙ-S(O)-(CH₂)ₙ-#, ^{∗}-(CH₂)ₙ-S(O)₂-(CH₂)ₙ-#, ^{∗}-(CH₂)ₙ-cyclopropylene-(CH₂)ₘ-#, ^{∗}-(CH₂)ₙ-cyclobutylidene-(CH₂)ₘ-#, ^{∗}-(CH₂)ₙ-cyclopentylene-(CH₂)ₘ-#, ^{∗}-(CH₂)ₙ-cyclohexylene-(CH₂)ₘ-#, ^{∗}-(CH₂)ₙ-S-S-(CH₂)ₘ-#, ^{∗}-(CHR^{L})(CH₂)ₙ-S-S-(CH₂)ₘ(CHR^{L})-# and ^{∗}-(CH₂)ₙ-(CHR^{L})-S-S-(CHR^{L})-(CH₂)ₘ-#, wherein * represents a linking site for the N atom of the benzisoselenazole moiety, and # represents a linking site for the Q moiety; n is 0, 1, 2, 3, 4, 5 or 6, m is 0, 1, 2, 3, 4, 5 or 6, R^{N} represents C₁₋₆ alkyl, and R^{L} represents halogen, hydroxyl, amino, carboxyl or sulfhydryl. According to an embodiment of the present disclosure, in the compounds of formula (I), Q is selected from H, C₁₋₆ alkyl, C₃₋₇ cycloalkyl, C₁₋₆ alkoxy, C₁₋₆ alkylthio, C₁₋₆ alkylamido, C₁₋₆ alkylacyloxy, C₂₋₆ alkenyl, benzisoselenazolyl, saccharide residue, amino acid residue, R^{e}-NH-R^{f}, 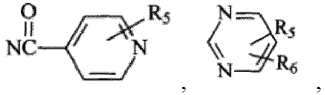 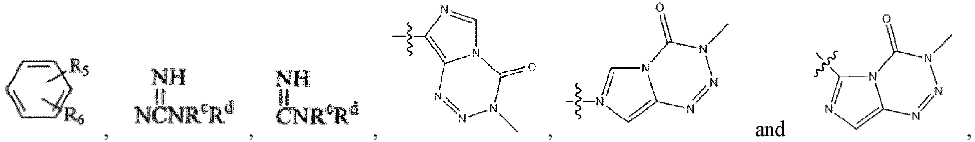 wherein 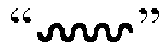 represents a linking group for L;
R₅ and R₆ are each independently selected from hydrogen, halogen, cyano, C₁₋₆ alkyl, C₁₋₆ alkoxy, C₁₋₆ alkylthio, N(C₁₋₆ alkyl)₂, NH(C₁₋₆ alkyl), COOR^{b} and SO₃R^{b};
R^{b} is selected from hydrogen, C₁₋₆ alkyl and C₁₋₆ alkoxy;
R^{c} and R^{d} are each independently selected from hydrogen, C₁₋₆ alkyl and phenyl, or R^{c} and R^{d} together with a nitrogen atom to which they are linked form a nitrogen-containing heterocycle or nitrogen-containing heteroaryl ring;
R^{e} and R^{f} are each independently selected from C₁₋₆ alkyl and C₃₋₇ cycloalkyl;
the C₁₋₆ alkyl, C₃₋₇ cycloalkyl, C₁₋₆ alkoxy, C₁₋₆ alkylthio, C₁₋₆ alkylamido, C₁₋₆ alkylacyloxy, C₂₋₆ alkenyl, C₃₋₇ cycloalkyl, benzisoselenazolyl, saccharide residue and amino acid residue may optionally be substituted with one or more substituents selected from the following: cyano, hydroxyl, sulfhydryl, halogen, amino, nitro, oxo, thio, C₁₋₆ alkyl, C₃₋₇ cycloalkyl and C₁₋₆ alkoxy.

According to an embodiment of the present disclosure, the saccharide residue described above may be a *D*-glucopyranosyl group, such as 1,3,4,6-tetra-*O*-acetyl-2-deoxy-*D*-glucopyranosyl.

According to an embodiment of the present disclosure, the amino acid residue described above is substituted with sulfhydryl, and further linked to the L moiety by the sulfhydryl.

According to an embodiment of the present disclosure, in the compounds of formula (I), R is selected from hydrogen, hydroxyl, halogen, COOH, SO₃H, C₁₋₆ alkyl and C₁₋₆ alkoxy.

According to an embodiment of the present disclosure, in the compounds of formula (I), R is selected from hydrogen, hydroxyl, fluorine, chlorine, bromine, iodine, COOH, SO₃H, methyl, fluoromethyl, difluoromethyl, trifluoromethyl, ethyl, *n*-propyl, isopropyl, *n*-butyl, isobutyl, *tert*-butyl, *n*-pentyl, isopentyl, methoxy, ethoxy, *n*-propoxy*,* isopropoxy, *n-butoxy,* isobutoxy, *tert*-butoxy, *n*-pentoxy and isopentoxy.

According to an embodiment of the present disclosure, the benzisoselenazole derivatives of formula (I) are compounds of the following formula (I-1): 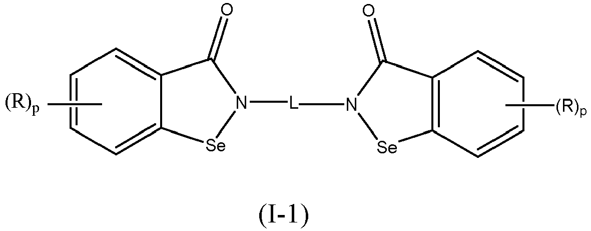 wherein R, L and p each independently have the definitions given above in the compounds of formula (I). According to the present disclosure, the benzisoselenazole derivatives of formula (I-1) are selected from the compounds in Table 1 below:

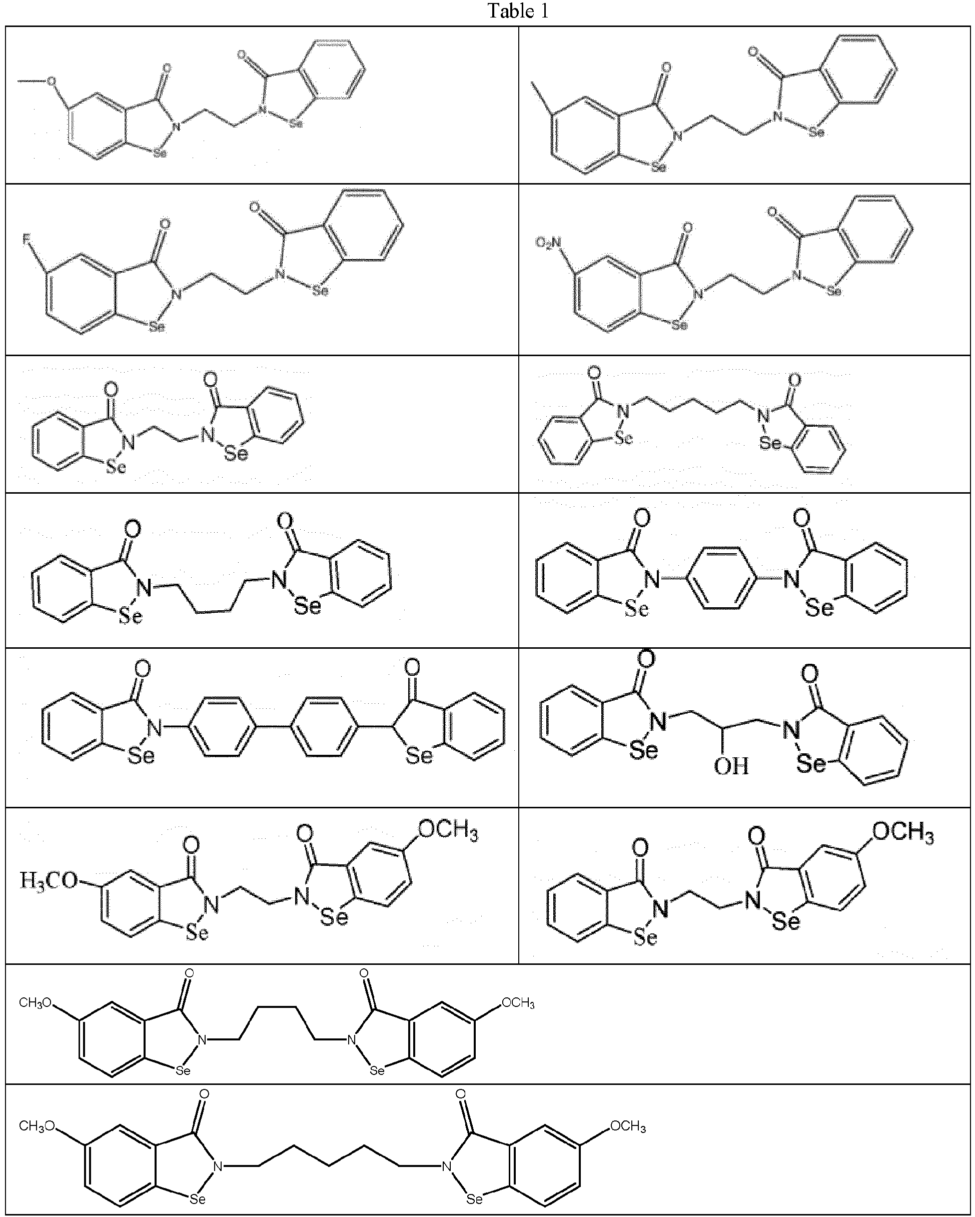

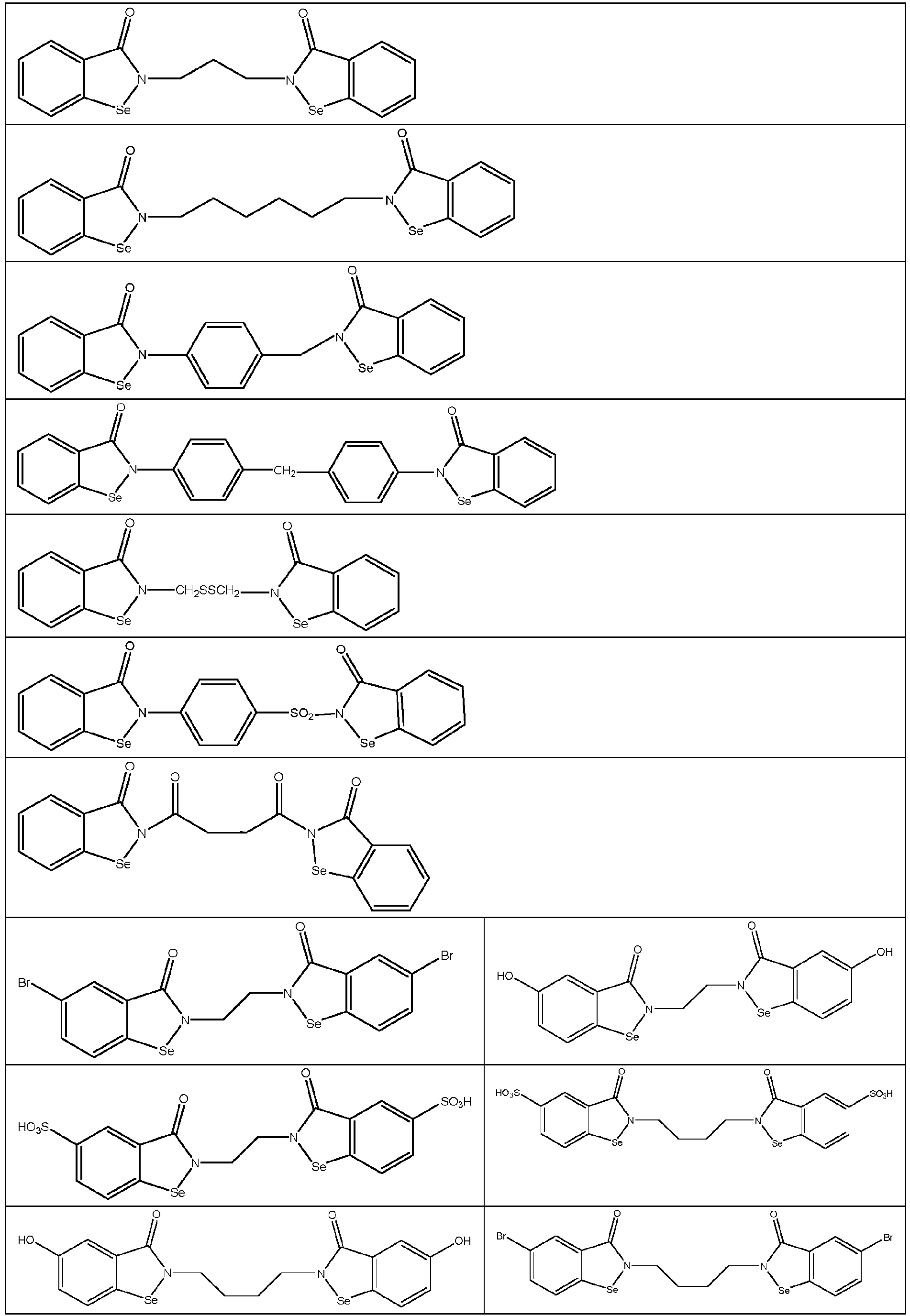

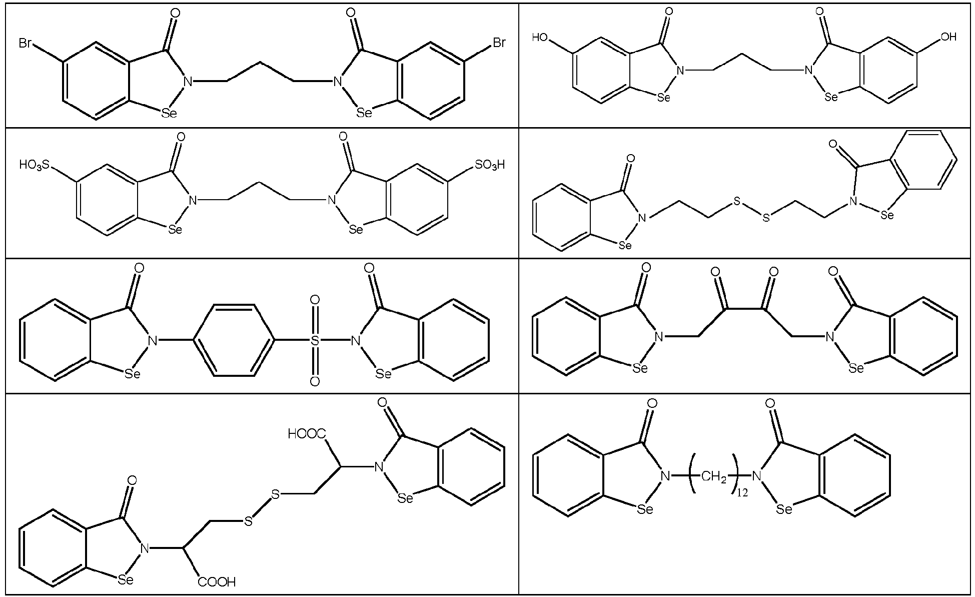

In an embodiment of the present disclosure, the benzisoselenazole derivatives of formula (I) are compounds of the following formula (I-2a), formula (I-2b) or formula (I-2c):

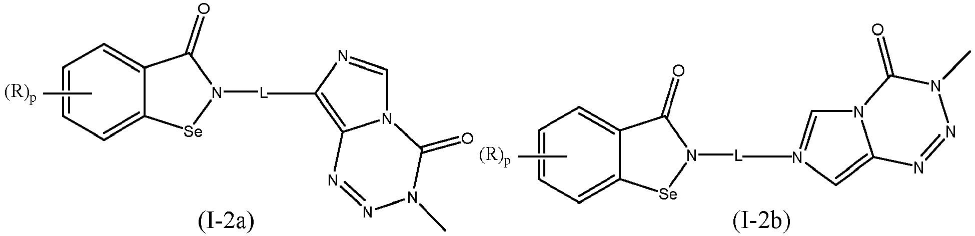

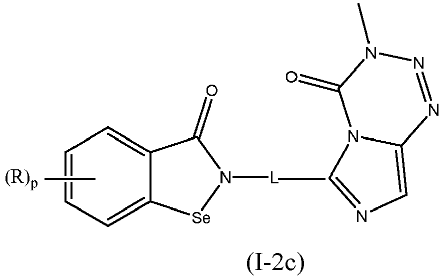

In the compounds of formula (I-2a), formula (I-2b) and formula (I-2c) described above, R, L and p each have the definitions given above in the compounds of formula (I).

According to the present disclosure, the benzisoselenazole derivatives of formula (I-2a), formula (I-2b) or formula (I-2c) are selected from the compounds in Table 2 below:

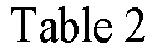

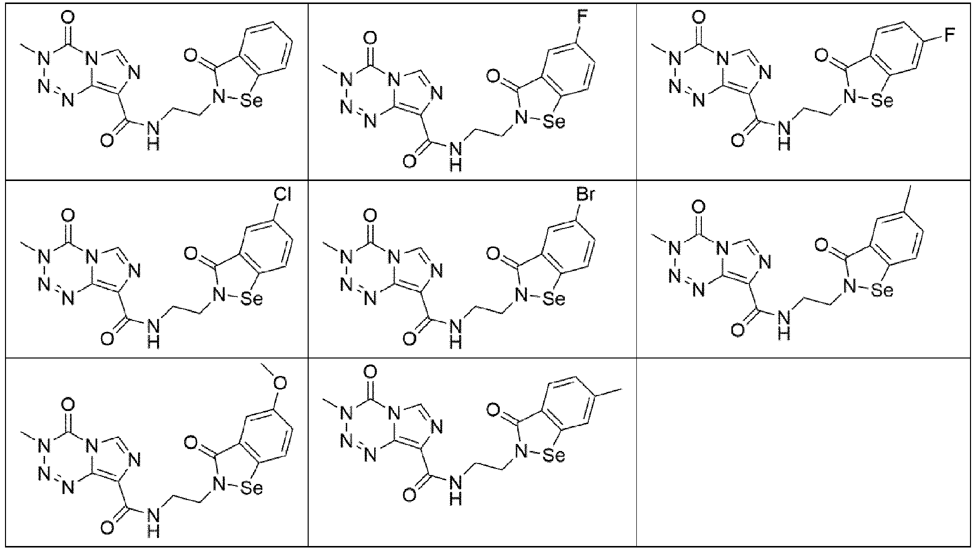

In an embodiment of the present disclosure, the benzisoselenazole derivatives of formula (I) are compounds of the following formula (I-3): 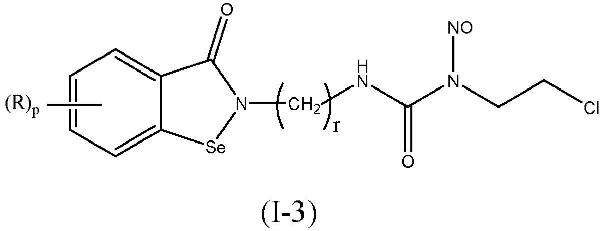
wherein R and p each have the definitions given above in the compounds of formula (I);
r is an integer from 0 to 12, preferably an integer from 2 to 4.

According to the present disclosure, the benzisoselenazole derivatives of formula (I-3) are selected from the compounds in Table 3 below:

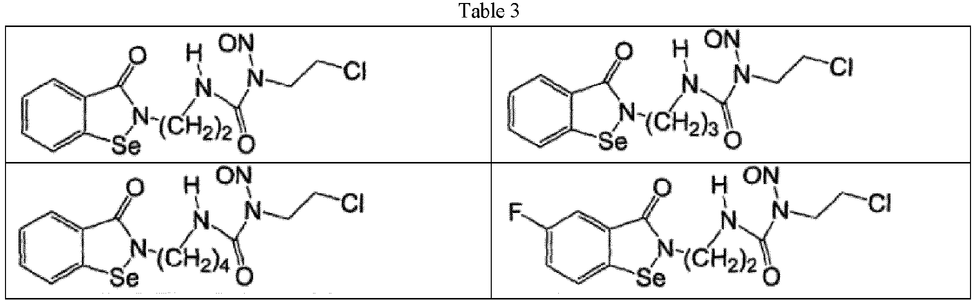

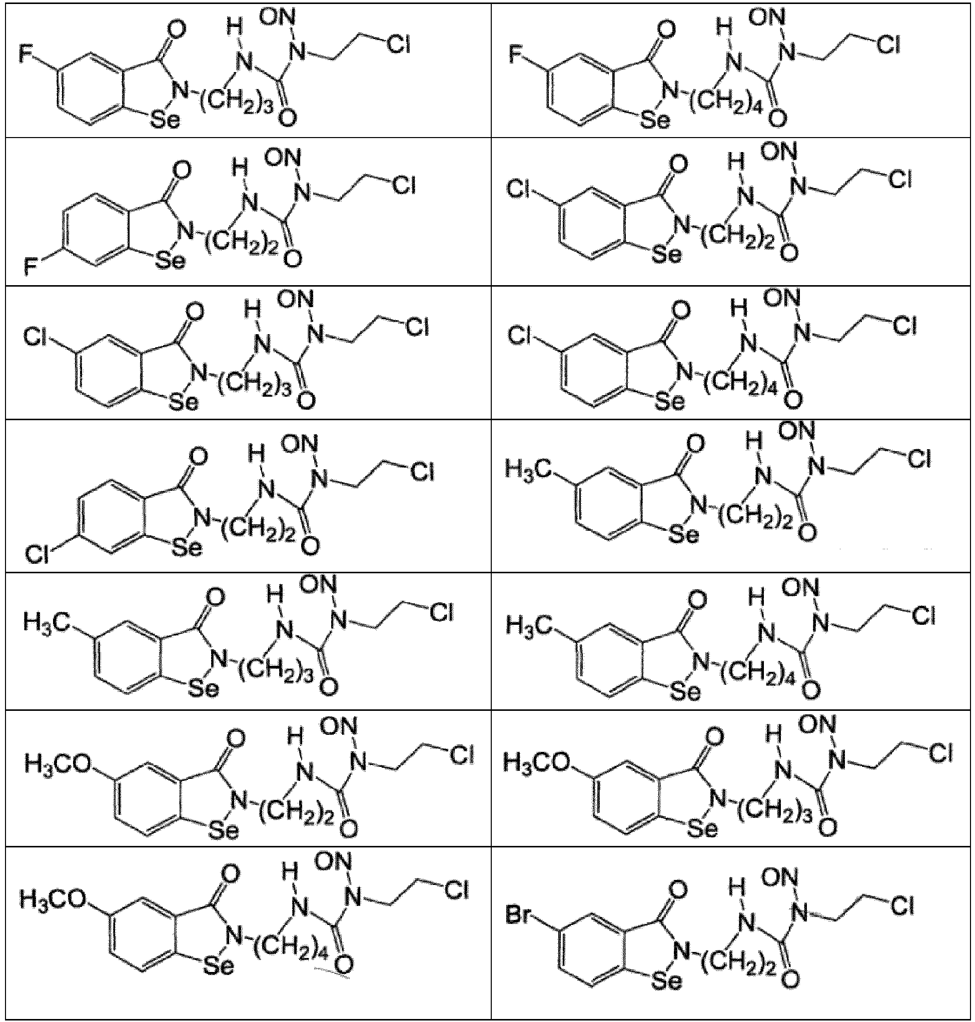

In an embodiment of the present disclosure, the benzisoselenazole derivatives of formula (I) are compounds of the following formula (I-4): 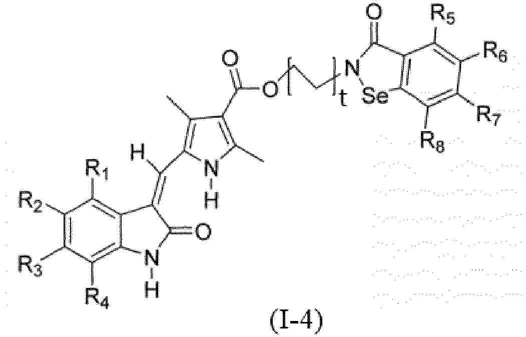 wherein R₁, R₂, R₃, R₄, R₅, R₆, R₇ and R₈ are each independently H, C₁₋₆ alkyl, C₁₋₆ alkoxy or halogen; t is an integer from 1 to 4, preferably 2 or 3.

According to the present disclosure, the benzisoselenazole derivatives of formula (I-4) are selected from the compounds in Table 4 below:

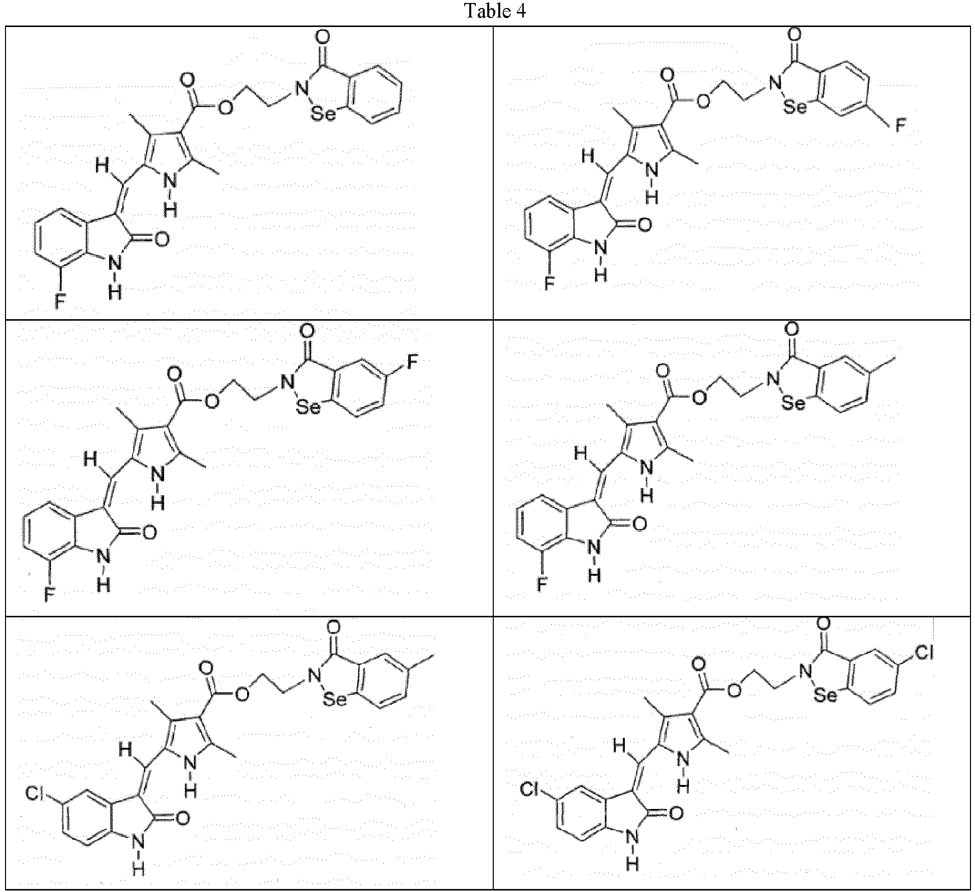

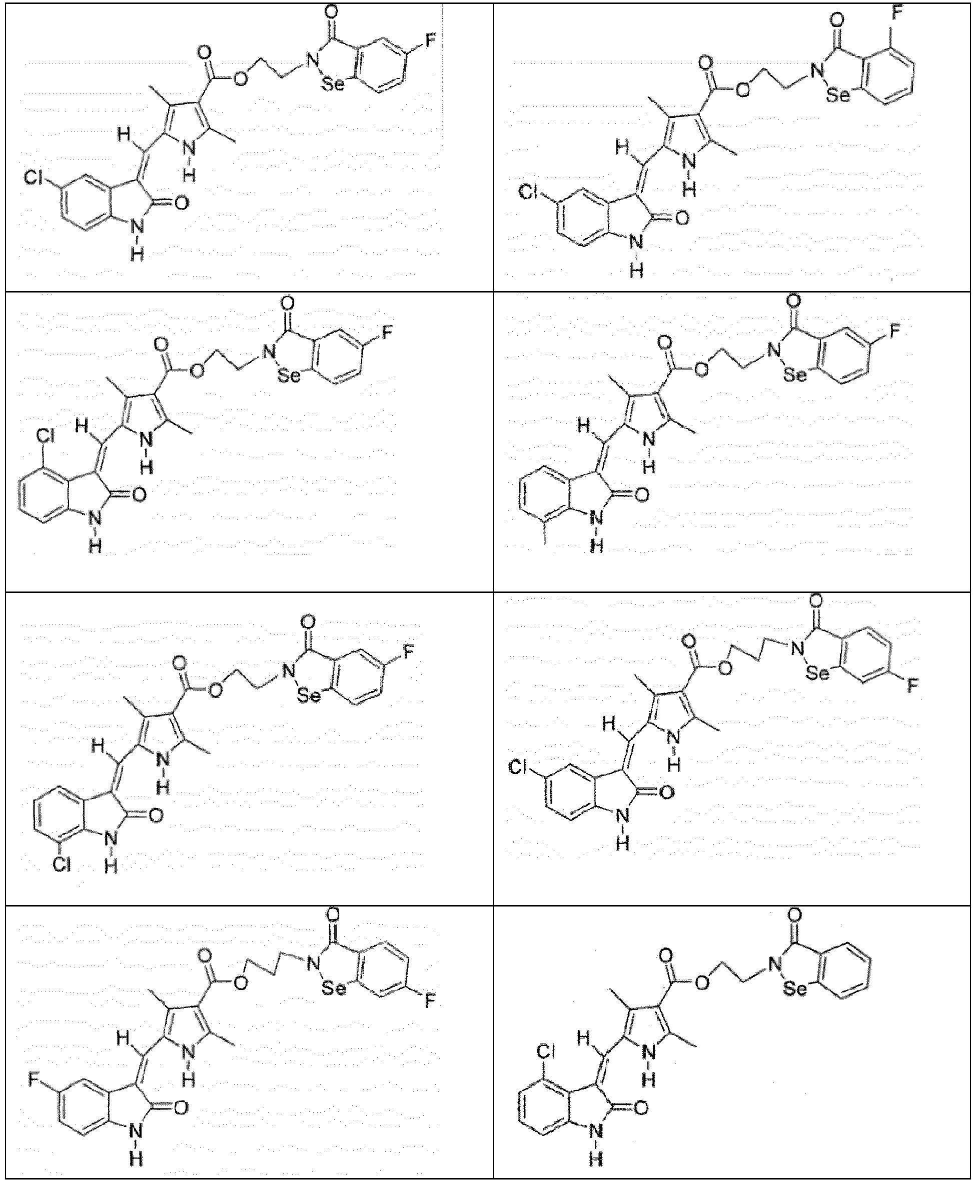

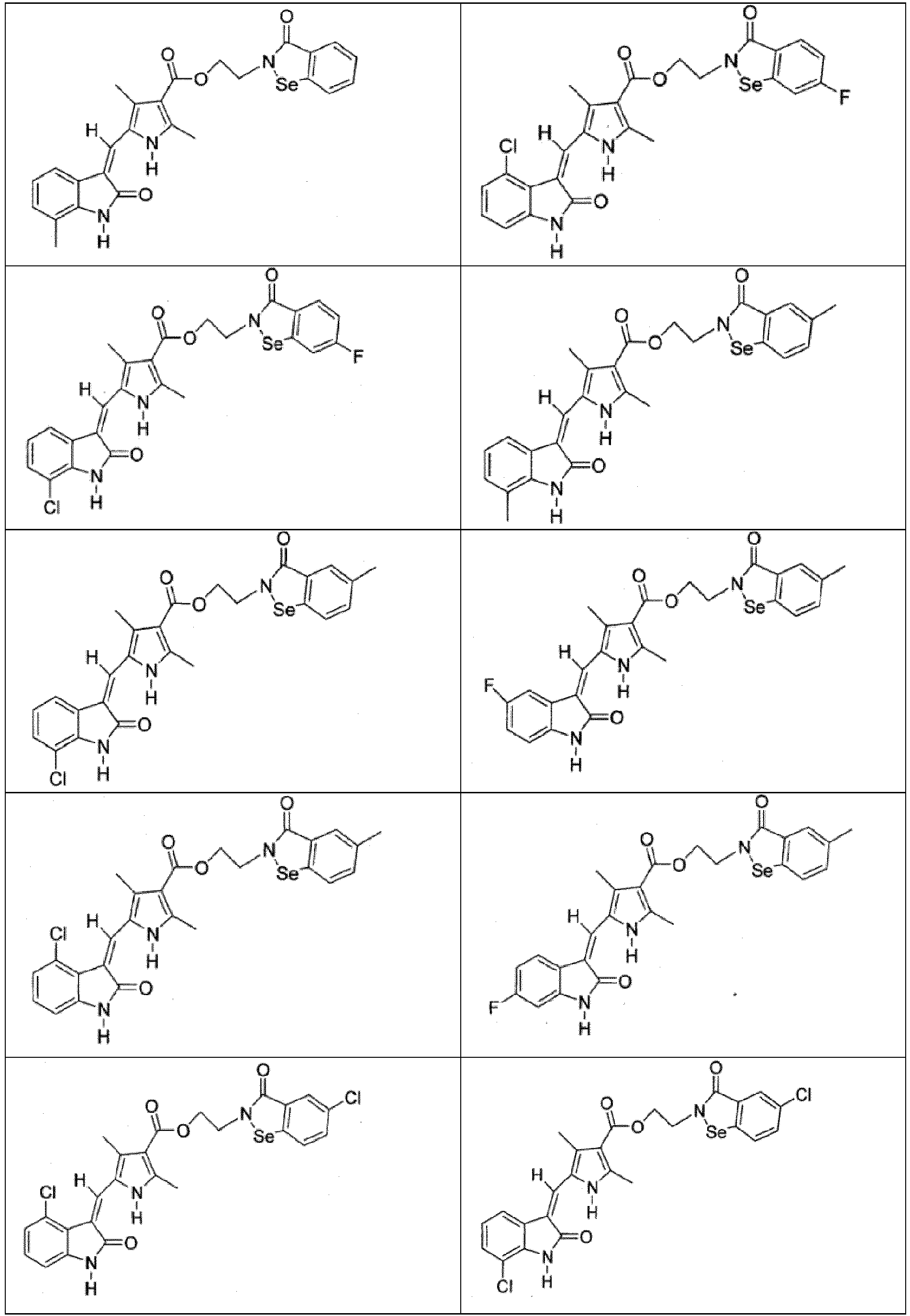

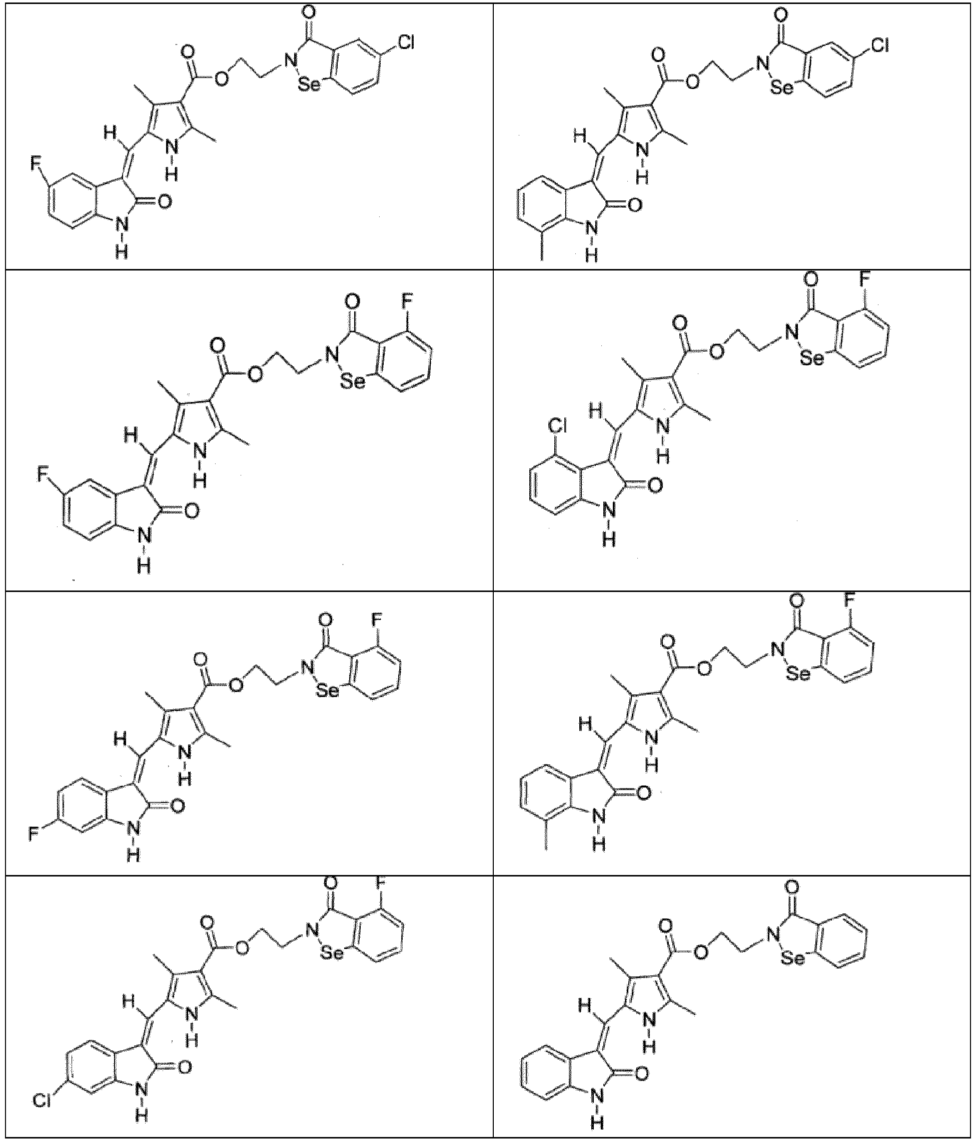

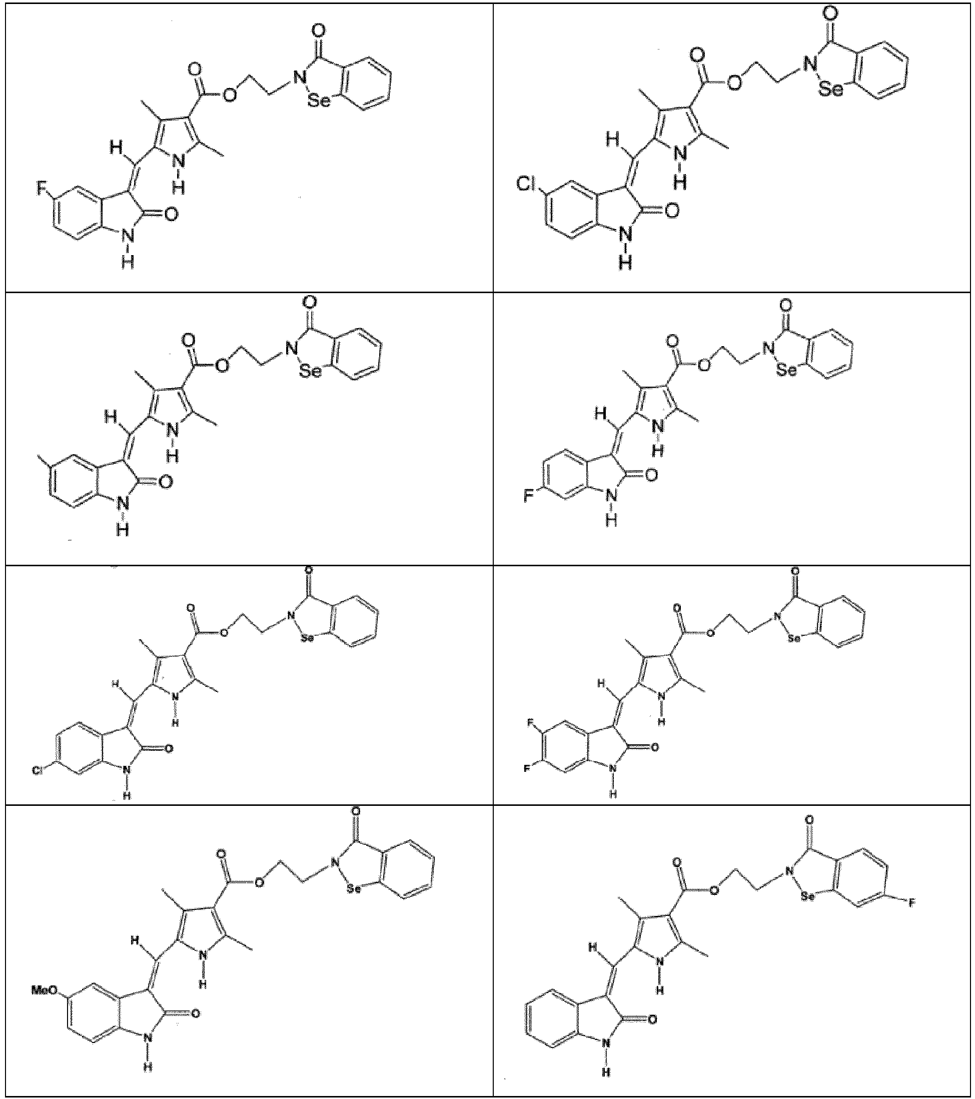

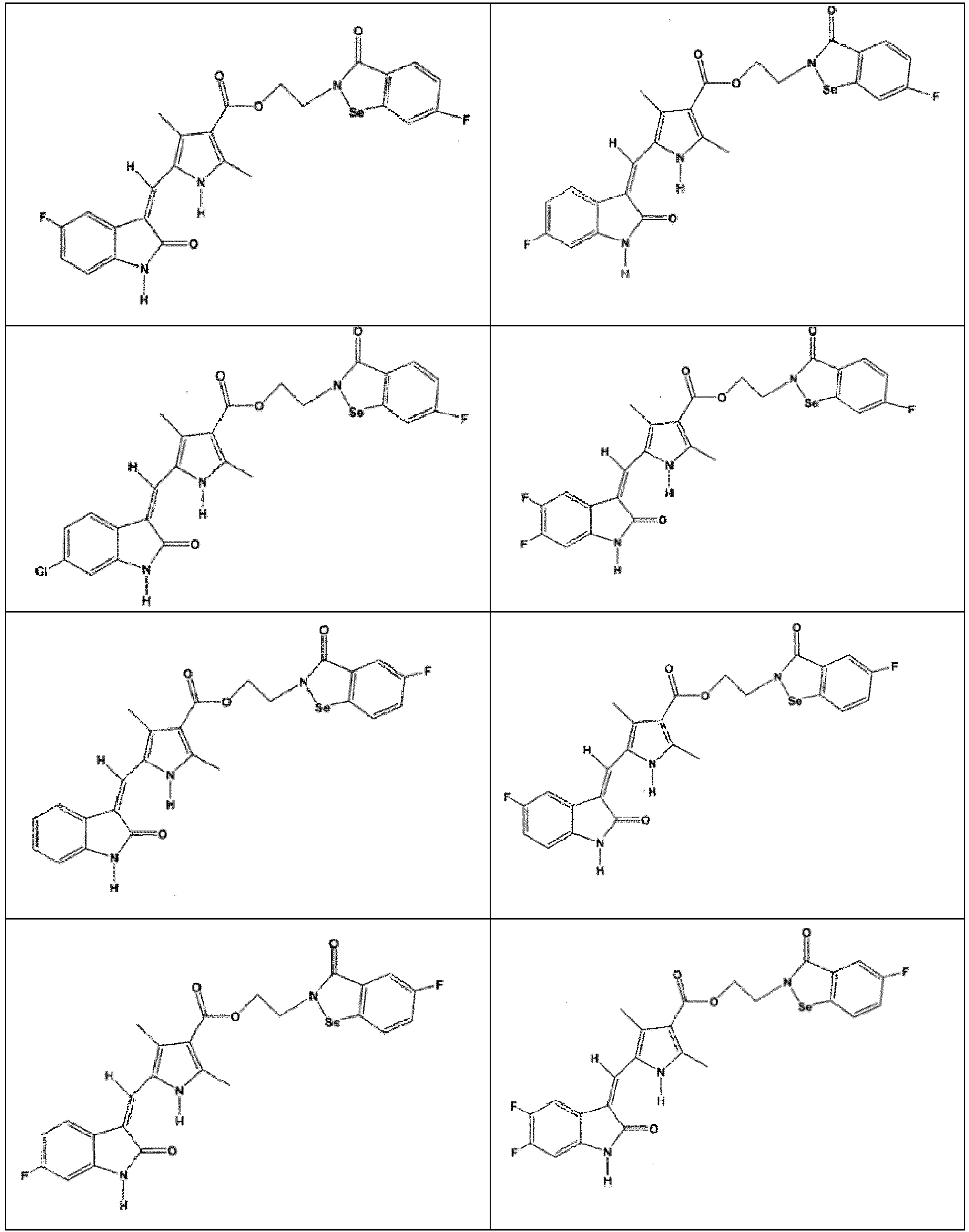

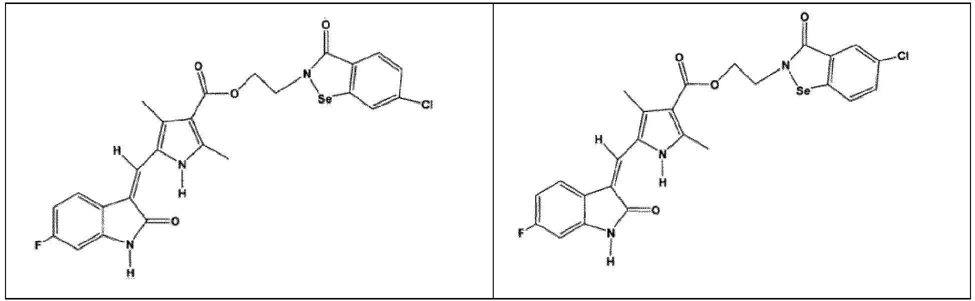

In an embodiment of the present disclosure, the benzisoselenazole derivatives of formula (I) are compounds of the following formula (I-5a) or formula (I-5b):

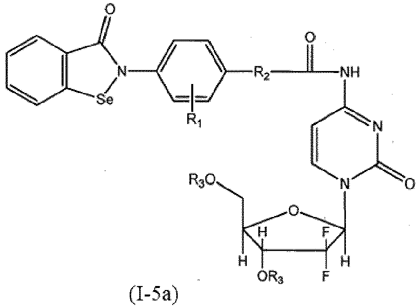

In formula (I-5a), R₁ is selected from hydrogen, halogen, nitrilyl, C₁₋₆ alkyl, C₁₋₆ alkoxy, C₁₋₆ alkylthio, N(C₁₋₆ alkyl)₂, NH(C₁₋₆ alkyl), COOH, -COR_{b}, CONHR_{b}, CONR_{c}R_{d}, SO₃R, aryl, heteroaryl, cycloalkyl and heterocyclyl, which may be optionally substituted with C₁₋₆ alkyl, C₁₋₆ alkoxy, halogen, nitrilyl, aryl or heteroaryl, preferably with -H, -CH₃, halogen, -OAc or -OH, and more preferably with -H, -CH₃, -Cl, -F, -OAc or -OH;
R₂ is selected from C₁₋₁₂ alkylene, phenylene, biphenylene, triphenylene, cyclohexylene, cyclopentane, -RₐSSRₐ-, -(RₐO)ᵤRₐ-, -(CH₂)(RₐO)₃Rₐ-, -RₐN(CH₃)₂Rₐ- and -RₐNHRₐ-;
Rₐ is C₁₋₆ alkylene; R_{b} is H, C₁₋₆ alkyl or C₁₋₆ alkoxy; R_{c} and R_{d} are independently selected from H, C₁₋₆ alkyl and phenyl, or R_{c} and R_{d} together with a N to which they are linked form a nitrogen-containing heterocycle or nitrogen-containing heteroaryl ring;
R₃ is selected from -H, phenylcarbonyl and C₁₋₆ alkyloxycarbonyl;
u is an integer from 0 to 4;
   or

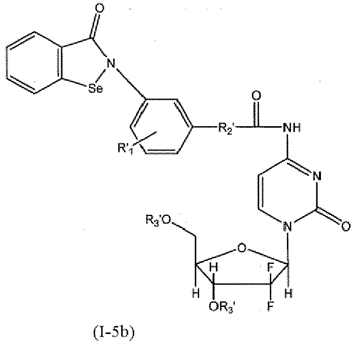

In formula (I-5b), R₁' is selected from hydrogen, halogen, nitrilyl, C₁₋₆ alkyl, C₁₋₆ alkoxy, C₁₋₆ alkylthio, N(C₁₋₆ alkyl)₂, NH(C₁₋₆ alkyl), COOH, -COR_{b}, CONHR_{b}, CONR_{c}R_{d}, SO₃R, aryl, heteroaryl, cycloalkyl and heterocyclyl, which may be optionally substituted with C₁₋₆ alkyl, C₁₋₆ alkoxy, halogen, nitrilyl, aryl or heteroaryl, preferably with -H, -CH₃, halogen, -OAc or -OH, and more preferably with -H, -CH₃, -Cl, -F, -OAc or -OH;
R₂' is selected from C₁₋₁₂ alkylene, phenylene, biphenylene, triphenylene, cyclohexylene, cyclopentane, -RₐSSRₐ-, -(RₐO)ᵤRₐ-, -(CH₂)(RₐO)₃Rₐ-, -RₐN(CH₃)₂Rₐ- and -RₐNHRₐ-;
Rₐ is C₁₋₆ alkylene; R_{b} is H, C₁₋₆ alkyl or C₁₋₆ alkoxy; R_{c} and R_{d} are independently selected from H, C₁₋₆ alkyl and phenyl, or R_{c} and R_{d} together with a N to which they are linked form a nitrogen-containing heterocycle or nitrogen-containing heteroaryl ring;
R₃' is selected from -H, phenylcarbonyl and C₁₋₆ alkyloxycarbonyl;
u is an integer from 0 to 4.

According to the present disclosure, the benzisoselenazole derivatives of formula (I-5a) or formula (I-5b) are selected from the compounds in Table 5 below:

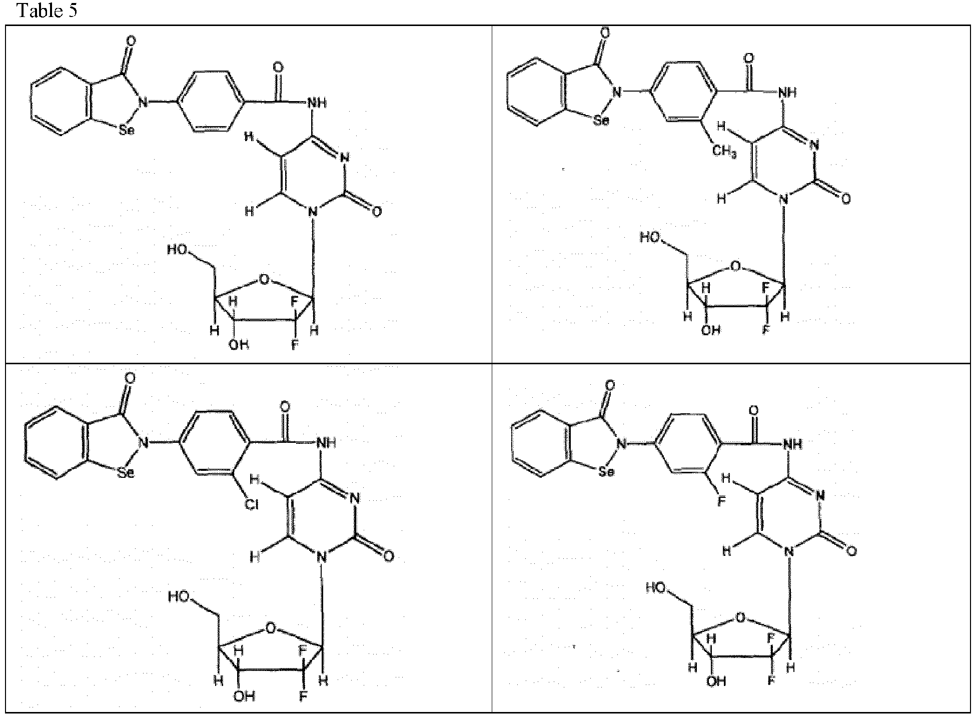

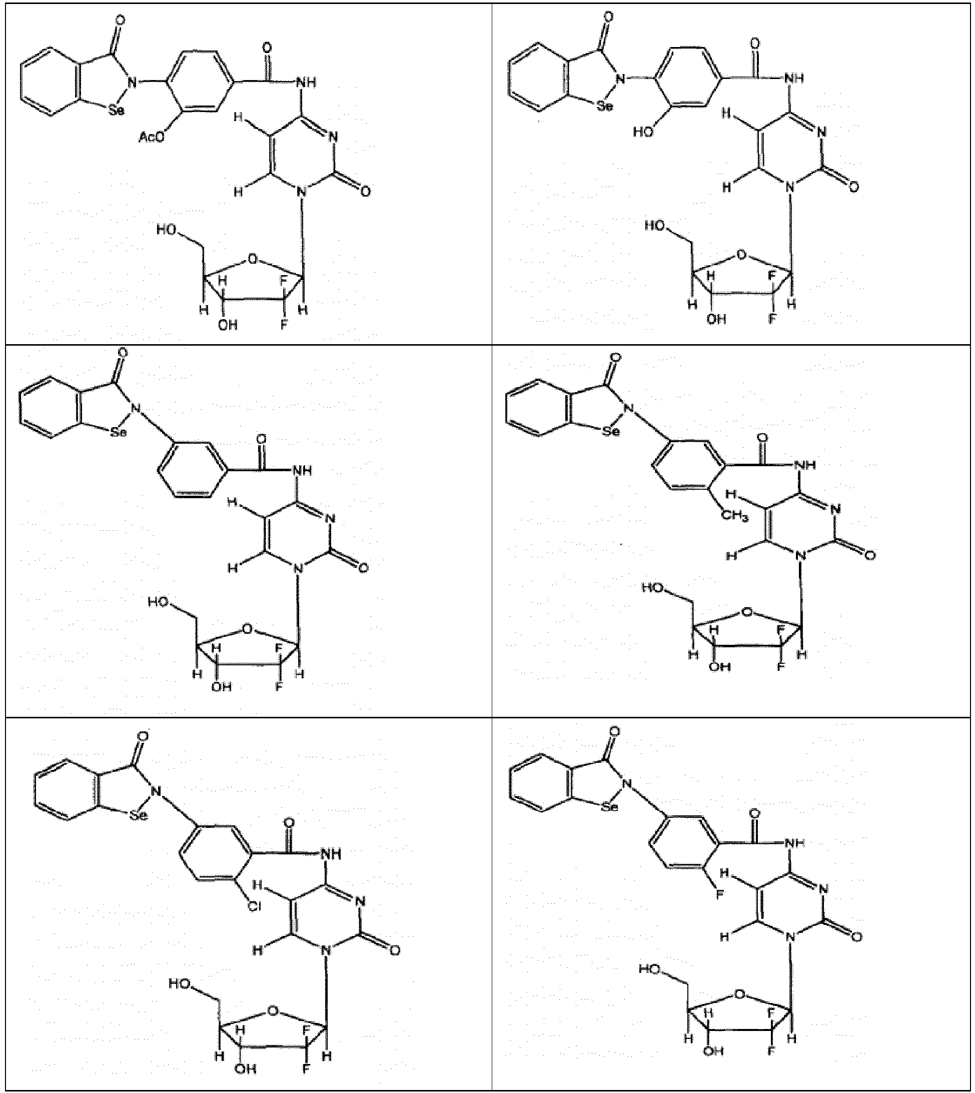

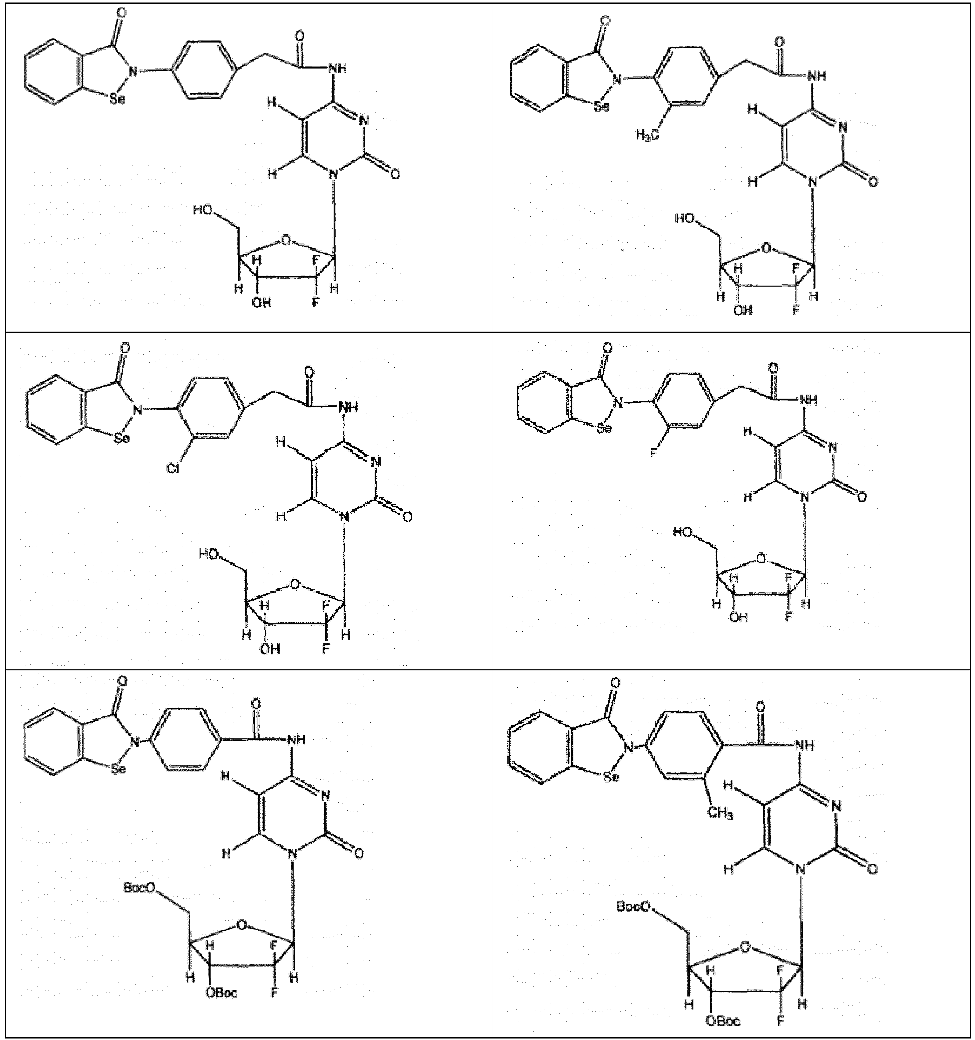

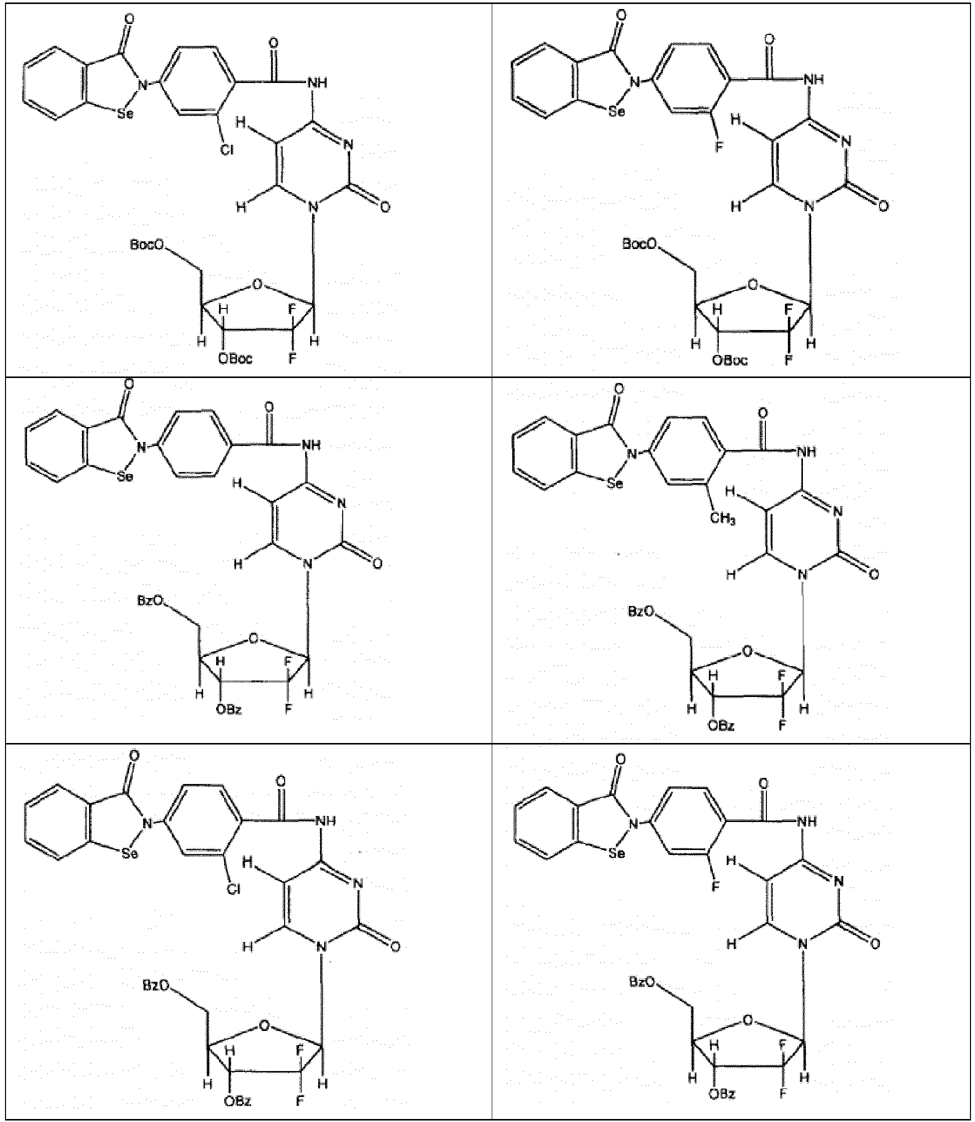

In an embodiment of the present disclosure, the benzisoselenazole derivatives of formula (I) are compounds of the following formula (I-6): 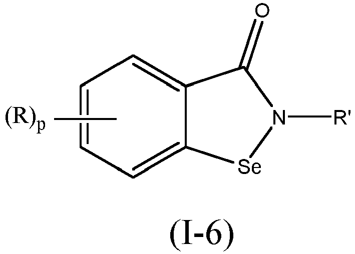
wherein R and p each have the definitions given above in the compounds of formula (I);
R' is selected from hydrogen, C₁₋₆ alkyl, C₃₋₇ cycloalkyl, C₁₋₆ alkyl-OH, R^{e}-NH-R^{f}, 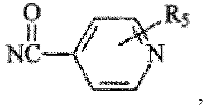 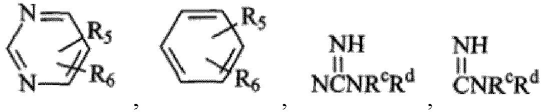 and saccharide residue;
R₅ and R₆ are each independently selected from hydrogen, halogen, cyano, C₁₋₆ alkyl, C₁₋₆ alkoxy, C₁₋₆ alkylthio, N(C₁₋₆ alkyl)₂, NH(C₁₋₆ alkyl), COOR^{b} and SO₃R^{b};
R^{b} is selected from hydrogen, C₁₋₆ alkyl and C₁₋₆ alkoxy;
and R^{d} are each independently selected from hydrogen, C₁₋₆ alkyl and phenyl, or R^{c} and R^{d} together with a nitrogen atom to which they are linked form a nitrogen-containing heterocycle or nitrogen-containing heteroaryl ring;
R^{e} and R^{f} are each independently selected from C₁₋₆ alkyl and C₃₋₇ cycloalkyl.

According to an embodiment of the present disclosure, the saccharide residue may be a *D*-glucopyranosyl group, such as 1,3,4,6-tetra-*O*-acetyl-2-deoxy-*D*-glucopyranosyl.

According to the present disclosure, the benzisoselenazole derivatives of formula (I-6) are selected from the compounds in Table 6 below:

**Table 6**

| | |
|---|---|
| 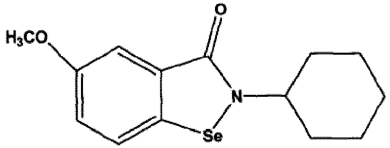 | 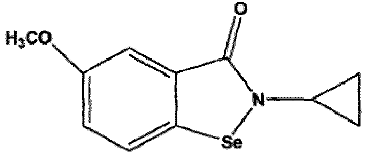 |
| 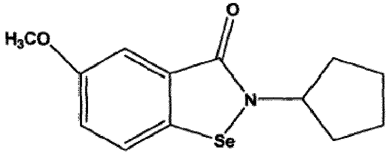 | 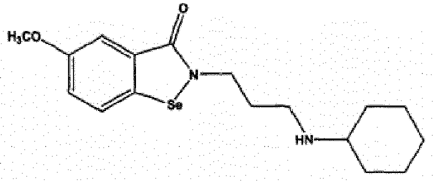 |
| 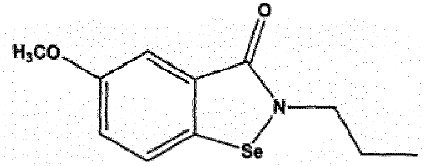 | 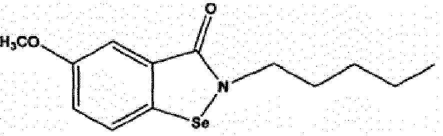 |
| 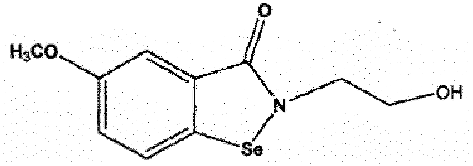 | 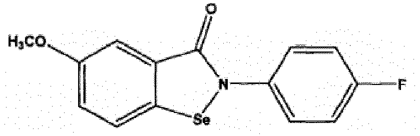 |
| 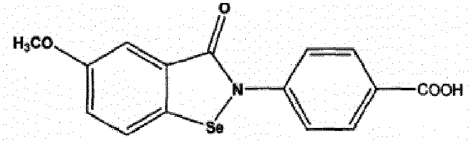 | |
| 4-{*N*-[1,2-Benzisoselenazol-3(2*H*)-one]formylhydrazine}-pyridine | 2-[1,2-Benzisoselenazol-3(2*H*)-one]-4,6-dimethylpyr imidine |
| 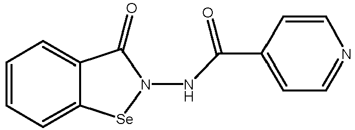 | 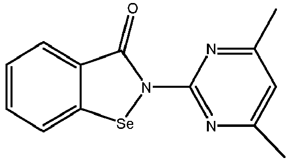 |
| 4-[1,2-Benzisoselenazol-3(2*H*)-one]-2-hydroxybenzoic acid | *N*-[1,2-Benzisoselenazol-3(2*H*)-one]-aminoguanidin e |
| 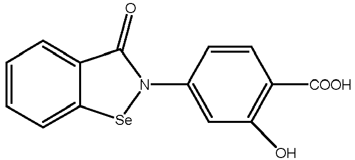 | 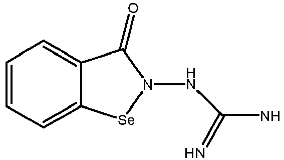 |
| *N*-[1,2-Benzisoselenazol-3(2*H*)-one]-guanidine | 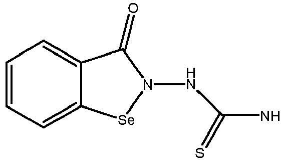 |
| 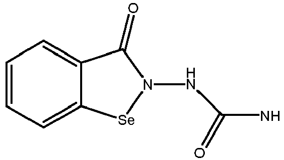 | |
| 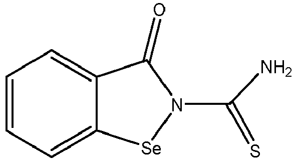 | 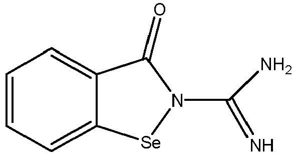 |
| 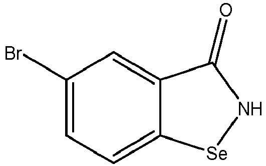 | 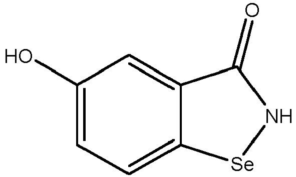 |
| 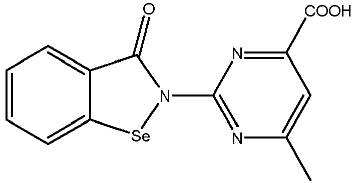 | |

The present disclosure also provides use of benzisoselenazole derivatives of the following formula (II) for the manufacturing of anti-coronavirus medicaments: 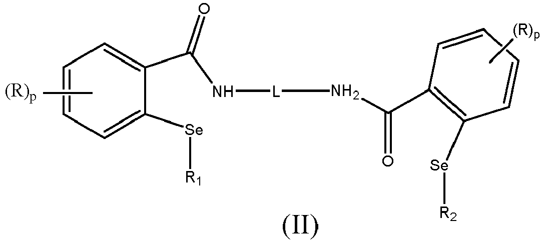 wherein R, p and L each have the definitions given above in the compounds of formula (I); R₁ and R₂ are each independently selected from C₁₋₆ alkyl, C₁₋₆ alkoxy, C₁₋₆ alkylthio and 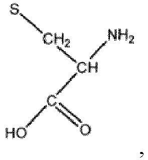 wherein the S atom is linked to the Se atom by a single bond.

According to an embodiment of the present disclosure, R₁ and R₂ are both 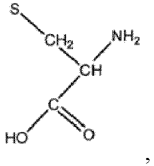 wherein the S atom is linked to the Se atom by a single bond.

According to the present disclosure, the benzisoselenazole derivatives of formula (II) are selected from the compounds in Table 7 below:

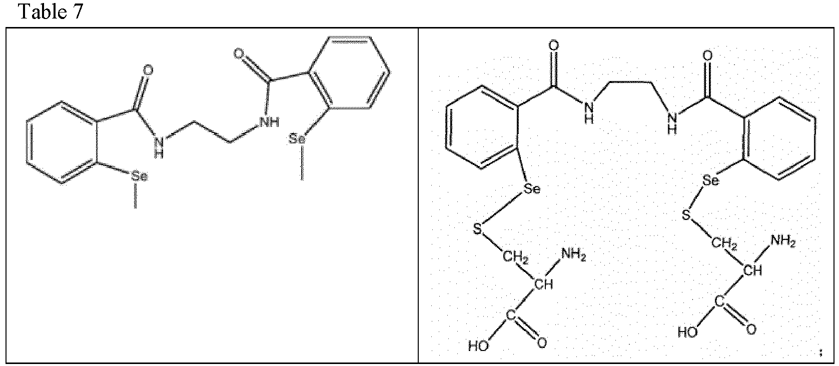

The present disclosure also provides use of benzisoselenazole derivatives of the following formula (III) for the manufacturing of anti-coronavirus medicaments: 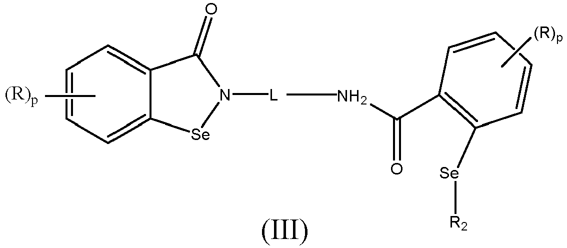 wherein R, p and L each have the definitions given above in the compounds of formula (I); R₂ is selected from C₁₋₆ alkyl, C₁₋₆ alkoxy, C₁₋₆ alkylthio and 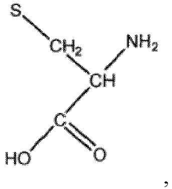 wherein the S atom is linked to the Se atom by a single bond.

According to the present disclosure, the benzisoselenazole derivatives of formula (III) are the compounds in Table 8 below:

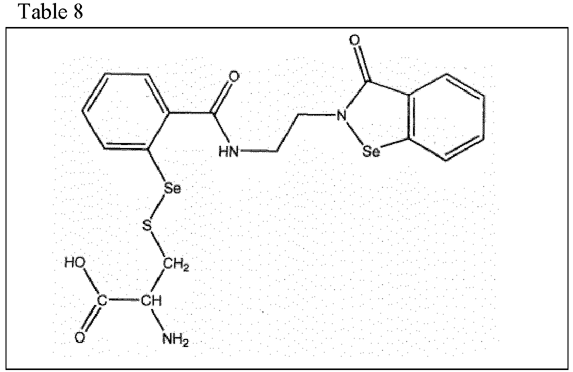

According to the present disclosure, the coronaviruses include HCoV-229E, HCoV-OC43, HCoV-NL63, HCoV-HKU1, SARS-CoV, MERS-CoV and 2019-nCoV, particularly 2019-nCoV.

The present disclosure also provides use of the benzisoselenazole derivatives of formula (I) described above for the manufacturing of medicaments for the treatment of diseases caused by coronaviruses.

According to the present disclosure, the coronaviruses include HCoV-229E, HCoV-OC43, HCoV-NL63, HCoV-HKU1, SARS-CoV, MERS-CoV and 2019-nCoV, particularly 2019-nCoV.

According to the present disclosure, the diseases caused by coronaviruses include respiratory diseases, such as upper respiratory diseases and/or lower respiratory diseases, such as acute or chronic infection of the upper or lower respiratory tract.

According to the present disclosure, the diseases caused by coronaviruses include severe acute respiratory syndrome, Middle East respiratory syndrome and coronavirus disease 2019 (COVID-19).

According to the present disclosure, the diseases caused by coronaviruses include interstitial lung disease (ILD). In some embodiments, the medicaments are capable of restoring pulmonary function and treating pulmonary injuries.

In some embodiments, the medicaments are capable of inhibiting spleen swelling.

In some embodiments, the medicaments are capable of treating or ameliorating pulmonary inflammation, e.g., capable of inhibiting CD8+ T cells, CD4+ T cells and the B cell count.

In some embodiments, the medicaments are capable of inhibiting/alleviating the symptoms of pulmonary fibrosis complicated by interstitial pneumonia. In one aspect, the medicaments are capable of improving in a patient the acidity and alkalinity of the blood, the partial pressure of oxygen, the base excess of interstitial fluid, the actual bicarbonate, the total carbon dioxide, the oxygen saturation and/or the lactate level. Illustratively, 1,4-[bis(1,2-benzisoselenazol-3(2*H*)-one)]butane produces significant improvements to the function of pulmonary ventilation and the acid-base balance state in a patient when administered at doses of 90 mg/kg and 180 mg/kg of the subject's body weight. In yet another aspect, the medicaments are further capable of lowering the NF-κB, TNF-α, IL-2, IL-6, IL-1β and/or TGF-β levels and improving the IFN-γ level in a subject's bronchoalveolar lavage fluid. In yet another aspect, the medicaments are capable of reducing structural changes in the lung tissues and inflammatory cell infiltration and improving the pulmonary fibrosis condition at the early stage of pulmonary fibrosis and/or at the formation stage of pulmonary fibrosis.

According to an embodiment of the present disclosure, the medicaments are particularly suitable for preventing/treating interstitial pneumonia. In some embodiments, the medicaments are used for preventing/treating fibrotic interstitial pneumonia; they are capable of controlling the development and progression of pulmonary fibrosis. In some embodiments, the medicaments are capable of controlling the development and progression of pulmonary fibrosis-associated interstitial pneumonia.

In some embodiments, the medicaments are capable of inhibiting ECM deposition. Illustratively, 1,4-[bis(1,2-benzisoselenazol-3(2*H*)-one)]butane is capable of inhibiting ECM deposition when administered at a dose of 180 mg/kg of the subject's body weight.

In some embodiments, the medicaments are capable of reducing the deposition of bronchial submucosal and pulmonary interstitial collagen fibers.

In addition, the present disclosure also provides a method of treatment of coronavirus infections in a mammal, which comprises administering to the mammal in need a therapeutically effective amount of a benzisoselenazole derivative of formula (I) described above.

### Beneficial Effects

The benzisoselenazole derivatives of the present disclosure are capable of effectively inhibiting the activity of the 2019-nCoV 3CLpro proteolytic enzyme and further the activation of the 2019-nCoV RNA polymerase and viral replication, so viruses are finally eliminated. Therefore, they are effective against coronavirus and diseases caused thereby.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the present specification.

The term "alkyl" refers to a linear or branched saturated monovalent hydrocarbon group containing a specified number of carbon atoms. For example, "C₁₋₁₂ alkyl" refers to linear and branched alkyl groups containing 1, 2, 3, 4, 5, 6, 7, 8, 9 or 10 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof.

The term "alkoxy" refers to -O-alkyl, wherein the alkyl has the definition described above.

The term "alkylthio" refers to -S-alkyl, wherein the alkyl has the definition described above.

The term "cycloalkyl" refers to a saturated monovalent monocyclic or bicyclic hydrocarbon ring containing a specified number of carbon atoms. The term "C₃₋₁₂ cycloalkyl" refers to a saturated monovalent monocyclic or bicyclic hydrocarbon ring containing 3, 4, 5, 6, 7, 8, 9, 10, 11 or 12 carbon atoms. The C₃₋₁₂ cycloalkyl may be a monocyclic hydrocarbon group, such as cyclopropyl, cyclobutyl, cyclopentyl, cyclohexyl, cycloheptyl, cyclooctyl, cyclononyl or cyclodecyl, or a bicyclic hydrocarbon group, such as a decaline ring.

The term "alkylene" refers to a divalent alkyl group containing a specified number of carbon atoms; for example, C₁₋₁₂ alkylene refers to a divalent C₁₋₁₂ alkyl group.

The term "alkenyl" refers to a linear or branched monovalent hydrocarbon group containing a specified number of carbon atoms and containing one or more olefinic bonds, e.g., C₂₋₁₂ alkenyl, preferably C₂₋₆ alkenyl; for example, "C₂₋₆ alkenyl" means containing 2, 3, 4, 5 or 6 carbon atoms (i.e., C₂₋₆ alkenyl), or containing 2 or 3 carbon atoms (i.e., C₂₋₃ alkenyl). It should be understood that in the case that the alkenyl comprises more than one double bond, the double bonds can be separated from one another or conjugated. The alkenyl is, for example, vinyl, allyl, (*E*)-2-methylvinyl, (*Z*)-2-methylvinyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl or 1-isopropylvinyl.

The term "heterocyclyl" refers to a saturated monovalent monocyclic or bicyclic hydrocarbon ring containing a specified number of ring atoms, e.g., 3 to 14 ring atoms, which contains 1 to 5 heteroatoms independently selected from N, O and S, and is preferably "3- to 10-membered heterocyclyl". The term "3- to 10-membered heterocyclyl" refers to a saturated monovalent monocyclic or bicyclic hydrocarbon ring containing 1 to 5, preferably 1 to 3, heteroatoms selected from N, O and S. The heterocyclyl may be connected to the rest of the molecule through any of the carbon atoms or the nitrogen atom (if present). In particular, the heterocyclyl may include, but is not limited to: 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl or trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. The ring containing nitrogen atoms may be partially unsaturated, i.e., it may contain one or more double bonds, for example, but not limited to, 2,5-dihydro-1*H*-pyrrolyl, 4*H*-[1,3,4]thiadiazinyl, 4,5-dihydrooxazolyl or 4*H*-[1,4]thiazinyl, or it may be benzo-fused, for example, but not limited to, dihydroisoquinolyl. According to the present disclosure, the heterocyclyl is non-aromatic.

The term "aryl" refers to a monovalent aromatic or partially aromatic monocyclic, bicyclic or tricyclic hydrocarbon ring containing a specified number of carbon atoms, e.g., 6 to 20 carbon atoms, preferably 6 to 14 carbon atoms, and preferably to "C₆₋₁₄ aryl". The term "C₆₋₁₀ aryl" preferably refers to a monovalent aromatic or partially aromatic monocyclic, bicyclic or tricyclic hydrocarbon ring containing 6, 7, 8, 9, 10, 11, 12, 13 or 14 carbon atoms ("C₆₋₁₄ aryl"), in particular a ring containing 6 carbon atoms ("C₆ aryl"), such as phenyl or biphenyl, a ring containing 9 carbon atoms ("C₉ aryl"), such as indanyl or indenyl, a ring containing 10 carbon atoms ("C₁₀ aryl"), such as tetrahydronaphthyl, dihydronaphthyl or naphthyl, a ring containing 13 carbon atoms ("C₁₃ aryl"), such as fluorenyl, or a ring containing 14 carbon atoms ("C₁₄ aryl"), such as anthryl. When the C₆₋₁₄ aryl is substituted, it may be monosubstituted or polysubstituted. In addition, the substitution site is not limited, and may be, for example, ortho-substitution, para-substitution, or meta-substitution.

The term "heteroaryl" refers to a monovalent monocyclic, bicyclic or tricyclic aromatic ring group containing a specified number of ring atoms, e.g., 3 to 20 ring atoms, and containing 1 to 5 heteroatoms independently selected from N, O, S, Se, Te, and so on, e.g., "5- to 14-membered heteroaryl". The term "5- to 14-membered heteroaryl" refers to a monovalent monocyclic, bicyclic or tricyclic aromatic ring group containing 5, 6, 7, 8, 9, 10, 11, 12, 13 or 14 ring atoms, particularly 5 or 6 or 9 or 10 carbon atoms, and containing 1 to 5, preferably 1 to 3, heteroatoms independently selected from N, O and S, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl and the like and benzo derivatives thereof, such as benzofuranyl, benzothienyl, benzoxazolyl, benzoisoxazolyl, benzimidazolyl, benzotriazolyl, indazolyl, indolyl, isoindolyl, benzisoselenazolyl and the like; or pyridinyl, pyridazinyl, pyrimidinyl, pyrazinyl, triazinyl and the like and benzo derivatives thereof, such as quinolyl, quinazolinyl, isoquinolyl and the like; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl and the like.

The term "oxo" means that the carbon atom, nitrogen atom or sulfur atom in the substituent is substituted with an oxy group formed after oxidation (=O).

The term "thio" refers to the group C=S formed by replacing the oxygen atom in carbonyl with a sulfur atom.

The pharmaceutically acceptable salts of the benzisoselenazole derivatives of the present disclosure include: acid addition salts formed from the compounds and inorganic or organic acids; the inorganic acids are selected from at least one of hydrochloric acid, hydrofluoric acid, hydrobromic acid, hydroiodic acid, perchloric acid, sulfuric acid, pyrosulfuric acid, phosphoric acid, and nitric acid; the organic acids are selected from at least one of formic acid, acetic acid, acetoacetic acid, pyruvic acid, trifluoroacetic acid, propionic acid, butyric acid, caproic acid, enanthic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid, and thiocyanic acid;
alternatively, the pharmaceutically acceptable salts of the benzisoselenazole derivatives of the present disclosure are alkali metal salts, alkaline earth metal salts or ammonium salts of the compounds, or salts formed from the compounds and organic bases providing physiologically acceptable cations, e.g., salts formed from the compounds and at least one of the following substances: sodium ion, potassium ion, calcium ion, magnesium ion, morpholine, piperidine, triethylamine, tripropylamine, tributylamine, diisopropylamine, diisopropylethylenediamine, pyridine, dimethylamine, diethylamine, N-methylglucamine, dimethylglucamine, ethylglucamine, lysine, dicyclohexylamine, 1,6-hexanediamine, ethanolamine, glucosamine, meglumine, sarcosine, serinol, trihydroxymethylaminomethane, aminopropanediol, and 1-amino-2,3,4-butanetriol.

The compounds disclosed herein may exist in the form of a solvate (e.g., hydrate), and the compounds disclosed herein contain a polar solvent as a structural element of the crystal lattice of the compound, particularly, for example, water, methanol or ethanol. The amount of polar solvent, especially water, can exist in a stoichiometric or non-stoichiometric ratio.

According to the molecular structure, the compounds disclosed herein may be chiral and may therefore exist in various enantiomeric forms. These compounds may therefore exist in racemic or optically active form. The compounds disclosed herein or intermediates thereof may be separated into enantiomers by chemical or physical methods well known to those skilled in the art, or used in this form for synthesis. The corresponding stable isomers can be separated according to known methods, such as extraction, filtration or column chromatography.

### BRIEF DESCRIPTION OF THE DRAWINGS

FIG. 1 is a schematic diagram of 24-h building of animal models and administration.
FIG. 2 is a schematic diagram of 7-d building of animal models and administration.
FIG. 3 shows changes in body weight of mice of 24-h modeling.
FIG. 4 shows changes in body weight of mice of 7-d modeling.

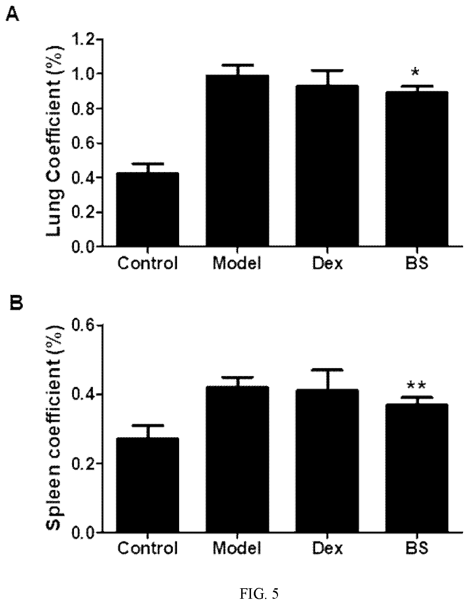

FIG. 5 shows lung and spleen coefficients of mice in 24-h modeling groups.

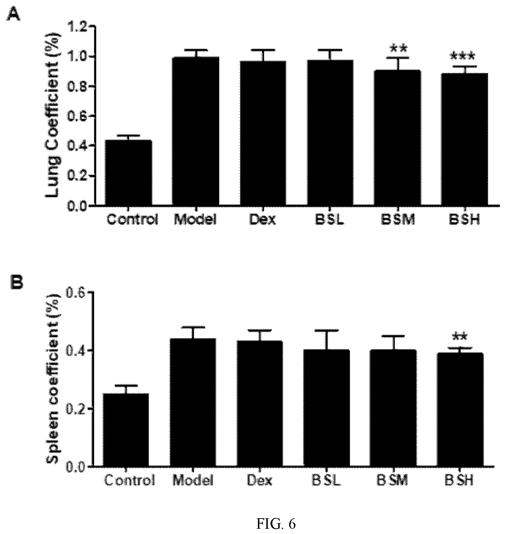

FIG. 6 shows lung and spleen coefficients of mice in 7-d modeling groups.

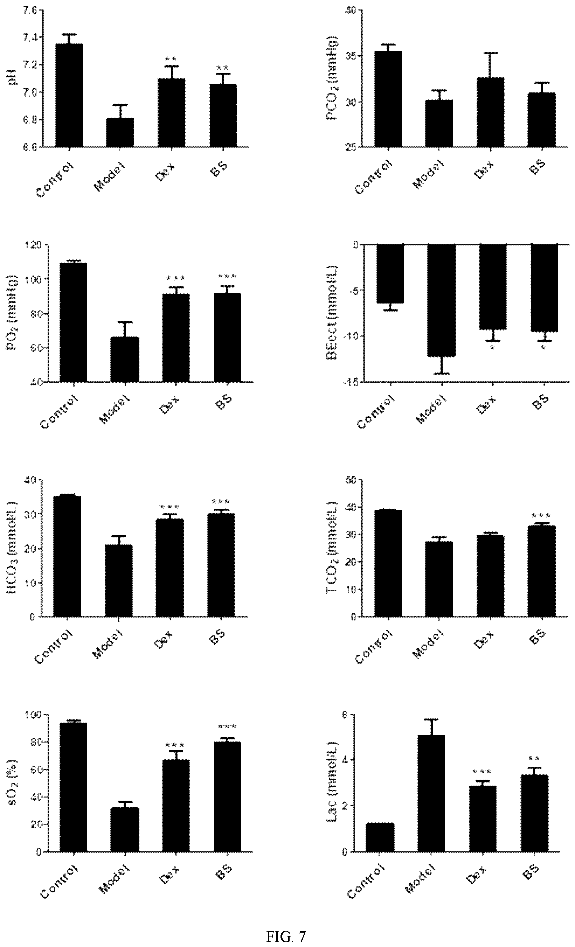

FIG. 7 shows comparisons between blood gas analysis results of mice in 24-h modeling groups after administration ended.

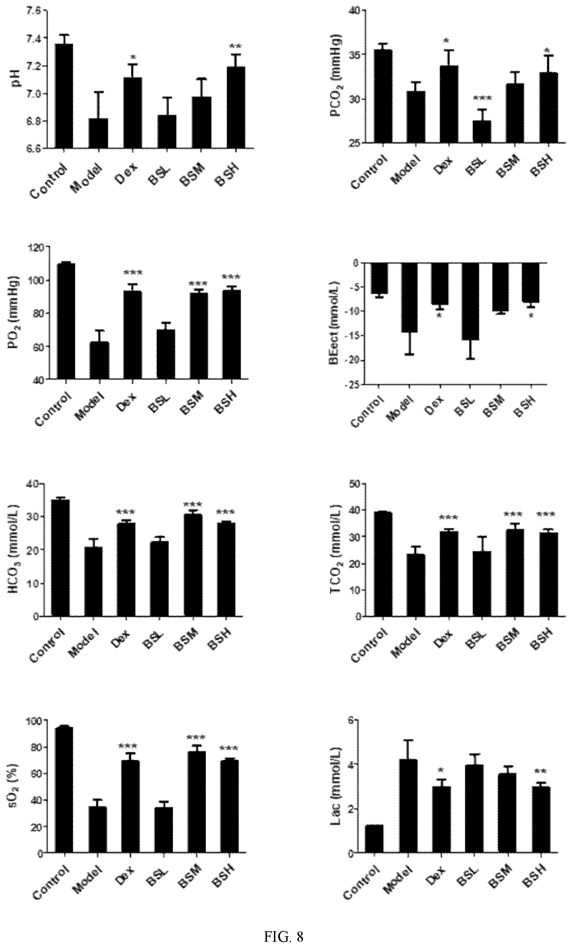

FIG. 8 shows comparisons between blood gas analysis results of mice in 7-d modeling groups after administration ended.
FIG. 9 shows comparisons between the inflammatory cell counts in bronchoalveolar lavage fluids of 7-d modeling groups.

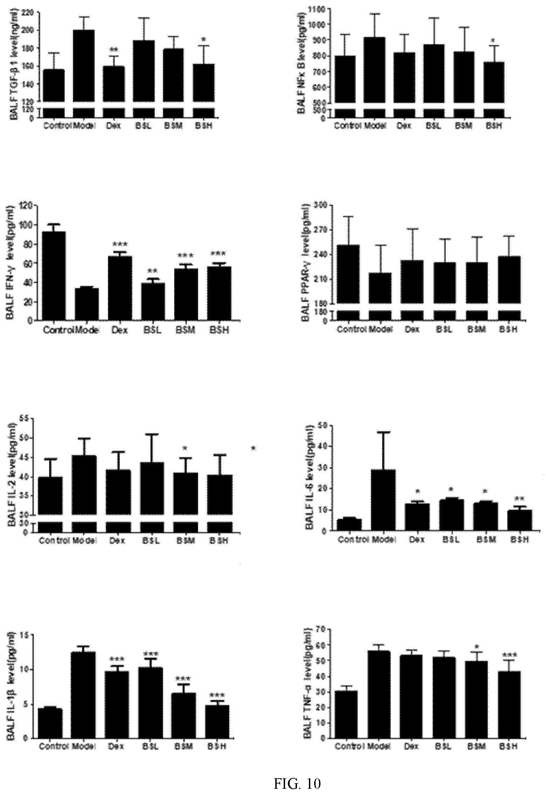

FIG. 10 shows comparisons of cytokines in bronchoalveolar lavage fluids.
FIG. 11 shows the hydroxyproline content in the lungs of mice on day 35 of administration after 24-h modeling.
FIG. 12 shows pulmonary fibrosis scores of mice in 24-h modeling groups.
FIG. 13 shows HE results of the lung tissues of mice in 24-h modeling groups.
FIG. 14 shows pulmonary fibrosis scores of mice in 7-d modeling groups.
FIG. 15 shows HE results of the lung tissues of mice in 7-d modeling groups.

### DETAILED DESCRIPTION

The use of the present disclosure will be illustrated in further detail by the following description of specific embodiments. It should be understood that the following embodiments are merely exemplary illustration and explanation of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared using known methods.

The benzisoselenazole derivatives of the present disclosure can be prepared using methods known in the art or methods in the literature. For example, reference may be made to the preparation methods in the following documents: the preparation methods described in CN106977472A, particularly the preparation methods described in Examples 1 to 34; the preparation methods described in CN106146371A, particularly the preparation methods described in Examples 1 to 5; the preparation methods described in CN104119329A, particularly the preparation methods described in Examples 1 to 68; the preparation methods described in CN102898402A, particularly the preparation methods described in Examples 1 to 96; the preparation methods described in CN102234254A, particularly the preparation methods described in Examples 1 to 13; the preparation methods described in CN101921303A, particularly the preparation methods described in Examples 1 to 6; the preparation methods described in CN101786976A, particularly the preparation method described in Example 1; the preparation methods described in CN100497324C, particularly the preparation methods described in Examples 1 to 9; the preparation methods described in CN1990475A, particularly the preparation methods described in Examples 1 to 17; the preparation methods described in CN1704409A, particularly the preparation methods described in Examples 1 to 9; the preparation methods described in CN1280279C, particularly the preparation methods described in Examples 1 to 12; the preparation methods described in CN1242999C, particularly the preparation methods described in Examples 1 to 4; the preparation methods described in CN202010287745.6, particularly the preparation methods in Examples 1 to 8. The documents are incorporated herein by reference in their entirety.

### Biological Example 1

2019-nCoV is an RNA virus. Its proliferation depends on the regeneration of maternal RNA by RNA-dependent RNA polymerase (RdRp). The 2019-nCoV 3CLpro protein is a key protein for 2019-nCoV's key protein RdRp to be in activated form. The 2019-nCoV 3CLpro protein hydrolyzes the virus's pp1a and pp1ab proteins to form a mature product nsp1-16, which is further assembled into active RdRp. At present, 2019-nCoV 3CLpro has been considered as one of the drug targets for combating coronaviruses. Based on the basic characteristic that the 2019-nCoV 3CLpro protein is a proteolytic enzyme, a screening system was established for determining the activity of the 2019-nCoV 3CLpro protein using a fluorescence method. The 2019-nCoV 3CLpro protein can specifically cleave substrates where Gln (Q) is at position P1. In the determination of its activity, a fluorescent polypeptide can be used as the substrate. The generation of fluorescent signals can be detected to reflect the activity of the proteolytic enzyme.

**Table 9:**

| Compound structures | IC₅₀ (µg/ml) |
|---|---|
| 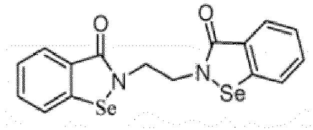 | 0.52±0.14 |
| 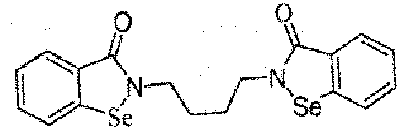 | 0.05±0.00 |
| 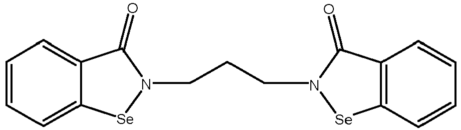 | 0.05±0.01 |
| 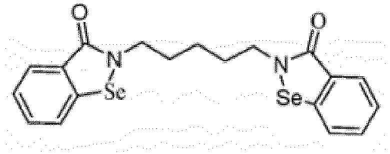 | 0.04±0.00 |
| 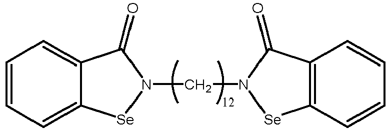 | 0.02±0.00 |
| 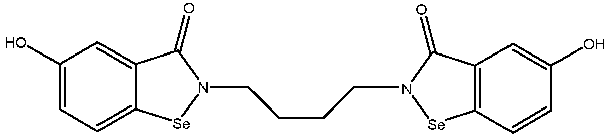 | 0.02±0.00 |
| 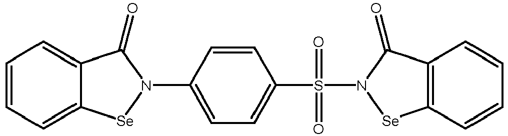 | 0.66±0.28 |
| 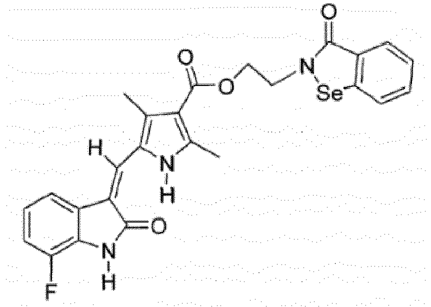 | 0.86±0.28 |
| 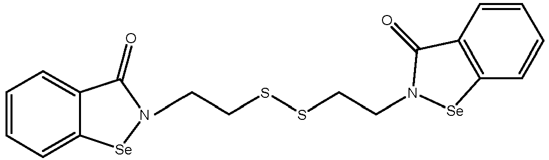 | 0.75±0.28 |

From the above results, it can be seen that the benzisoselenazole derivatives of the present disclosure can effectively inhibit the activity of the 2019-nCoV 3CLpro protease, further that of the RNA-dependent RNA polymerase (RdRp) and thereby viral replication. Therefore, they are effective against coronavirus and diseases caused thereby.

### Biological Example 2

### Objective:

In an *in vitro* cell test, the cytotoxicity of test samples was determined according to the growth of drug-treated Vero E6 cells. The inhibitory effects of the test samples on viral replication were determined by observing the cytopathic effects (CPE) produced by the drug-treated Vero E6 cells with viral infections. During observation, it was found that the drugs' inhibitory effects on the SARS-CoV-2 virus could be observed when the drugs were at low concentrations of 1-2.5 µM, and that reasonable evaluations could not be made in cases with higher concentrations due to the drugs' toxicity background for CPE, which suggests that the drugs with these concentrations have inhibitory effects on the SARS-CoV-2 virus.

### Materials and methods:

1. Material source: the 2019-nCoV virus strain was isolated and obtained from Prevention and Control Center, Wuhan Institute of Virology. The virus strain was passaged twice through Vero E6, and the virus strain was harvested 6 days after inoculation. The virus was preserved at -80 °C, and virus isolation, TCID₅₀ assays and so forth were all carried out in a BSL-3 laboratory.
2. Reagents and instruments

VeroE6 cells (ATCC^{®} CRL1586^{™}), cell culture medium MinimumEssentialMedium (Gibco, Ref. No. 41500-034), and fetal bovine serum (PANSera ES, Cat. No. 2602-P130707); instruments and apparatus included a carbon dioxide incubator, a cell counter and an inverted microscope.

### 3. Methods

Median tissue culture infectious dose (TCID₅₀) assay: The virus culture on day 6 after inoculation (the cells were all diseased) was subjected to one freezing-thawing cycle and centrifuged, and the supernatant was collected and cryopreserved in aliquots. One tube of the cryopreserved virus solution was half-log diluted from 10⁻² to 10⁻⁷. A 96-well plate with a single layer of Vero-E6 adherent cells growing in it was washed twice with a cell maintenance solution (MEM containing 2% fetal bovine serum), and 100 µL of the diluted virus solution was added, 4 replicate wells per dilution. The cell maintenance solution was used as a negative control. The plate was incubated in a 36 °C, 5% COz incubator for 90 min, during which the plate was shaken once every 30 min. After the incubation, the virus solution was pipetted off. After the cells were washed once with the cell maintenance solution, 200 µL of the cell maintenance solution was added to each well. The plate was incubated in the 36 °C, 5% COz incubator, and pathological changes in the cells were observed. The experiment ended 6 days after inoculation. The TCID₅₀ value was calculated using the Reed-Muench method and the cells were counted.

### 4. Viral proliferation and titer assay

The virus stock solution was diluted to 5 TCID₅₀/100 µL, and 7.8 mL of the virus solution (converted according to the base area of the culture vessel and the 100 µL/well amount in a TCID₅₀ assay) was inoculated into a T25 (the base area was 25 cm²) cell culture flask (the density of adherent cells was 95%-100%, the cell quantity was about 2.42 × 10⁶/flask, MOI was about 1.74 × 10⁻⁴ TCID₅₀/cell). The flask was incubated in a 36 °C, 5% COz incubator for 90 min, during which the cell flask was shaken once every 30 min. After the incubation, the virus solution was pipetted off, and the cells were washed once with the cell maintenance solution. To the cell flask was added 10 mL of the cell maintenance solution, and the flask was incubated in the 36 °C, 5% COz incubator. From the start of the incubation to day 6 when the experiment ended, pathological changes in the cells were observed every 24 h, meanwhile 0.55 mL of the proliferation product was collected using a pipet and centrifuged, and the supernatant was cryopreserved at -80 °C for later use. The supernatant collected each day was half-log diluted from the original concentration to 10⁻⁵ and inoculated onto a 96-well cell plate, 3 well per dilution. Pathological changes in the cells were observed every day. The TCID₅₀ value was calculated, and the cells were counted.

### 5. Drug formulation (suspensions of the compounds of the present disclosure in 5‰ CMC-Na solution)

Representative compounds of the present disclosure are of the following structure (BS for short):

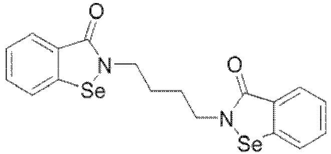

The results are shown in Table A:

**Table A. Cytotoxicity of BS to cells and inhibition of SARS-CoV-2 virus**

| | 20µm | 10µm | 5µm | 2.5µm | 1.25µm | 0.625µm | 0.3µm | 0.16µm | virus | cell |
|---|---|---|---|---|---|---|---|---|---|---|
| Antiviral activity | ++ | ++ | ++ | ++ | +++ | ++++ | ++++ | ++++ | ++++ | - |
| | ++ | ++ | ++ | +++ | +++ | ++++ | ++++ | ++++ | ++++ | - |
| Cytotoxicity | ++ | ++ | ++ | + | - | - | - | - | ++++ | - |

### Biological Example 3

### 1. Interstitial lung disease animal model

Bleomycin is a chemotherapeutic drug for use in the treatment of a variety of tumors, such as lymphoma, testicular cancer, ovarian cancer and malignant pleural effusion. The most serious adverse effect of bleomycin is pulmonary toxicity, which manifests itself first as endothelial cell and interstitial capillary edema, necrosis of type II alveolar epithelial cells and inflammatory mediator release. Subsequently, the fibroblasts proliferate and transform into myofibroblasts. At present, bleomycin is the most important and widely applied pulmonary fibrosis inducer in animal models. The model generation process comprises the presentation of main element characteristic processes of interstitial lung disease, such as inflammation, inflammation accompanied by the development and progression of fibrosis, irreversible fibrosis formation and the like.

In this experiment, the pathological model process obtained by administration to mice exhibited inflammation (24 h after administration), fibrosis and so on, and mainly exhibited fibrosis in the later stage of the model. Specifically, inflammation and epithelial cell apoptosis occurred on about day 10 and pulmonary fibrosis on day 14; the pulmonary fibrosis pathological change was most significant on days 21-28. The degree and distribution of bleomycin-induced pulmonary fibrosis are dose dependent. A single dose of bleomycin will lead to relatively transient changes, while repeated doses will lead to long-term pathological changes. This is more similar to the purely irreversible stage of pulmonary fibrosis (including IPF) in humans.

Dex (dexamethasone) was selected as a positive control in this study to investigate the effects of the compounds of the present disclosure (e.g., the active drugs listed in Table 9) on the control of the development and progression of fibrosis in the early inflammatory stage in a bleomycin-induced mouse lung disease model. Representative compounds of the present disclosure are of the following structure (BS for short):

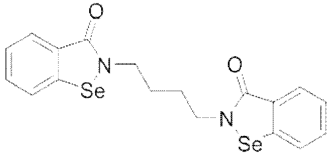

Meanings of abbreviations:
Model stands for a model group, Dex for dexamethasone, BSL for a low-dose BS group, BSM for medium-dose BS group, and BSH for a high-dose BS group.

### 2. Information about test sample and control

### 2.1. Test sample

Name: BS

### 2.2. Control

Name: dexamethasone (Dex), lot No. D107196 (CAS: 2392-39-4)
Strength and purity: 250 mg, 98%; appearance and physicochemical property: white powder Manufacturer: Shanghai Aladdin Biochemical Technology Co., Ltd.

### 2.3. Drug for induction

Name: bleomycin; lot No. B1010423 (CAS: 9041-93-4)
Strength and purity: 25 mg, 1.5-2.0 units/mg
Appearance and physicochemical property: white powder
Manufacturer: Shanghai Aladdin Biochemical Technology Co., Ltd.

### 3. Experimental design

### 3.1. Animal screening and grouping

Animals of 24-h modeling were randomly divided into 5 groups according to the table below, 3 animals per control group and 5 animals per test group.

| Group | Administration dosage (mg/kg/dose) | Administration concentration (mg/mL) | Administration volume (mL/kg) |
|---|---|---|---|
| Control group | 0 | 0 | 10 |
| Model | 0 | 0 | 10 |
| Dex | 1-1.5 | 0.1-0.15 | 10 |
| BS | 90 | 9 | 10 |

Animals of 7-d modeling were randomly divided into 7 groups according to the table below, 6 animals per control group and 12 animals per test group.

| Group | Administration dosage (mg/kg/dose) | Administration concentration (mg/mL) | Administration volume (mL/kg) |
|---|---|---|---|
| Control group | 0 | 0 | 10 |
| Model | 0 | 0 | 10 |
| Dex | 1-1.5 | 0.1-0.15 | 10 |
| BSL | 36 | 3.6 | 10 |
| BSM | 90 | 9 | 10 |
| BSH | 180 | 18 | 10 |

### 3.2. Route and frequency of administration

Drug administration began 24 h or 7 d after modeling. BS was administered daily by single gavage administration, and Dex was administered every two days by single intraperitoneal injection. The day of the first administration was counted as D1, the next day as D2, and so forth. The normal groups and Model groups were given 200 µL of normal saline daily by gavage administration.

### 3.3. Dosage selection

The administration regimen for BS was 1 dose/day.

BS has no toxic and side effects when administered by gavage administration at a total dose within the ranges of 36, 90 and 180 mg/kg. Accordingly, the above dosages were selected as the dosages for administration in this experiment.

### 4. Detection indexes

### 4.1. Body weight

All the animals were observed once daily during the experiment for death or dying, feces, ingestion, mental state and behavior and activity. Abnormalities were recorded in time. All the animals were weighed once before grouping and once before each dose.

### 4.2. Organ coefficients

Detection animals: all the animals.

Detection period: after the mice died naturally.

Method: after being euthanized, the animals were dissected, and the heart, liver, spleen, lung and kidney were weighed.

Detection indexes: organ coefficients.

### 4.3. Blood gas analysis

1. After being anaesthetized, a mouse was fixed to a board in a supine position.
2. After the skin was disinfected with alcohol, a midline cut of about 1.5 cm was made downward in the skin of the neck. The connective tissue between the sternohyoid muscle and the sternothyroid muscle was separated along the anterior border of the sternomastoid muscle with a tissue forceps. The pulsating common carotid artery could be seen on the deep right side of the trachea. A common carotid artery of about 1.5 cm in length was separated. The distal end was ligated with silk thread, and the proximal end was clipped with an artery clip and threaded with a silk thread for later use. The mouse board was placed under a dissecting microscope, and a V-shaped centripetal incision was gently made in a site near the distal end of the artery with a microscopy scissor. A PE microcatheter was inserted into the artery and fixed by making a knot with silk thread. The artery clip was removed, and 0.1-0.2 mL of blood was collected with a 1 mL syringe. The proximal end was clipped with an artery clip, and the syringe was removed. The blood was immediately analyzed on an AbboTTi-STAT blood gas microanalyzer.

### 4.4. Inflammatory cell assay of bronchoalveolar lavage fluid

After the mouse was fixed, its trachea and chest cavity were exposed, and the trachea and the esophagus were bluntly separated with an ophthalmic curved forceps. Two sutures were made between the trachea and the esophagus. An intravenous trocar was inserted into the trachea and ligated at the site where it entered the trachea and at its distal end. A 1 mL syringe was connected to draw in 1 mL of pre-cooled PBS and infuse it into the lung tissue. Significant filling in the lung could be seen with the naked eye. The syringe was held for 10 s and withdrawal was performed. The infusion/withdrawal cycle was repeated 3 times. The withdrawn BALF was transferred to a clean EP tube and centrifuged at 4 °C, and the supernatant was collected and preserved at -80 °C. The cells were subjected to routine tests.

### 4.5. Inflammatory factor assay of bronchoalveolar lavage fluid

After the mouse was fixed, its trachea and chest cavity were exposed, and the trachea and the esophagus were bluntly separated with an ophthalmic curved forceps. Two sutures were made between the trachea and the esophagus. An intravenous trocar was inserted into the trachea and ligated at the site where it entered the trachea and at its distal end. A 1 mL syringe was connected to draw in 1 mL of pre-cooled PBS and infuse it into the lung tissue. Significant filling in the lung could be seen with the naked eye. The infusion was stopped for 10 s and withdrawal was performed. The infusion/withdrawal cycle was repeated 3 times. The withdrawn BALF was transferred to a clean EP tube and centrifuged at 4 °C, and the supernatant was collected and preserved at -80 °C. The bronchoalveolar lavage fluid was assayed for inflammatory factors by ELISA.

### 4.6. Assay of lung tissues for hydroxyproline content

(I) Extraction of total protein from liver tissues
   1. Cryopreserved liver tissues were taken out from liquid nitrogen. A 100 mg sample was cut off from each liver tissue sample and placed into a 5 mL centrifuge tube without DNase and RNase. 2 mL of pre-cooled normal saline was added. The tissues were cut into as many pieces as possible and centrifuged at 2000 rpm/min for 2 min, and the supernatant was discarded.
   2. To the precipitate was added 1 mL of RIPA lysis buffer (mixed protease inhibitor Cocktail was added in a ratio of 1:100 before use, and the resulting mixture was preserved on ice). A tissue homogenate was prepared on ice with an electric homogenizer (6000 rpm 30 s × 3), lysed on ice for 30 min, and centrifuged at 4 °C at 16,000 rpm/min for 15 min. The supernatant, the total protein extracted, was transferred to a new EP tube and preserved at -20 °C for later use.
(II) Protein concentration assay by BCA
   1. To a 96-well plate was added 20 µL of each of 0, 125, 250, 500, 1000 and 2000 µg/mL standard BSA protein solutions. To sample wells were added 2 µL of sample, followed by additional 18 µL of PBS to make 20 µL.
   2. A proper BCA working solution was freshly prepared according to the quantity of the standard substance and samples. After being well mixed, the prepared solution was added at 200 µL/well. The plate was incubated at 37 °C for 30 min, and the absorbance at 570 nm was measured on a microplate reader. The sample protein concentration was calculated from the standard curve.
(III) HYP level assay
   1. The desired plate was taken out of an aluminum foil bag that had been equilibrated at room temperature for 20 min. Standard substance wells and sample wells were set. To the standard substance wells were added 50 µL of standard solutions with different concentrations. To the sample wells was added 10 µL of test sample, followed by 40 µL of sample dilution. Nothing was added to the blank well.
   2. To each well, except for the blank well, was added 100 µL of horseradish peroxidase (HRP)-labeled test antibody. The reaction wells were sealed with a plate-sealing film. The plate was incubated in an incubator at 37 °C for 1 h.
   3. The liquid was discarded, and the plate was tap dried. Each well was filled with washing buffer, and the plated was let stand for 1 min. The liquid was discarded, and the plate was tap dried. The washing was repeated 5 times.
   4. To each well was added 50 µL of each of substrates A and B. The plate was incubated in an incubator at 37 °C for 15-30 min, protected from light.
   5. To each well was added 50 µL of terminating solution, and the absorbance was immediately measured at a wavelength of 450 nm.

### 4.7. Lung HE pathological analysis

### 1. Paraffin embedding and sectioning

1) The lung tissue was photographed and then fixed in 10% formaldehyde for 48 h.
2) The lung tissue was completely dehydrated through 105%, 80%, 90%, 95% and 100%.
3) The lung tissue was subjected to clearing in xylene I for 20 min and in xylene II for 60 min.
4) The lung tissue was immersed in soft wax for 1 h, in soft wax for 2 h, and in hard wax for 2 h.
5) The wax-impregnated tissue block was embedded in paraffin. Specifically, a mold was prepared first, and then the tissue was laid flat at the bottom of the mold; after the section face was arranged facedown, the embedding box cover was put on the mold; molten wax was poured into the mold until it overflowed the mold, and then the mold was gently lifted up and laid flat in a freezing platform; after being rapidly cooled and frozen for about 10 min, the mold was took out.
6) Sectioning method

The embedded block to be sectioned was fixed onto a specimen stage with the outer section face of the embedded block parallel to the section of the specimen holder, and a small length of the embedded block was exposed. After the cutter was pushed to the outer edge, the screw of the blade holder was loosened, and a blade was place into the holder in such a way that the angle between the plane of the blade and the section face of the tissue was 15 ° and the upper and lower borders of the embedded block were parallel to the knife edge. The desired thickness of sections was adjusted on the micro-displacement device. In adjusting the thickness, the needle should not be between two marks; otherwise, the sectioning machine could easily broke. The cutter was positioned near the specimen stage. The rotary wheel was rotated by the right hand, and the a brush was held by the left hand to hold the sections at the lower end of the knife edge and to hold the cut wax strip. When a wax strip of a certain length was formed, the rotation was stopped by the right hand, and another brush was held to gently pick up the wax strip. After sectioning, the related parts of the sectioning machine should be wiped clean in time.

### 2. HE staining

1) A flat section was selected for HE staining.
2) The section was dewaxed in xylene I for 20 min.
3) The section was dewaxed in xylene II for 20 min.
4) The section was soaked in absolute ethanol for 10 min.
5) Stepwise rehydrating, in absolute ethanol for 5 min, in 90% ethanol for 5 min, in 80% ethanol for 5 min, and in 100% ethanol for 5 min; and a water wash.
6) Staining with hematoxylin for 3-5 min. A water wash.
7) Differentiating with 1% HCl-105% ethanol for 5-10 s. A water wash, and bluing.
8) Staining with eosin for 30-60 s. A water wash.
9) Washing with 80% ethanol for 3-5 s, with 95% ethanol for 3-5 s, and with absolute ethanol for 3-5 s.
10) Coverslipping with neutral resin, observing under a microscope, and photographing.

HE sectioning: 3 sections were consecutively made; flat sections of complete tissue were selected for HE staining; each of the sections was photographed in 3 randomly selected fields of view at 200× magnification. The obtained images were scored.

### 5. Data acquisition and statistical analysis

### 5.1. Data acquisition

The results and data obtained from the assays and observation required by the protocol were recorded by hand in proper tables, or the data were directly acquired by computer.

### 5.2. Statistical analysis

The measurement data were expressed as means ± standard deviations. All data were processed using the Graphpad prism 5.0 statistical software. A Masson analysis was performed by analyzing the total area of the 3 fields of view and the collagen positive area using Image J. The effects of the blue nuclei could be excluded by setting a threshold. CVF% was given by dividing the collagen positive area by the total area.

### 6. Results

### Part I. Analysis of body weight changes and organ coefficients in each group after BS administration

### 6.1. Body weight

In this experiment, 105 C57 mice were randomly selected and subjected to organ drip administration of a single dose of bleomycin at 2.5 mg/kg for 24 h or 7 d for induction. After 7 days of induction, 3 model mice were randomly selected and dissected to determine whether the modeling was successful. After modeling, the survival of the mice was recorded. The living states of the mice were observed and their body weight was measured at a fixed time.

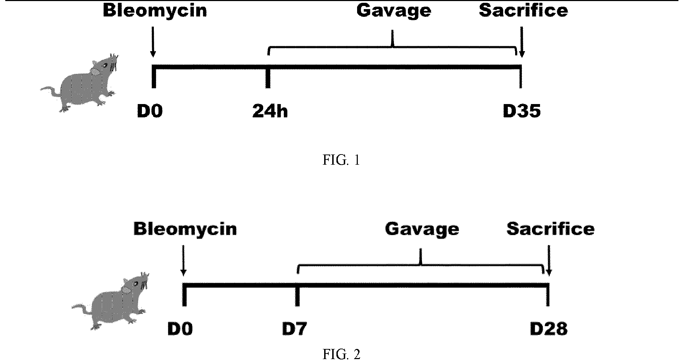

A schematic diagram of the 24-h building of animal models and administration is shown in FIG. 1. After 24 h of induction, the mice were randomly divided into a Model group, a Dex group and a BS group, 5 mice per group. A normal control group comprised 3 mice.

A schematic diagram of the 7-d building of animal models and administration is shown in FIG. 2. After 7 d of induction, the mice were randomly divided into a Model group, a Dex group, a BSL group, a BSM group and a BSH group, 12 mice per group. A normal control group comprised 6 mice.

**Table 1. Changes in body weight of mice of 24-h modeling**

| Days | D1 | D7 | D14 | D21 | D28 | D35 |
|---|---|---|---|---|---|---|
| Control | 23.10±1.22 | 23.33±0.86 | 24.47±0.25 | 24.70±0.10 | 25.03±0.06 | 25.13±0.06 |
| Model | 20.40±0.85 | 19.74±0.93 | 18.96±1.12 | 18.78±1.03 | 18.48±1.03 | 18.22±1.01 |
| Dex | 19.92±1.38 | 20.02±0.96 | 19.24±0.95 | 18.90±0.78 | 18.56±0.71 | 18.36±0.66 |
| BS | 20.34±1.01 | 19.50±1.35 | 18.84±1.70 | 18.52±1.75 | 18.30±1.60 | 18.02±1.75 |

During the period of housing, the mice of the control group had soft and smooth hair and good mental states, and exhibited normal activity and quick responses to stimulation. The mice of the Model group had dull and dry hair and poor mental states and exhibited little activity and slow responses to stimulation. The BS groups of various doses were more active than the Model group.

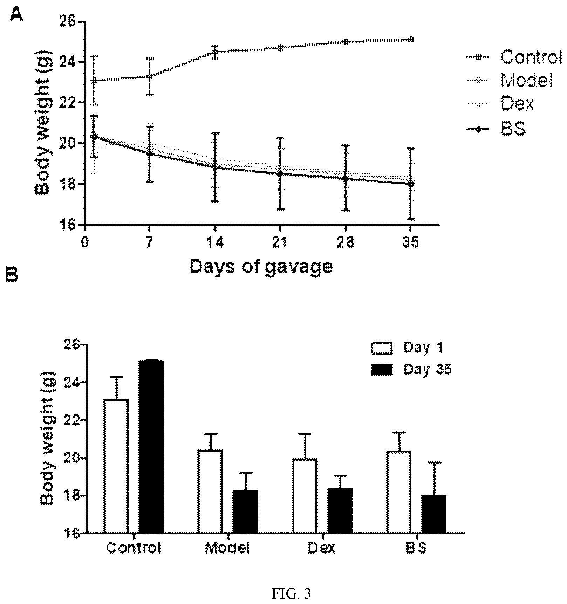

The changes in body weight of the mice in the 24-h modeling groups are shown in FIG. 3. A of FIG. 3 is a graph showing changes in body weight of the mice in each group during the administration. B of FIG. 3 shows the rates of change in body weight of the mice in each group during the administration. C of FIG. 3 is a graph showing comparisons between the body weight of the mice in the groups on day 1 of the administration and that on day 35 of the administration.

The changes in body weight of the mice in each group are shown in A of FIG. 3 and in Table 1. The body weight of the mice in each administration group, except for the control group, decreased. The mice in the Model group had poor mental states and had great losses of body weight. Their body weight decreased from 20.40 ± 0.85 g to 18.22 ± 1.01 g-by 12.25%. On day 35 of the administration, the mice of the control group weighed 25.13 ± 0.06 g, the mice of the Model group weighed 18.22 ± 1.01 g, the mice of the Dex group weighed 18.36 ± 0.66 g, and the mice of the BS group weighed 18.02 ± 1.75 g.

The rates of change in body weight of the mice in the groups are shown in B of FIG. 3. The body weight of the control group increased by 8.10%, the body weight of the Model group decreased by 12.25%, the body weight of the Dex group decreased by 8.48%, and the body weight of the BS group decreased by 13.58%, from day 1 to day 35 of the administration.

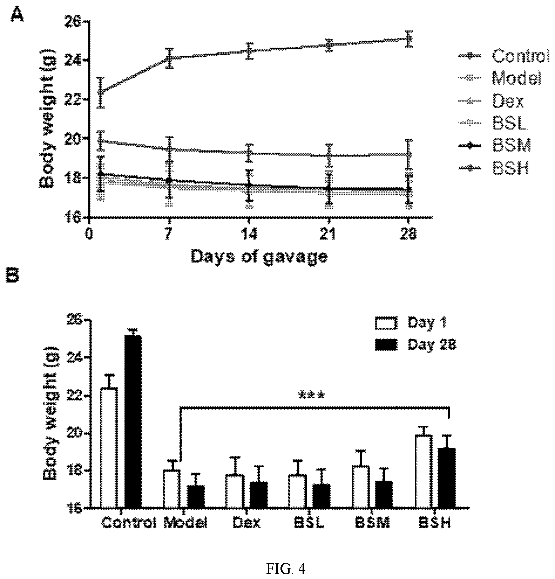

The changes in body weight of the mice in the 7-d modeling groups are shown in FIG. 4. A of FIG. 4 is a graph showing changes in body weight of the mice in each group during the administration. B of FIG. 4 shows the rates of change in body weight of the mice in each group during the administration. C of FIG. 4 is a graph showing comparisons between the body weight of the mice in the groups on day 1 of the administration and that on day 28 of the administration.

**Table 2. Changes in body weight of mice of 7-d modeling**

| Days | D1 | D7 | D14 | D21 | D28 |
|---|---|---|---|---|---|
| Control | 22.37±0.74 | 24.10±0.46 | 24.47±0.39 | 24.75±0.29 | 25.10±0.40 |
| Model | 18.02±0.52 | 17.67±0.67 | 17.34±0.76 | 17.24±0.60 | 17.19±0.63 |
| Dex | 17.79±0.89 | 17.61±1.02 | 17.45±0.96 | 17.43±0.92 | 17.38±0.85 |
| BSL | 17.80±0.72 | 17.49±0.84 | 17.37±0.80 | 17.23±0.76 | 17.23±0.82 |
| BSM | 18.20±0.87 | 17.89±0.92 | 17.63±0.79 | 17.46±0.73 | 17.41±0.72 |
| BSH | 19.88±0.47 | 19.45±0.62 | 19.26±0.44 | 19.13±0.54 | 19.19±0.71 |

The changes in body weight of the mice in each group are shown in A of FIG. 4 and in Table 2. The body weight of the mice in each administration group, except for the control group, decreased. The mice in the Model group had poor mental states and had losses of body weight. Their body weight decreased from 18.02 ± 0.52 g to 17.19 ± 0.63 g-by -4.85%. On day 28 of the administration, the mice of the control group weighed 25.10 ± 0.40 g, the mice of the Model group weighed 17.19 ± 0.63 g, the mice of the Dex group weighed 17.38 ± 0.85 g, the mice of the BSL group weighed 17.23 ± 0.82 g, the mice of the BSM group weighed 17.41 ± 0.72 g, and the mice of the BSH group weighed 19.19 ± 0.71 g.

The rates of change in body weight of the mice in the groups are shown in B of FIG. 4. The body weight of the control group increased by 10.91%, the body weight of the Model group decreased by 4.85%, the body weight of the Dex group decreased by 2.42%, the body weight of the BSL group decreased by 3.39%, the body weight of the BSM group decreased by 4.53%, and the body weight of the BSH group decreased by 3.62%, from day 1 to day 28 of the administration. In C of FIG. 4 are shown comparisons between the body weight of the mice in the groups on day 1 of the administration and that on day 28 of the administration.

### 6.2. Organ coefficients

The lung coefficient can dynamically reflect the progression of lung diseases. As pulmonary inflammation and fibrosis progress, lung exudation increases, the water content increases, various proteins infiltrate lung tissues, and the collagen content of the lung increases, which lead to increases in the weight of the lung and also the lung coefficient. The lung coefficients of the mice in the Model group significantly increased due to inflammation and extracellular matrix deposition, suggesting the successful building of the pulmonary fibrosis mouse models. During the experiment, the modelled mice showed the symptoms of pulmonary fibrosis and increases in the spleen coefficients caused by Model as an external stimulus.

After the administration to the mice in the 24-h modeling groups was completed, the lung and spleen coefficients of the animals in the groups were recorded and analyzed, as shown in Table 3. Significant changes are observed in the lung coefficient and spleen coefficient.

**Table 3. Lung and spleen coefficients of mice in 24-h modeling groups**

| Group | Control | Model | Dex | BS |
|---|---|---|---|---|
| Lung coefficient (%) | 0.42±0.06 | 0.99±0.06 | 0.93±0.09 | 0.89±0.04 |
| Spleen coefficient (%) | 0.27±0.04 | 0.42±0.03 | 0.41±0.06 | 0.37±0.02 |

**Table 4. Organ coefficients of mice in 7-d modeling groups**

| Group | Control | Model | Dex | BSL | BSM | BSH |
|---|---|---|---|---|---|---|
| Lung coefficient (%) | 0.43±0.04 | 0.99±0.05 | 0.96±0.08 | 0.97±0.07 | 0.90±0.09 | 0.88±0.05 |
| Spleen coefficient (%) | 0.25±0.03 | 0.44±0.04 | 0.43±0.04 | 0.40±0.07 | 0.40±0.05 | 0.39±0.02 |

After the administration to the mice in the 7-d modeling groups was completed, the lung and spleen coefficients of the animals in the groups were recorded and analyzed, as shown in Table 4. Significant changes are observed in the lung coefficient and spleen coefficient.

BS can ameliorate pulmonary inflammation and fibrosis. The experimental results suggest that BS, when administered at the experimental dose of 180 mg/kg, could significantly decrease the spleen coefficient of the pulmonary fibrosis model mice, and the effect was better than that of Dex, and that BS had a certain inhibitory effect on spleen swelling in mice. Under the conditions of this experiment, BS (36, 90 and 180 mg/kg) has no significant toxic or side effects on mice.

After the administration to the mice in the 24-h modeling groups was completed, the lung and spleen coefficients of the animals in the groups were recorded and analyzed. The lung coefficient results suggest that BS1801, when administered at the experimental dose of 90 mg/kg either alone or in combination with other drugs, could significantly reduce increases in the lung coefficient of the pulmonary fibrosis model mice, and the effect are better than that of Dex. BS can ameliorate pulmonary inflammation and fibrosis. The spleen coefficient results suggest that BS, when administered at the experimental dose of 90 mg/kg, could significantly decrease the spleen coefficient of the pulmonary fibrosis model mice, and the effect was better than that of Dex, and that BS had a certain inhibitory effect on spleen swelling in mice.

After the administration to the mice in the 7-d modeling groups was completed, the lung and spleen coefficients of the animals in the groups were recorded and analyzed. The lung coefficient results suggest that the low, medium and high-dose BS groups showed dose-dependent effects on the lung coefficient. When administered either at the experimental dose of 90 mg/kg or 180 mg/kg alone or at the experimental dose of 90 mg/kg in combination with other drugs, BS could significantly reduce increases in the lung coefficient of the pulmonary fibrosis model mice, and the effect was better than that of Dex. BS can ameliorate pulmonary inflammation and fibrosis. The spleen coefficient results suggest that BS, when administered at the experimental dose of 180 mg/kg, could significantly decrease the spleen coefficient of the pulmonary fibrosis model mice, and the effect was better than that of Dex, and that BS had a certain inhibitory effect on spleen swelling in mice.

### Part II. Blood gas analysis after BS administration

### 6.3. Blood gas analysis

In a blood gas analysis, the H⁺ concentration in the blood and gases (mainly COz and Oz) dissolved in the blood can be determined to directly reflect the function of pulmonary ventilation and the acid-base balance state. They are used to determine in the organism whether the acid-base balance is upset, whether anoxia occurred, the level of anoxia, and so forth. The analysis results are as follows:

**Table 5. Comparisons between the blood gas analysis results of the mice in the 24-h modeling groups after the administration ended**

| Index | Unit | Control | Model | Dex | BS |
|---|---|---|---|---|---|
| pH | mmHg | 7.35±0.07 | 6.80±0.11 | 7.09±0.10 | 7.05±0.08 |
| PCO₂ | mmHg | 35.48±0.76 | 30.10±1.07 | 32.52±2.73 | 30.84±1.21 |
| PO₂ | mmol/L | 109.17±1.65 | 66.06±9.03 | 91.20±4.09 | 91.52±4.83 |
| BEect | mmol/L | -6.33±0.82 | -12.20±1.92 | -9.20±1.30 | -9.40±1.14 |
| HCO₃⁻ | mmol/L | 34.97±0.94 | 20.68±2.91 | 28.28±1.56 | 30.00±1.20 |
| TCO₂ | % | 38.90±0.39 | 27.24±1.96 | 29.32±1.52 | 33.20±1.01 |
| SaO₂ | mmol/L | 93.83±1.60 | 31.80±4.66 | 67.20±5.93 | 79.40±3.71 |
| Lac | mmHg | 1.21±0.02 | 5.05±0.71 | 2.87±0.23 | 3.31±0.34 |

| | | | | | |
|---|---|---|---|---|---|
| *p < 0.05, ** p < 0.01, ***p < 0.001 vs. the Model group. | | | | | |

The results show that BS, when administered at the experimental dose of 90 mg/kg, made significant improvements to the function of pulmonary ventilation and the acid-base balance states in the mice that developed pulmonary fibrosis in the interstitial lung disease models, and the effects were better than those of Dex (1-1.5 mg/kg) in general.

**Table 6. Comparisons between the blood gas analysis results of the mice in the 7-d modeling groups after the administration ended**

| Index | Unit | Control | Model | Dex | BSL | BSM | BSH |
|---|---|---|---|---|---|---|---|
| PH | mmHg | 7.35±0.07 | 6.81±0.20 | 7.10±0.11 | 6.83±0.14 | 6.97±0.13 | 7.18±0.10 |
| PCO₂ | mmHg | 35.48±0.76 | 30.82±1.03 | 33.60±1.92 | 27.45±1.35 | 31.62±1.34 | 32.82±2.02 |
| PO₂ | mmol/L | 109.17±1.65 | 62.63±6.98 | 92.55±5.19 | 70.08±4.42 | 91.47±2.57 | 93.33±2.86 |
| BEect | mmol/L | -6.33±0.82 | -14.00±4.86 | -8.50±1.05 | -15.67±4.13 | -9.83±0.75 | -8.00±1.10 |
| HCO₃⁻ | mmol/L | 34.97±0.94 | 20.52±2.88 | 27.82±1.08 | 22.30±1.51 | 30.57±1.42 | 27.82±0.91 |
| TCO₂ | % | 38.90±0.39 | 23.37±2.82 | 31.70±1.31 | 24.13±6.00 | 32.45±2.30 | 31.38±1.30 |
| SaO₂ | mmol/L | 93.83±1.60 | 34.83±5.27 | 69.00±5.87 | 34.00±4.86 | 75.83±5.00 | 69.50±1.87 |
| Lac | mmHg | 1.21±0.02 | 4.18±0.91 | 2.95±0.36 | 3.95±0.51 | 2.56±0.35 | 2.94±0.22 |

| | | | | | | | |
|---|---|---|---|---|---|---|---|
| *p < 0.05, ** p < 0.01, ***p < 0.001 vs. the Model group. | | | | | | | |

The results show that BS, when administered at the experimental doses of 90 mg/kg and 180 mg/kg, made significant improvements to the function of pulmonary ventilation and the acid-base balance states in the pulmonary fibrosis model mice, and the effects were better than those of Dex (1-1.5 mg/kg) in general.

**Table 7-1. Comparisons between the blood gas analysis results of the mice in the groups after the administration ended**

| Index | Unit | Control | Model | |
|---|---|---|---|---|
| | | / | 24h | 7 days |
| pH | mmHg | 7.35±0.07 | 6.80±0.11 | 6.81±0.20 |
| PCO₂ | mmHg | 35.48±0.76 | 30.10±1.07 | 30.82±1.03 |
| PO₂ | mmol/L | 109.17±1.65 | 66.06±9.03 | 62.63±6.98 |
| BEect | mmol/L | -6.33±0.82 | -12.20±1.92 | -14.00±4.86 |
| HCO₃⁻ | mmol/L | 34.97±0.94 | 20.68±2.91 | 20.52±2.88 |
| TCO₂ | % | 38.90±0.39 | 27.24±1.96 | 23.37±2.82 |
| SaO₂ | mmol/L | 93.83±1.60 | 31.80±4.66 | 34.83±5.27 |
| Lac | mmHg | 1.21±0.02 | 5.05±0.71 | 4.18±0.91 |

**Table 7-2. Comparisons between the blood gas analysis results of the mice in the groups after the administration ended**

| Index | Unit | Dex | | BSM | |
|---|---|---|---|---|---|
| | | 24h | 7 days | 24h | 7 days |
| pH | mmHg | 7.09±0.10 | 7.10±0.11 | 7.05±0.08 | 6.97±0.13 |
| PCO₂ | mmHg | 32.52±2.73 | 33.60±1.92 | 30.84±1.21 | 31.62±1.34 |
| PO₂ | mmol/L | 91.20±4.09 | 92.55±5.19 | 91.52±4.83 | 91.47±2.57 |
| BEect | mmol/L | -9.20±1.30 | -8.50±1.05 | -9.40±1.14 | -9.83±0.75 |
| HCO₃⁻ | mmol/L | 28.28±1.56 | 27.82±1.08 | 30.00±1.20 | 30.57±1.42 |
| TCO₂ | % | 29.32±1.52 | 31.70±1.31 | 33.20±1.01 | 32.45±2.30 |
| SaO₂ | mmol/L | 67.20±5.93 | 69.00±5.87 | 79.40±3.71 | 75.83±5.00 |
| Lac | mmHg | 2.87±0.23 | 2.95±0.36 | 3.31±0.34 | 2.56±0.35 |

The blood gas analysis results directly reflect the respiratory function and the acid-base balance states of the pulmonary fibrosis mice. Under the conditions of this experiment, the blood gas analysis results of the mice in the 24-h and 7-d modeling groups are consistent. BS can significantly improve in the blood gas analysis of mice the acidity and alkalinity of the blood, the partial pressure of oxygen, the base excess of interstitial fluid, the actual bicarbonate, the total carbon dioxide, the oxygen saturation and the lactate level, and directly improve the pulmonary function of the pulmonary fibrosis mice.

After 24 h and 7 d of modeling, BS, when administered at the experimental doses of 90 mg/kg and 180 mg/kg, made significant improvements to the function of pulmonary ventilation and the acid-base balance states in the pulmonary fibrosis model mice, and the effects were better than those of Dex (1-1.5 mg/kg) in general.

### Part III. Inflammatory cell assay and inflammatory factor assay of bronchoalveolar lavage fluid

### 6.4. Inflammatory cell assay of bronchoalveolar lavage fluid

Leukocytes mainly serve the function of defense. Different kinds of leukocytes are involved in the organism's defense responses in different ways. In an adult, the leukocyte count is (5-9) × 10^9/L, of which neutrophils make up 0.50-0.100, eosinophils make up 0.005-0.05, basophils make up 0.00-0.01, monocytes make up 0.03-0.08, and lymphocytes make up 0.20-0.40.

Experiments show that in bronchoalveolar lavage fluid and pathological sections of lung tissues in the early stage of pulmonary injuries, infiltration of neutrophils and macrophages significantly increased, and the increase was closely related to the degree of pulmonary fibrosis, which indicates that neutrophils and macrophages play important roles in the process of pulmonary fibrosis. Excessive accumulation of neutrophils and macrophages in the pulmonary vessels, interstitium, and alveolar wall can cause vascular and bronchial injuries, the main causes of which are reactive oxygen species (ROS) and proteolytic enzymes produced. Neutrophils and macrophages produce ROS, which can stimulate the production of TNF-α, IFN-γ and TGF-β and the like that mediate fibroproliferative responses and promote the formation of pulmonary fibrosis.

**Table 8. Absolute inflammatory cell counts in the bronchoalveolar lavage fluid of the 7-d modeling groups**

| Index (10^9/L) | Control | Model | Dex | BSL | BSM | BSH |
|---|---|---|---|---|---|---|
| Leukocyte | 0.17±0.02 | 1.03±0.07 | 0.57±0.02 | 0.87±0.04 | 0.69±0.06 | 0.43±0.04 |
| Neutrophil | 0.01±0.00 | 0.09±0.02 | 0.03±0.01 | 0.08±0.01 | 0.08±0.01 | 0.02±0.01 |
| Lymphocyte | 0.13±0.02 | 0.87±0.06 | 0.46±0.03 | 0.70±0.03 | 0.56±0.05 | 0.34±0.03 |
| Monocyte | 0.01±0.00 | 0.05±0.01 | 0.03±0.01 | 0.04±0.01 | 0.03±0.01 | 0.02±0.00 |

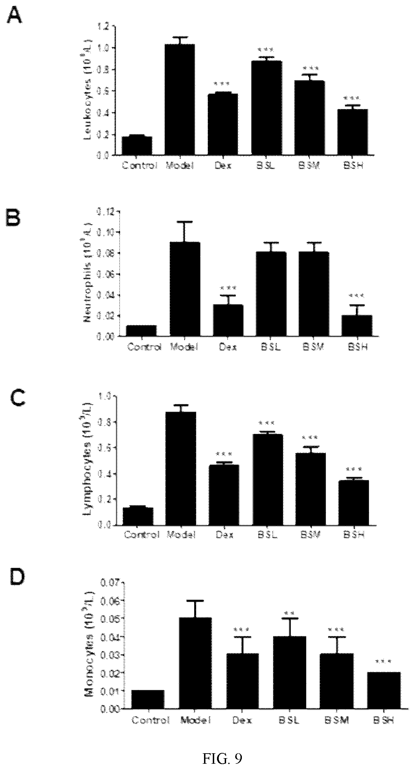

Upon the end of the administration, the leukocytes (lymphocytes, neutrophils and monocytes) in the bronchoalveolar lavage fluids of the mice in the 7-d modeling groups were classified and counted. The analysis results are shown in FIG. 9 and Table 8.

The absolute leukocyte counts, absolute lymphocyte counts and absolute monocyte counts in the bronchoalveolar lavage fluids of the mice in the Dex group, BSL group, BSM group and BSH group were all lower than those of the Model group. In addition, the low, medium and high-dose BS groups showed dose-dependent effects. The absolute neutrophil counts in the bronchoalveolar lavage fluids of the mice in the Dex group and BSH group significantly decreased with respect to the Model group.

**Table 9. Percent reductions in inflammatory cells in the bronchoalveolar lavage fluids of the 7-d modeling groups with respect to the Model group**

| Index (%) | Dex | BSL | BSM | BSH |
|---|---|---|---|---|
| Leukocyte | 45.32 | 16.29 | 32.90 | 58.39 |
| Neutrophil | 68.52 | 7.41 | 16.67 | 74.07 |
| Lymphocyte | 47.51 | 19.54 | 35.63 | 60.73 |
| Monocyte | 46.43 | 25.00 | 42.86 | 57.14 |

The experimental results suggest that after 7 d of modeling, BS, when administered at the experimental dose of 180 mg/kg, could significantly ameliorate pulmonary inflammation and improve the pulmonary function in the pulmonary fibrosis mice of the interstitial lung disease model mice, and the effects were better than those of Dex (1-1.5 mg/kg).

### 6.5. Inflammatory factor assay of bronchoalveolar lavage fluid

Abnormal expression of cytokines and imbalance of cytokine secretion caused by infiltration and activation of inflammatory cells play important roles in the development and progression of interstitial pneumonia. Cytokines that play an important role in pulmonary injuries include: TGF-β, TNF-α, IFN-γ and IL and the like.

**Table 10. Comparisons of cytokines in bronchoalveolar lavage fluid**

| pg/ml | TGF-β | NF-κB | IFN-γ | PPAR-γ |
|---|---|---|---|---|
| Control | 155.77±18.37 | 800.20±136.32 | 91.83±8.31 | 250.84±34.67 |
| Model | 199.60±14.78 | 917.31±149.96 | 33.19±2.47 | 217.09±33.86 |
| Dex | 159.28±11.09 | 818.72±119.21 | 66.48±4.88 | 232.49±39.00 |
| BSL | 188.54±24.55 | 867.20±174.12 | 38.74±4.74 | 229.67±28.11 |
| BSM | 178.54±14.64 | 824.16±155.14 | 53.31±5.29 | 230.31±30.32 |
| BSH | 161.82±20.96 | 759.34±104.53 | 55.70±4.27 | 237.75±24.92 |

| pg/ml | IL-2 | IL-6 | IL-1β | TNF-α |
|---|---|---|---|---|
| Control | 39.80±4.78 | 5.28±0.97 | 4.31±0.29 | 30.21±3.25 |
| Model | 45.29±4.66 | 28.77±18.00 | 12.49±0.85 | 56.12±4.02 |
| Dex | 41.67±4.69 | 12.84±1.02 | 9.59±0.90 | 53.21±3.80 |
| BSL | 43.46±7.51 | 14.41±1.33 | 10.26±1.29 | 51.98±4.37 |
| BSM | 40.84±3.88 | 13.13±1.03 | 6.50±1.38 | 49.57±5.97 |
| BSH | 40.37±5.25 | 9.47±1.96 | 4.78±0.69 | 43.24±7.14 |

**Table 11. Percent reductions in cytokines in bronchoalveolar lavage fluid with respect to the Model group**

| % | TGF-β | NF-κB | IFN-γ | PPAR-γ |
|---|---|---|---|---|
| Dex | 15.87 | 10.75 | -100.30 | -7.09 |
| BSL | 0.42 | 5.46 | -16.73 | -5.79 |
| BSM | 2.87 | 10.15 | -60.63 | -6.09 |
| BSH | 14.53 | 17.22 | -67.81 | -9.52 |

| % | IL-2 | IL-6 | IL-1β | TNF-α |
|---|---|---|---|---|
| Dex | 7.98 | 55.36 | 23.21 | 5.19 |
| BSL | 4.03 | 49.92 | 17.88 | 7.38 |
| BSM | 9.81 | 54.37 | 47.93 | 11.68 |
| BSH | 10.87 | 67.10 | 61.70 | 22.95 |

Previous studies have shown that Dex inhibits fibroblast proliferation and activation primarily through anti-inflammatory effects. When used alone, BSH has a greater inhibitory effect on inflammatory cells (leucocytes, lymphocytes, monocytes and neutrophils) and proinflammatory factors (NF-κB, TNF-α, IL-1β IL-2 and IL-6) compared to the positive drug Dex for pulmonary fibrosis. The fibrosis-mediated alveolar inflammatory injuries are ameliorated, and the fibroblast activation and proliferation and collagen synthesis, and thus the matrix protein deposition and fibrous tissue generation, are inhibited, and therefore pulmonary fibrosis is ameliorated.

In bleomycin-induced pulmonary fibrosis injuries, significant infiltration of macrophages, neutrophils and lymphocytes is found in inflammatory regions. These inflammatory cells can also produce a large amount of cytokines such as TGF-β, TNF-α, IFN-γ and IL and the like, so a complex cytokine network is formed. They regulate the proliferation and apoptosis of alveolar epithelial cells and interstitial fibroblasts, such that the deposition of extracellular matrix gradually increases, the activation of the fibrinolytic system is inhibited, and the formation of pulmonary fibrosis is finally promoted.

### Part IV. Assay of lung tissues for hydroxyproline content after BS administration

### 6.6. Assay of lung tissues for hydroxyproline content

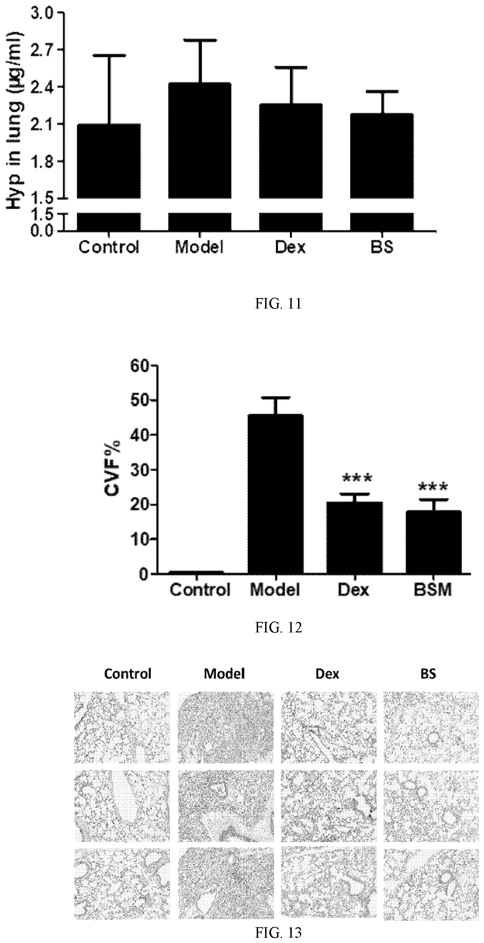

The liver homogenates of 3 animals from each of the 24-h modeling groups were assayed for hydroxyproline content. The results are shown in Table 12 and FIG. 11. After administration, the Dex group and BS groups were lower than the Model group in hydroxyproline content.

**Table 12. The hydroxyproline content in the lungs of mice on day 35 of administration after 24-h modeling**

| | Control | Model | Dex | BS |
|---|---|---|---|---|
| HYP(µg/ml) | 2.09±0.57 | 2.42±0.36 | 2.25±0.31 | 2.17±0.19 |

### Part V. Lung HE and Masson pathological analysis after BS administration

### 6.7. HE pathological analysis

The general standard Ashcroft scoring method for pulmonary fibrosis tissues was adopted in HE scoring. The HE pathological analysis is an outcome evaluation for irreversible pulmonary fibrosis that develops in interstitial lung disease models.

For 5 animals from each of the 24-h modeling groups, a total of 15 HE fields of view were scored. The results are shown in Table 13 and FIG. 13. The control group scored 0.00 ± 0.00, the Model group scored 6.00 ± 0.65, the Dex group scored 2.53 ± 0.52, and the BS group scored 3.00 ± 0.65. The Dex group and BS group scored significantly less than the Model group. The mean of fibrosis scores decreased from a level of 6 points to about 3 points. According to the scoring criteria, a score below 3 points indicates there has been no significant structural change accompanying in the lung tissues. Therefore, these results suggest that the occurrence of pathological structural changes in lung tissues can be effectively controlled in the BS group.

**Table 13. Pulmonary fibrosis scoring analysis for the 24-h modeling groups**

| Group | Control group | Model | Dex | BS |
|---|---|---|---|---|
| HE score | 0.00±0.00 | 6.00±0.65 | 2.53±0.52 | 3.00±0.65 |

**Table 14. 24-h pulmonary fibrosis score and benefit rate analysis**

| HE score and benefit rate | ≥3 points | | ≥4 points | | ≥5 points | |
|---|---|---|---|---|---|---|
| Dex group | 8/15 | 53.3% | 0/15 | 0% | 0/15 | 0% |
| BS group | 12/15 | 80.0% | 3/15 | 20.0% | 0/15 | 0% |

An HE score up to 5 points is considered as the occurrence of clear lung tissue damage and the occurrence of fibrotic tissues such as fibrotic cords. The pulmonary fibrosis score and benefit rate analysis is shown in Table 14 and FIG. 12. After 35 days of administration, no clear lung tissue damage occurred in the Dex group or BS group. The results suggest that in the early stage of pulmonary fibrosis (24-h model), BS, when administered at the dose of 90 mg/kg, could significantly reduce structural changes in lung tissues and reduce infiltration of inflammatory cells and ameliorate pulmonary fibrosis.

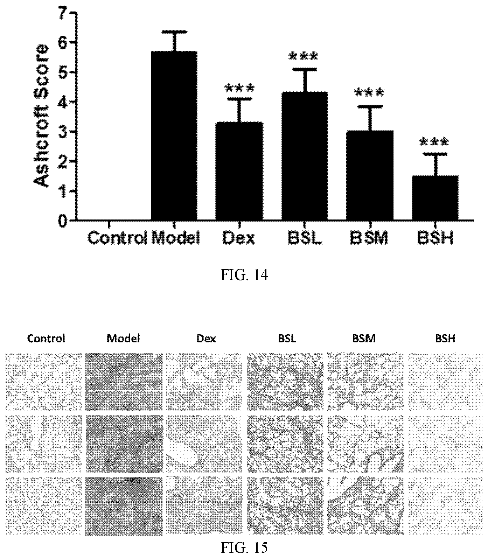

For 6 animals from each of the 7-d modeling groups, a total of 18 HE fields of view were scored. The results are shown in Table 15 and FIG. 15. The control group scored 0.00 ± 0.00, the Model group scored 5.67 ± 0.69, the Dex group scored 3.28 ± 0.83, the BSL group scored 4.28 ± 0.83, the BSM group scored 3.00 ± 0.84, and the BSH group scored 1.48 ± 0.78. The Dex group, BSL group, BSM group and BSH group had significant lower pulmonary fibrosis scores than the Model group. These results suggest that the occurrence of pathological structural changes in lung tissues can be effectively controlled in the BSH group.

**Table 15. Pulmonary fibrosis scoring analysis for the 7-d modeling groups**

| Group | Control group | Model | Dex | BSL | BSM | BSH |
|---|---|---|---|---|---|---|
| HE score | 0.00±0.00 | 5.67±0.69 | 3.28±0.83 | 4.28±0.83 | 3.00±0.84 | 1.48±0.78 |

**Table 16. 7-d pulmonary fibrosis score and benefit rate analysis**

| HE score and benefit rate | ≥4 points | | ≥5 points | | ≥6 points | |
|---|---|---|---|---|---|---|
| Dex group | 7/18 | 38.9% | 1/18 | 5.6% | 0/18 | 0% |
| BSL group | 15/18 | 83.3% | 6/18 | 33.3% | 1/18 | 5.6% |
| BSM group | 5/18 | 27.8% | 1/18 | 5.6% | 0/18 | 0% |
| BSH group | 0/18 | 0% | 0/18 | 0% | 0/18 | 0% |

An HE score up to 5 points is considered as the occurrence of clear lung tissue damage and the occurrence of fibrotic tissues such as fibrotic cords. The pulmonary fibrosis score and benefit rate analysis is shown in Table 16 and FIG. 14. In the BSM group, the probability of clear lung tissue damage was 5.6% (1/18), and the probability of pathological tissue change was 27.8% (5/18). The two kinds of probability for the clinical pulmonary fibrosis drug Dex group were 5.6% (1/18) and 38.9% (7/18), respectively. The BSM group and Dex group showed substantially equivalent effects. The BSH group did not show clear lung tissue damage or pathological tissue change. Since pathological changes in lung tissues are significant basis of pulmonary function loss, these results suggest that during the formation of pathological changes in lung tissues, the BS drug had the most significant functional effect in controlling the occurrence of pathological changes.

The control group had clear lung tissue structures and uniform cytoplasm and nuclei, and showed no significant inflammatory infiltration and collagen fiber deposition. In the mice of the Model group, the lung tissue structures were significantly and seriously changed and damaged, there was infiltration of large quantities of inflammatory cells, the lung tissue septa thickened or was even filled, and there was a large area with fibrotic changes in the lungs. The Dex group had much better lung structures than the Model group in general, but there were still changes in the lung tissue structures; there was damage and infiltration of inflammatory cells to the lung tissue structures, and an area with fibrotic changes could be seen in the lungs. The BSL group had relatively better lung structures than the Model group in general, but there were still infiltration of large quantities of inflammatory cells; the lung tissue structures were badly damaged, and a large area with fibrotic changes could be seen in the lungs. The BSM group had much better lung structures than the Model group in general, but there were still changes in the lung tissue structures; there was damage and infiltration of inflammatory cells to the lung tissue structures, and an area with fibrotic changes could be seen in the lungs. The BSH group had substantially complete lung tissue structures, and a few areas with pulmonary fibrosis and inflammatory cells could be seen. The results suggest that in the formation stage of pulmonary fibrosis (7-d model), BSL (36 mg/kg), BSM (90 mg/kg) and BSH (180 mg/kg) could significantly reduce structural changes in lung tissues and reduce infiltration of inflammatory cells and ameliorate pulmonary fibrosis. When administered at the dose of 180 mg/kg, BSH had a significant synergetic effect against ECM deposition.

In the early stage of pulmonary fibrosis (24-h model), BS, when administered at the dose of 90 mg/kg, could significantly reduce structural changes in lung tissues and reduce infiltration of inflammatory cells and ameliorate pulmonary fibrosis.

In the formation stage of the pulmonary fibrosis model (7-d model), BSL (36 mg/kg), BSM (90 mg/kg) and BSH (180 mg/kg) could significantly reduce structural changes in lung tissues and reduce infiltration of inflammatory cells and ameliorate pulmonary fibrosis. When administered at the dose of 180 mg/kg, BSH had a significant synergetic effect against ECM deposition.

The embodiments of the present disclosure have been described above. However, the present disclosure is not limited thereto. Any modification, equivalent, improvement and the like made without departing from the spirit and principle of the present disclosure shall fall within the protection scope of the present disclosure.

## Claims

1. Use of benzisoselenazole derivatives for the manufacturing of anti-coronavirus medicaments or medicaments for the treatment of diseases caused by coronaviruses, wherein the benzisoselenazole derivatives are compounds of formula (I), or stereoisomers, pharmaceutically acceptable salts, solvates, prodrugs or active metabolites thereof, 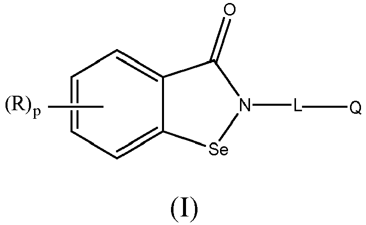 wherein R is selected from hydrogen, cyano, hydroxyl, sulfydryl, halogen, nitro, amino, COOH, SO₃H, C₁₋₁₂ alkyl, C₃₋₁₂ cycloalkyl, C₁₋₁₂ alkoxy, C₁₋₁₂ alkylthio, C₂₋₁₂ alkenyl, C₁₋₁₂ alkylamido, C₁₋₁₂ alkylacyloxy, 3- to 14-membered heterocyclyl, C₆₋₁₄ aryl and 5- to 14-membered heteroaryl, wherein the C₁₋₁₂ alkyl, C₃₋₁₂ cycloalkyl, C₁₋₁₂ alkoxy, C₁₋₁₂ alkylthio, C₁₋₁₂ alkylamido, C₁₋₁₂ alkylacyloxy, 3- to 14-membered heterocyclyl, C₆₋₁₄ aryl and 5- to 14-membered heteroaryl are optionally substituted with one or more substituents selected from the following: cyano, hydroxyl, sulfhydryl, halogen, amino, nitro, oxo, thio, COOH, SO₃H, C₁₋₁₂ alkyl, C₃₋₁₂ cycloalkyl, C₁₋₁₂ alkoxy, C₂₋₁₂ alkenyl, 3- to 14-membered heterocyclyl, C₆₋₁₄ aryl and 5- to 14-membered heteroaryl;
p is an integer from 0 to 4;
L is selected from ^{∗}-(CH₂)ₙ-#, ^{∗}-(CH₂)ₙ-NH-(CH₂)ₙ-#, ^{∗}-(CH₂)ₙ-NR^{N}-(CH₂)ₙ-#, ^{∗}-(CH₂)ₙ-CH(OH)-(CH₂)ₙ-#, ^{∗}-(CH₂)ₙ-NH-C(O)-(CH₂)ₙ-#, ^{∗}-(CH₂)ₙ-C(O)-NH-(CH₂)ₙ-#, ^{∗}-(CH₂)ₙ-O-(CH₂)ₙ-#, ^{∗}-(CH₂)ₙ-O-C(O)-(CH₂)ₙ-#, ^{∗}-(CH₂)ₙ-C(O)-O-(CH₂)ₙ-#, ^{∗}-(CH₂)ₙ-C(O)-(CH₂)ₙ-#, ^{∗}-(CH₂)ₙ-C(O)-C(O)-(CH₂)ₙ-#, ^{∗}-(CH₂)ₙ-S(O)-(CH₂)ₙ-#, ^{∗}-(CH₂)ₙ-S(O)₂-(CH₂)ₙ-#, ^{∗}-(CH₂)ₙ-C₃₋₈ cycloalkylene-(CH₂)ₘ-#, ^{∗}-(CH₂)ₙ-S-S-(CH₂)ₘ-#, ^{∗}-(CHR^{L})(CH₂)ₙ-S-S-(CH₂)ₘ(CHR^{L})-#, ^{∗}-(CH₂)ₙ-(CHR^{L})-S-S-(CHR^{L})-(CH₂)ₘ-#, phenylene, biphenylene, triphenylene, ^{∗}-phenyl-(CH₂)ₙ-#, ^{∗}-(CH₂)ₙ-phenyl-#, ^{∗}-phenyl-(CH₂)ₙ- phenyl-#, ^{∗}-phenyl-(CH₂)ₙ-S(O)₂-# and ^{∗}-(CH₂)ₙ-S(O)₂-phenyl-#, wherein * represents a linking site for the N atom of the benzisoselenazole moiety, and # represent a linking site for the Q moiety; n is an integer from 0 to 12, m is an integer from 0 to 12, R^{N} represents C₁₋₁₂ alkyl, and R^{L} represents halogen, hydroxyl, amino, carboxyl or sulfhydryl;
Q is selected from H, C₁₋₁₂ alkyl, C₃₋₁₂ cycloalkyl, C₁₋₁₂ alkoxy, C₁₋₁₂ alkylthio, C₁₋₁₂ alkylamido, C₁₋₁₂ alkylacyloxy, C₂₋₁₂ alkenyl, 3- to 14-membered heterocyclyl, C₆₋₁₄ aryl and 5- to 14-membered heteroaryl, wherein the C₁₋₁₂ alkyl, C₃₋₁₂ cycloalkyl, C₁₋₁₂ alkoxy, C₁₋₁₂ alkylamido, C₁₋₁₂ alkylacyloxy, 3- to 14-membered heterocyclyl, C₆₋₁₄ aryl and 5- to 14-membered heteroaryl are optionally substituted with one or more substituents selected from the following: cyano, hydroxyl, sulfhydryl, halogen, amino, nitro, oxo, thio, COOH, SO₃H, C₁₋₁₂ alkyl, C₃₋₁₂ cycloalkyl, C₁₋₁₂ alkoxy, C₂₋₁₂ alkenyl, 3- to 14-membered heterocyclyl, C₆₋₁₄ aryl and 5- to 14-membered heteroaryl.

2. The use as claimed in claim 1, wherein in the compounds of formula (I),
L is selected from ^{∗}-(CH₂)₂-#, ^{∗}-(CH₂)₃-#, ^{∗}-(CH₂)₄-#, ^{∗}-(CH₂)₅-#, ^{∗}-(CH₂)₆-#, ^{∗}-(CH₂)₇-#, ^{∗}-(CH₂)₈-#, ^{∗}-(CH₂)₉-#, ^{∗}-(CH₂)₁₀-#, ^{∗}-(CH₂)₁₁-#, ^{∗}-(CH₂)₁₂-#, cyclopentylene, cyclohexylene, phenylene, biphenylene, triphenylene, ^{∗}-phenyl-(CH₂)ₙ-#, ^{∗}-(CH₂)ₙ-phenyl-#, ^{∗}-phenyl-(CH₂)ₙ-phenyl-#^{∗}, ^{∗}-phenyl-S(O)₂-#, ^{∗}-(CH₂)ₙ-S(O)₂-phenyl-#, ^{∗}-(CH₂)ₙ-NH-(CH₂)ₙ-#, ^{∗}-(CH₂)ₙ-NR^{N}-(CH₂)ₙ-#, ^{∗}-(CH₂)ₙ-CH(OH)-(CH₂)ₙ-#, ^{∗}-(CH₂)ₙ-NH-C(O)-(CH₂)ₙ-#, ^{∗}-(CH₂)ₙ-C(O)-NH-(CH₂)ₙ-#, ^{∗}-(CH₂)ₙ-O-(CH₂)ₙ-#, ^{∗}-(CH₂)ₙ-O-C(O)-(CH₂)ₙ-#, ^{∗}-(CH₂)ₙ-C(O)-O-(CH₂)ₙ-#, ^{∗}-(CH₂)ₙ-C(O)-(CH₂)ₙ-#, ^{∗}-(CH₂)ₙ-C(O)-C(O)-(CH₂)ₙ-#, ^{∗}-(CH₂)ₙ-S(O)-(CH₂)ₙ-#, ^{∗}-(CH₂)ₙ-S(O)₂-(CH₂)ₙ-#, ^{∗}-(CH₂)ₙ-cyclopropylene-(CH₂)ₘ-#, ^{∗}-(CH₂)ₙ-cyclobutylidene-(CH₂)ₘ-#, ^{∗}-(CH₂)ₙ-cyclopentylene-(CH₂)ₘ-#, ^{∗}-(CH₂)ₙ-cyclohexylene-(CH₂)ₘ-#, ^{∗}-(CH₂)ₙ-S-S-(CH₂)ₘ-#, ^{∗}-(CHR^{L})(CH₂)ₙ-S-S-(CH₂)ₘ(CHR^{L})-# and ^{∗}-(CH₂)ₙ-(CHR^{L})-S-S-(CHR^{L})-(CH₂)ₘ-#, wherein * represents a linking site for the N atom of the benzisoselenazole moiety, and # represents a linking site for the Q moiety; n is 0, 1, 2, 3, 4, 5 or 6, m is 0, 1, 2, 3, 4, 5 or 6, R^{N} represents C₁₋₆ alkyl, and R^{L} represents halogen, hydroxyl, amino, carboxyl or sulfhydryl;
Q is selected from H, C₁₋₆ alkyl, C₃₋₇ cycloalkyl, C₁₋₆ alkoxy, C₁₋₆ alkylthio, C₁₋₆ alkylamido, C₁₋₆ alkylacyloxy, C₂₋₆ alkenyl, benzisoselenazolyl, saccharide residue, amino acid residue, R^{e}-NH-R^{f}, 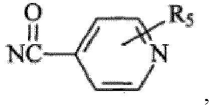 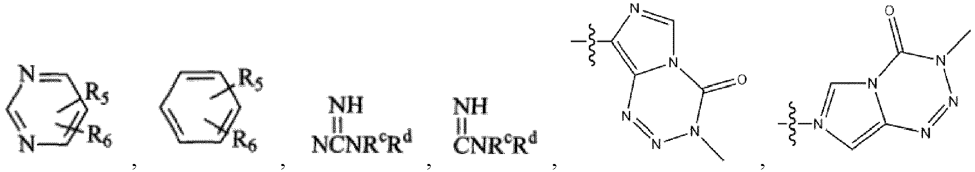 and 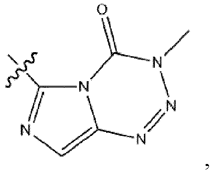 wherein 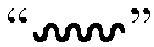 represents a linking group for L;
R₅ and R₆ are each independently selected from hydrogen, halogen, cyano, C₁₋₆ alkyl, C₁₋₆ alkoxy, C₁₋₆ alkylthio, N(C₁₋₆ alkyl)₂, NH(C₁₋₆ alkyl), COOR^{b} and SO₃R^{b};
R^{b} is selected from hydrogen, C₁₋₆ alkyl and C₁₋₆ alkoxy;
R^{c} and R^{d} are each independently selected from hydrogen, C₁₋₆ alkyl and phenyl, or R^{c} and R^{d} together with a nitrogen atom to which they are linked form a nitrogen-containing heterocycle or nitrogen-containing heteroaryl ring;
R^{e} and R^{f} are each independently selected from C₁₋₆ alkyl and C₃₋₇ cycloalkyl;
the C₁₋₆ alkyl, C₃₋₇ cycloalkyl, C₁₋₆ alkoxy, C₁₋₆ alkylthio, C₁₋₆ alkylamido, C₁₋₆ alkylacyloxy, C₂₋₆ alkenyl, C₃₋₇ cycloalkyl, benzisoselenazolyl, saccharide residue and amino acid residue may optionally be substituted with one or more substituents selected from the following: cyano, hydroxyl, sulfhydryl, halogen, amino, nitro, oxo, thio, C₁₋₆ alkyl, C₃₋₇ cycloalkyl and C₁₋₆ alkoxy.

3. The use as claimed in claim 1 or 2, wherein the benzisoselenazole derivatives of formula (I) are compounds of the following formula (I-1): 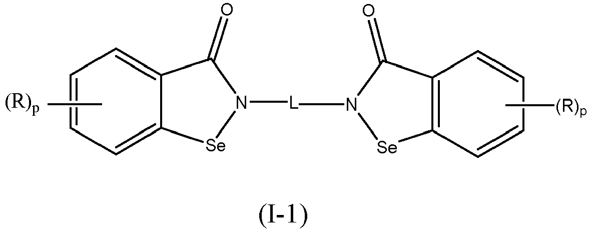 wherein R, L and p each have the definitions given in the compounds of formula (I) as claimed in claim 1 or 2; preferably, the benzisoselenazole derivatives of formula (I-1) are selected from the compounds in Table 1 below:

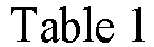

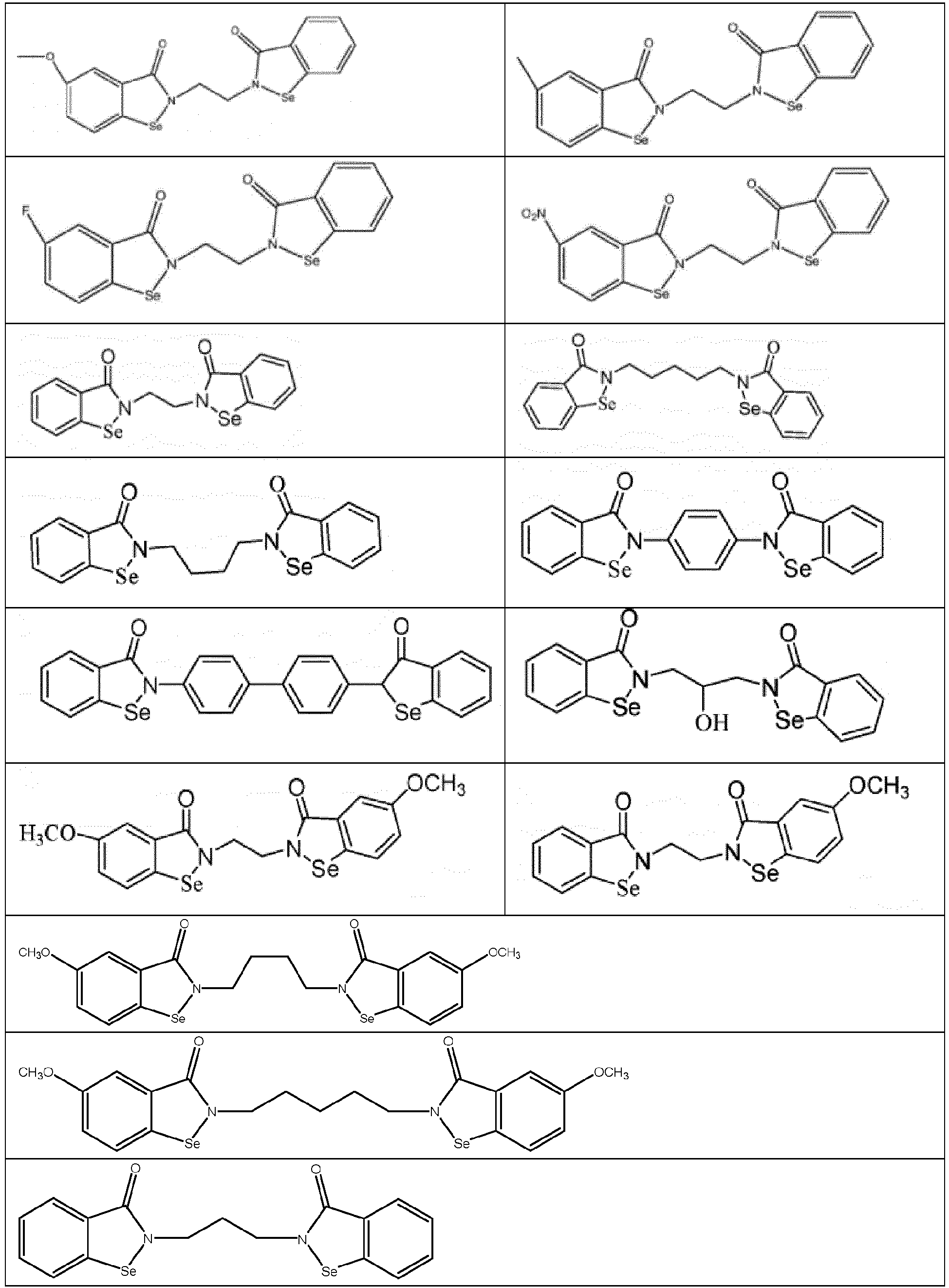

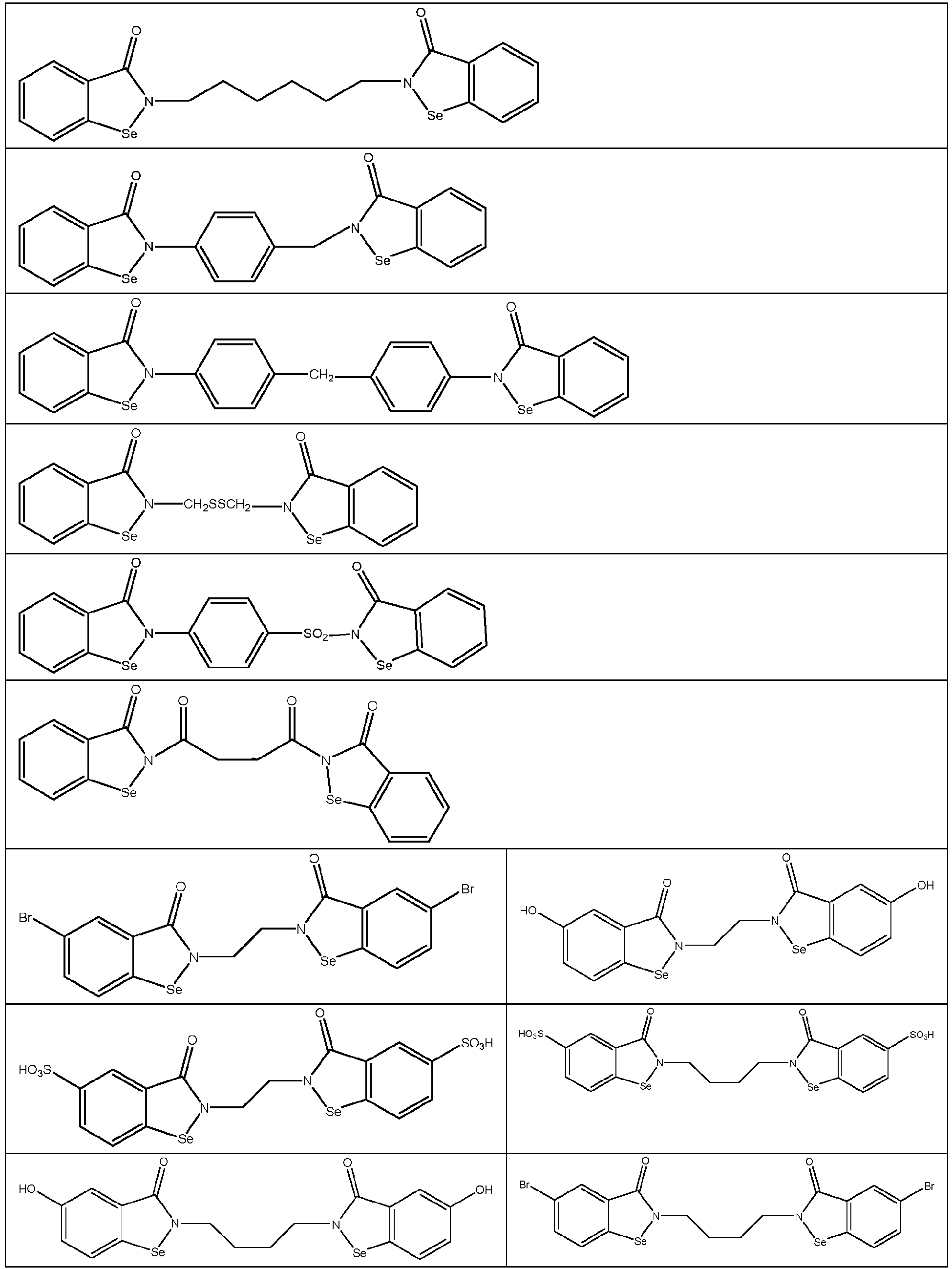

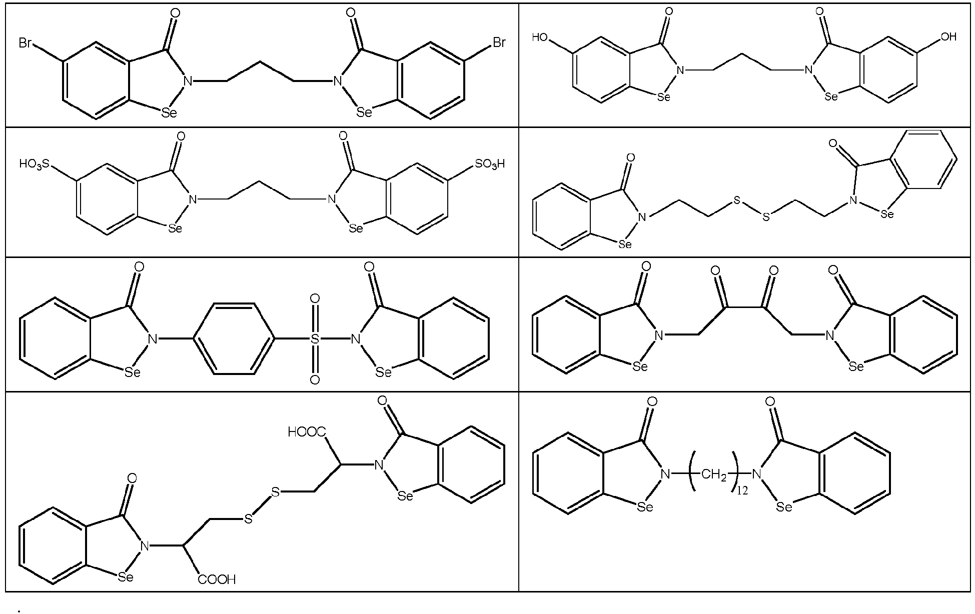

4. The use as claimed in claim 1 or 2, wherein the benzisoselenazole derivatives of formula (I) are compounds of formula (I-2a), formula (I-2b) or formula (I-2c): 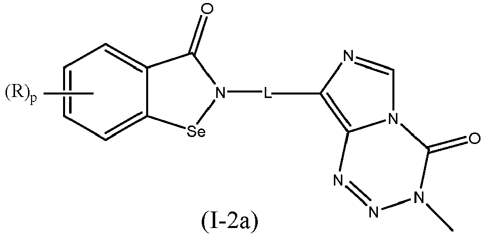 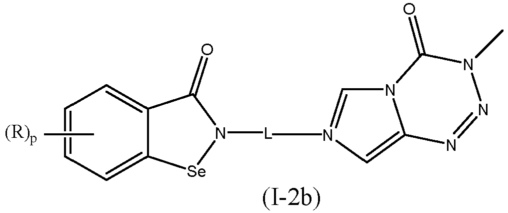 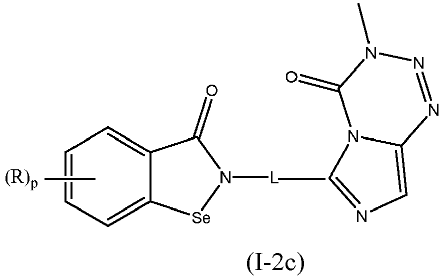 wherein R, L and p each have the definitions given in the compounds of formula (I) as claimed in claim 1 or 2; preferably, the benzisoselenazole derivatives of formula (I-2a), formula (I-2b) or formula (I-2c) are selected from the compounds in Table 2 below:

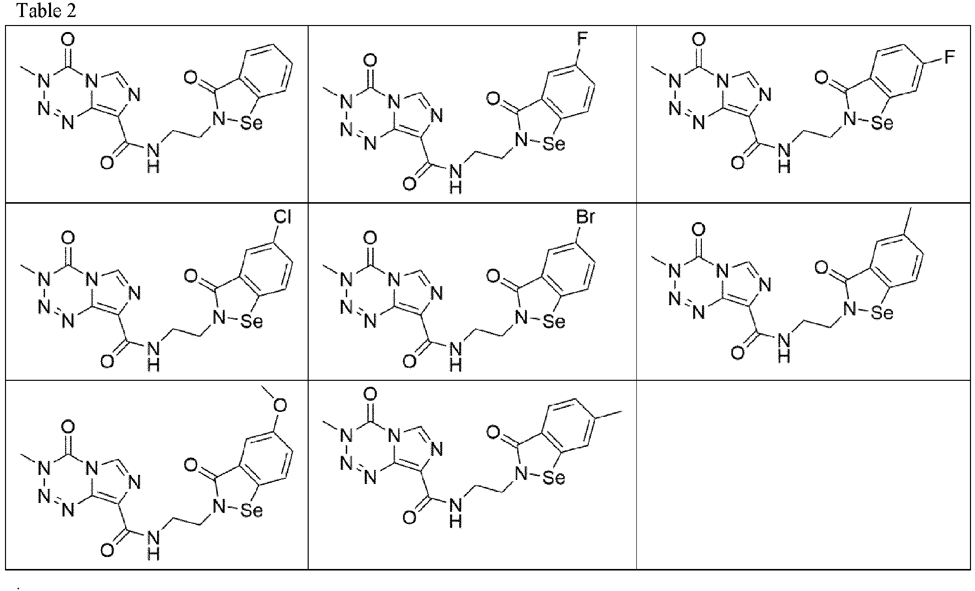

5. The use as claimed in claim 1 or 2, wherein the benzisoselenazole derivatives of formula (I) are compounds of formula (I-3): 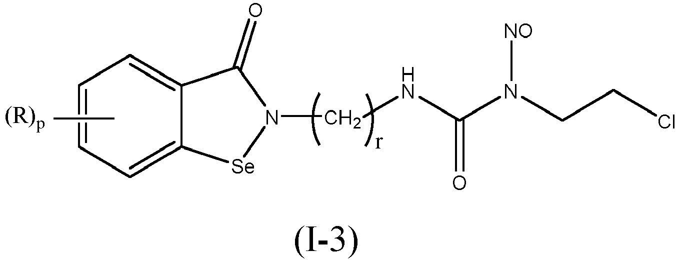 wherein Rand p each have the definitions given in the compounds of formula (I) as claimed in claim 1 or 2, and r is an integer from 0 to 12, preferably an integer from 2 to 4;
preferably, the benzisoselenazole derivatives of formula (I-3) are selected from the compounds in Table 3 below:

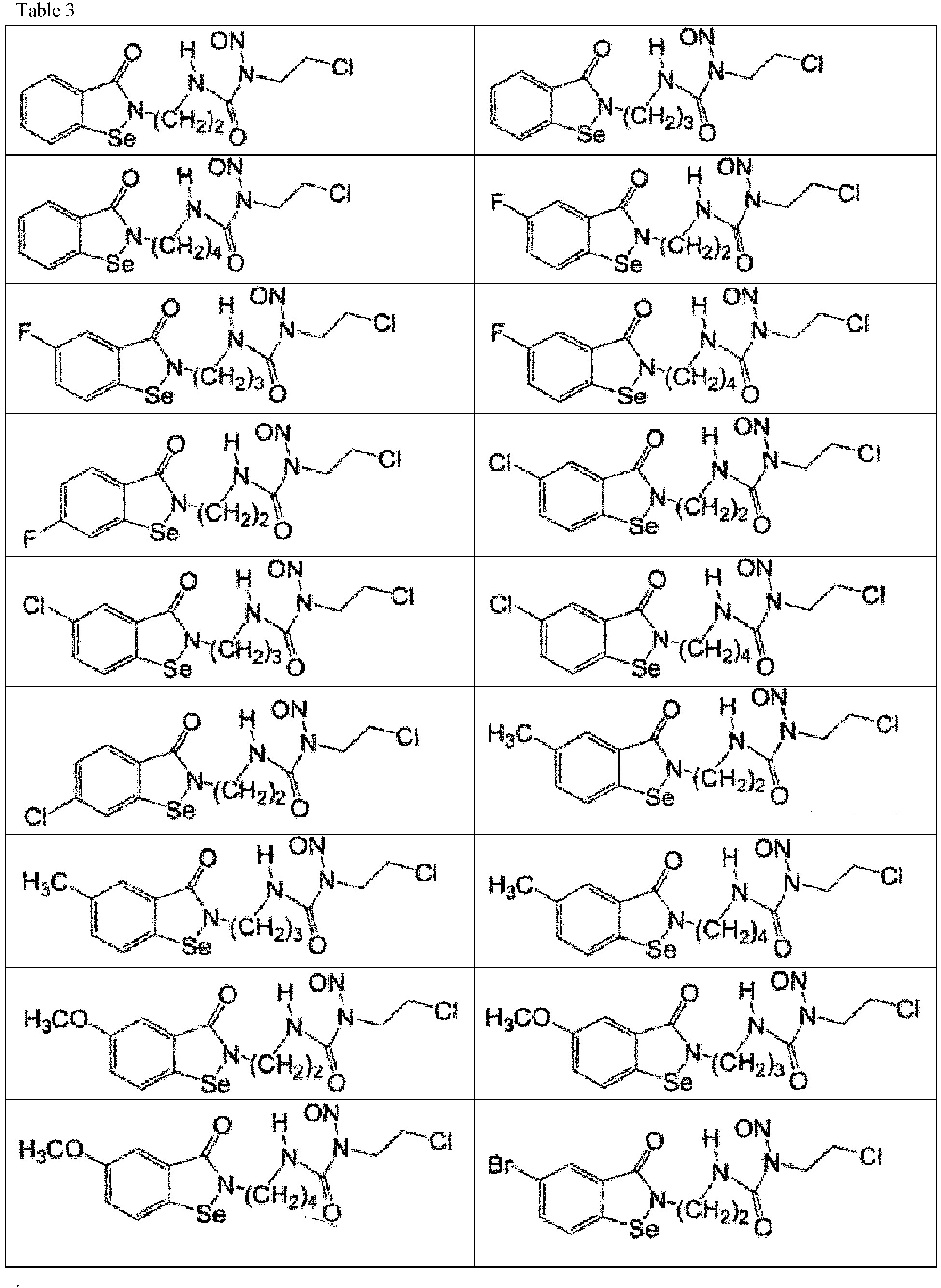

6. The compounds as claimed in claim 1 or 2, wherein the benzisoselenazole derivatives of formula (I) are compounds of formula (I-4): 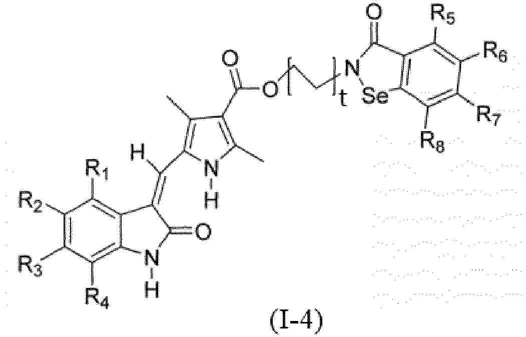 wherein R₁, R₂, R₃, R₄, R₅, R₆, R₇ and R₈ are each independently H, C₁₋₆ alkyl, C₁₋₆ alkoxy or halogen, and t is an integer from 1 to 4, preferably 2 or 3;
preferably, the benzisoselenazole derivatives of formula (I-4) are selected from the compounds in Table 4 below:

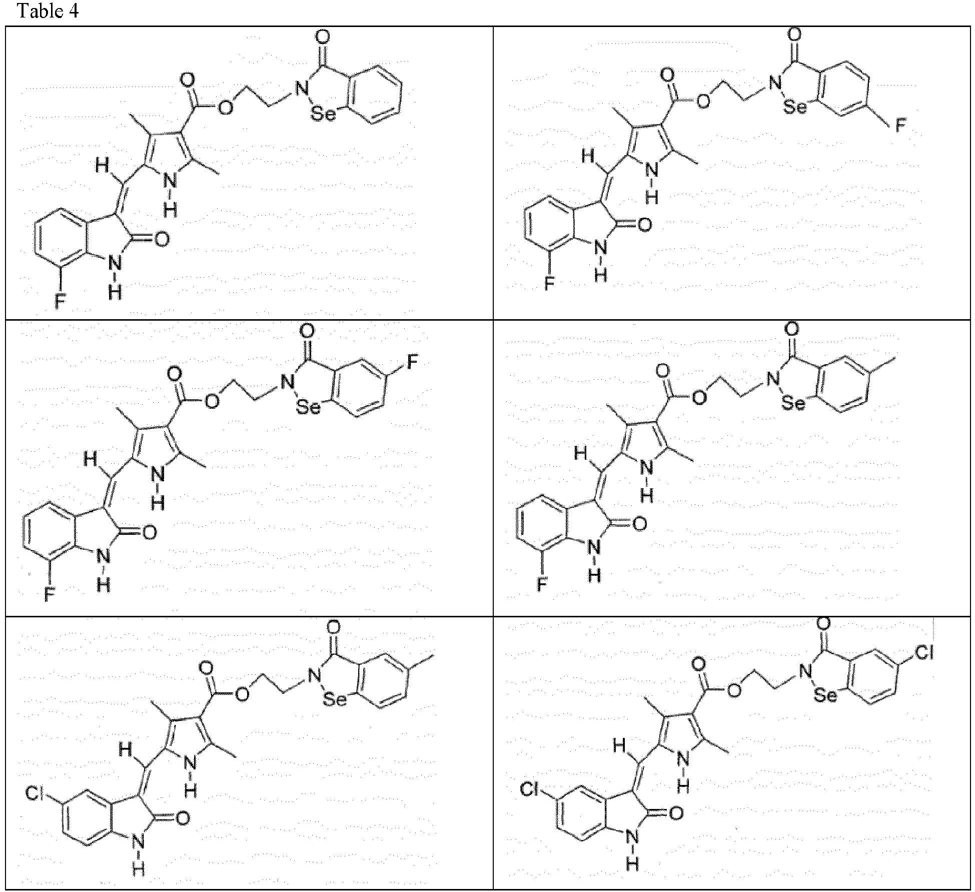

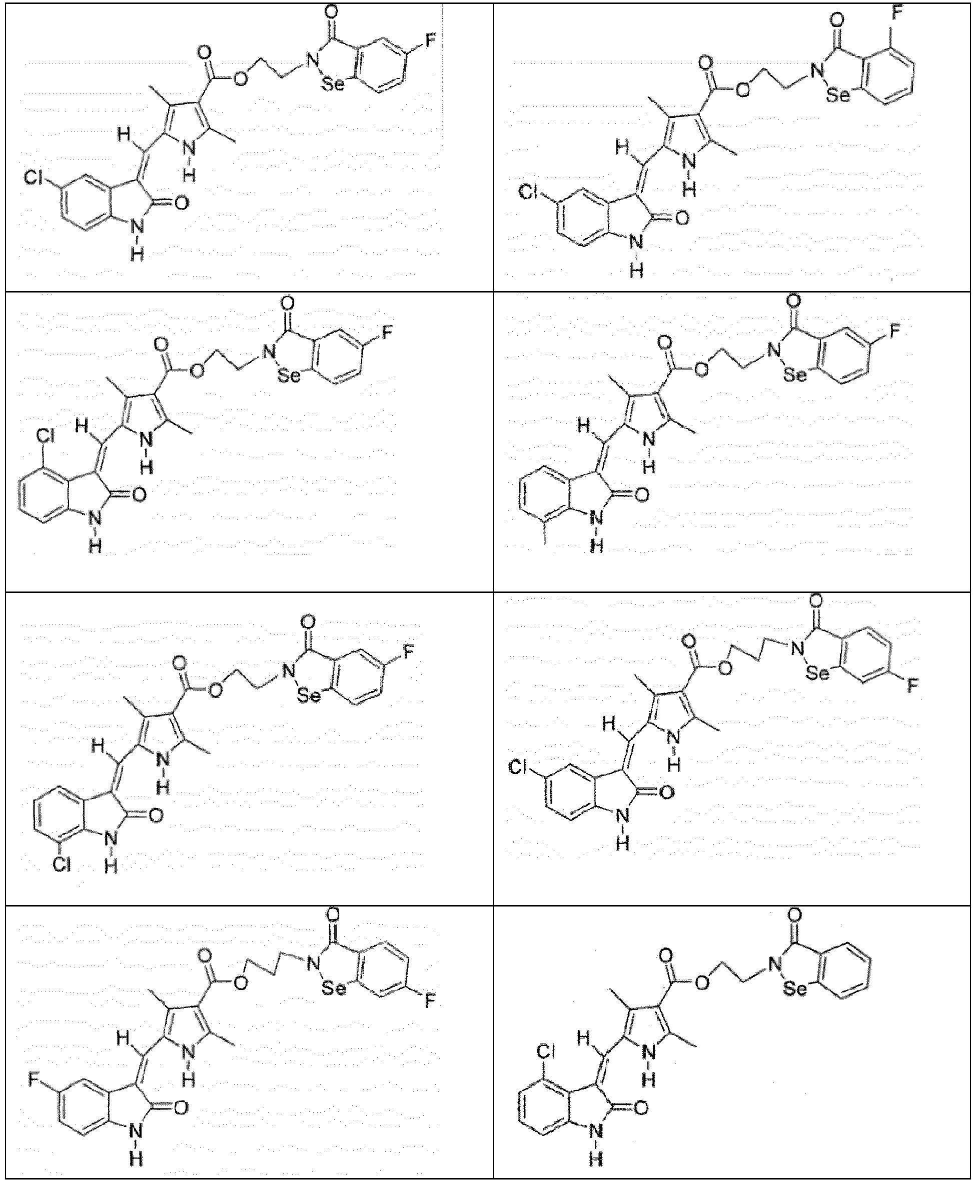

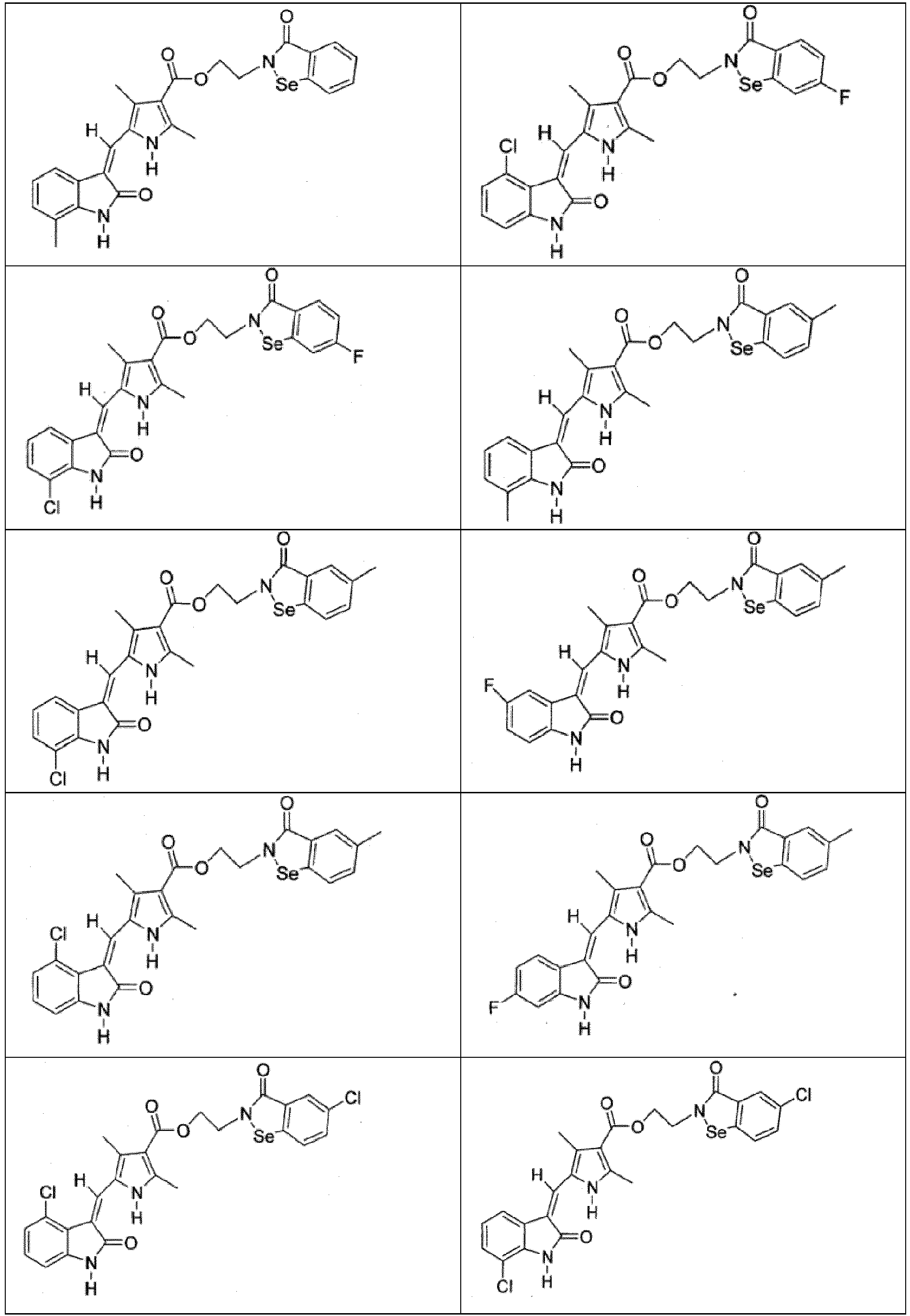

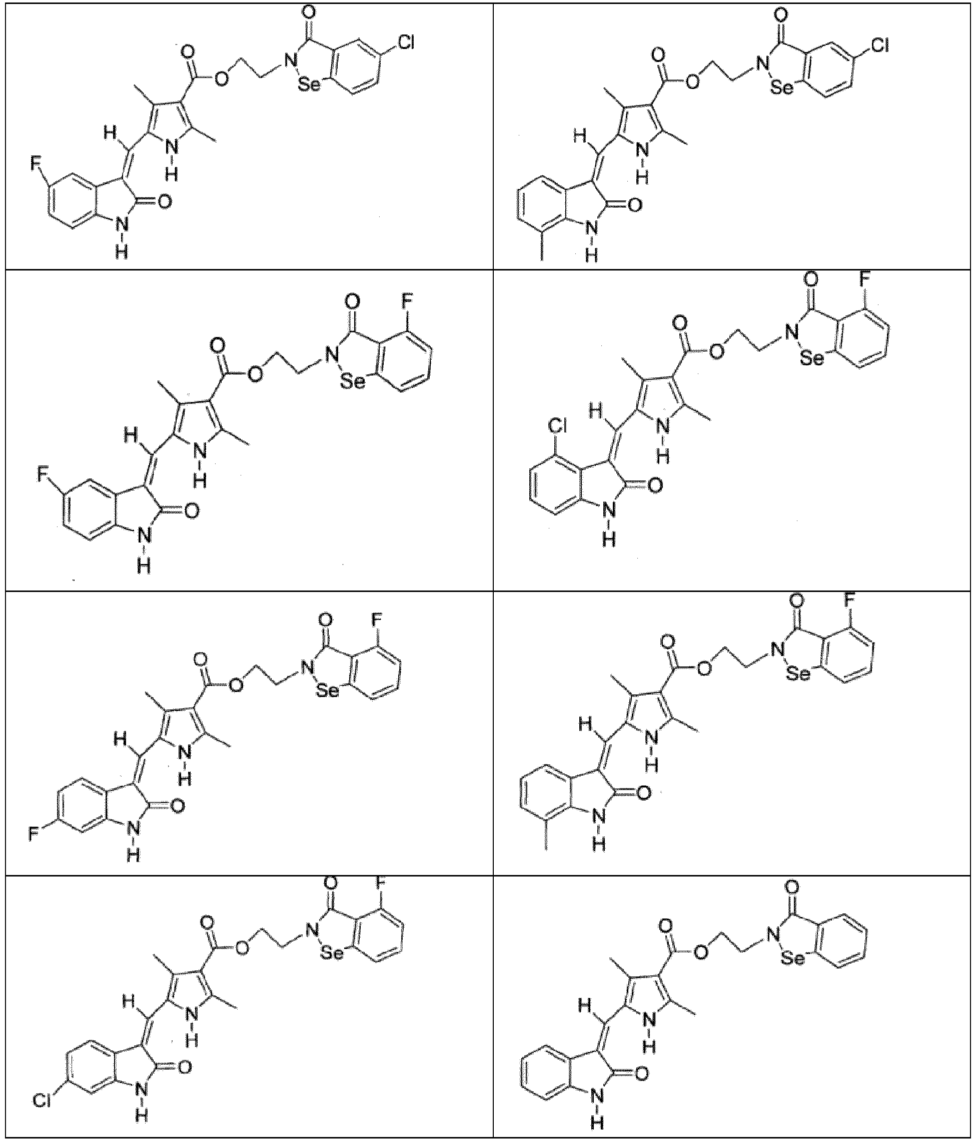

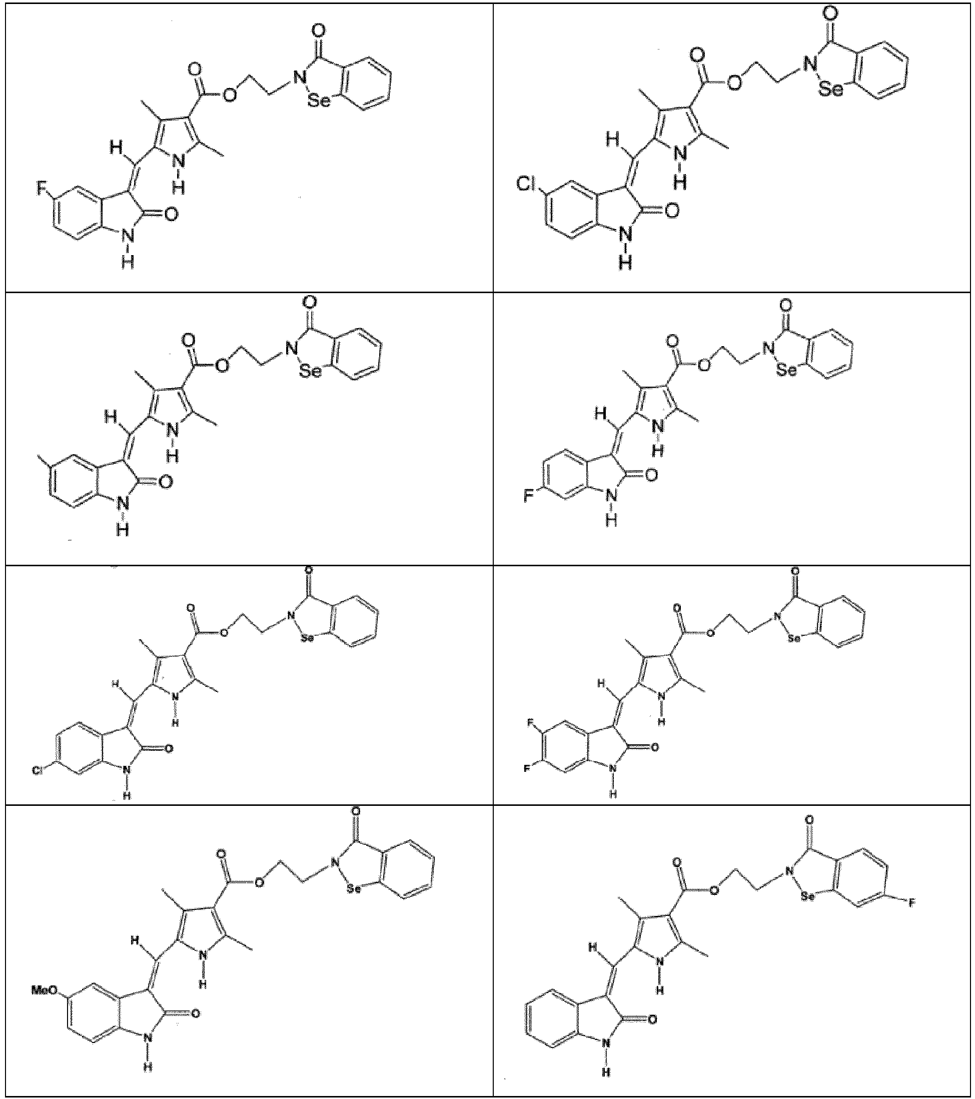

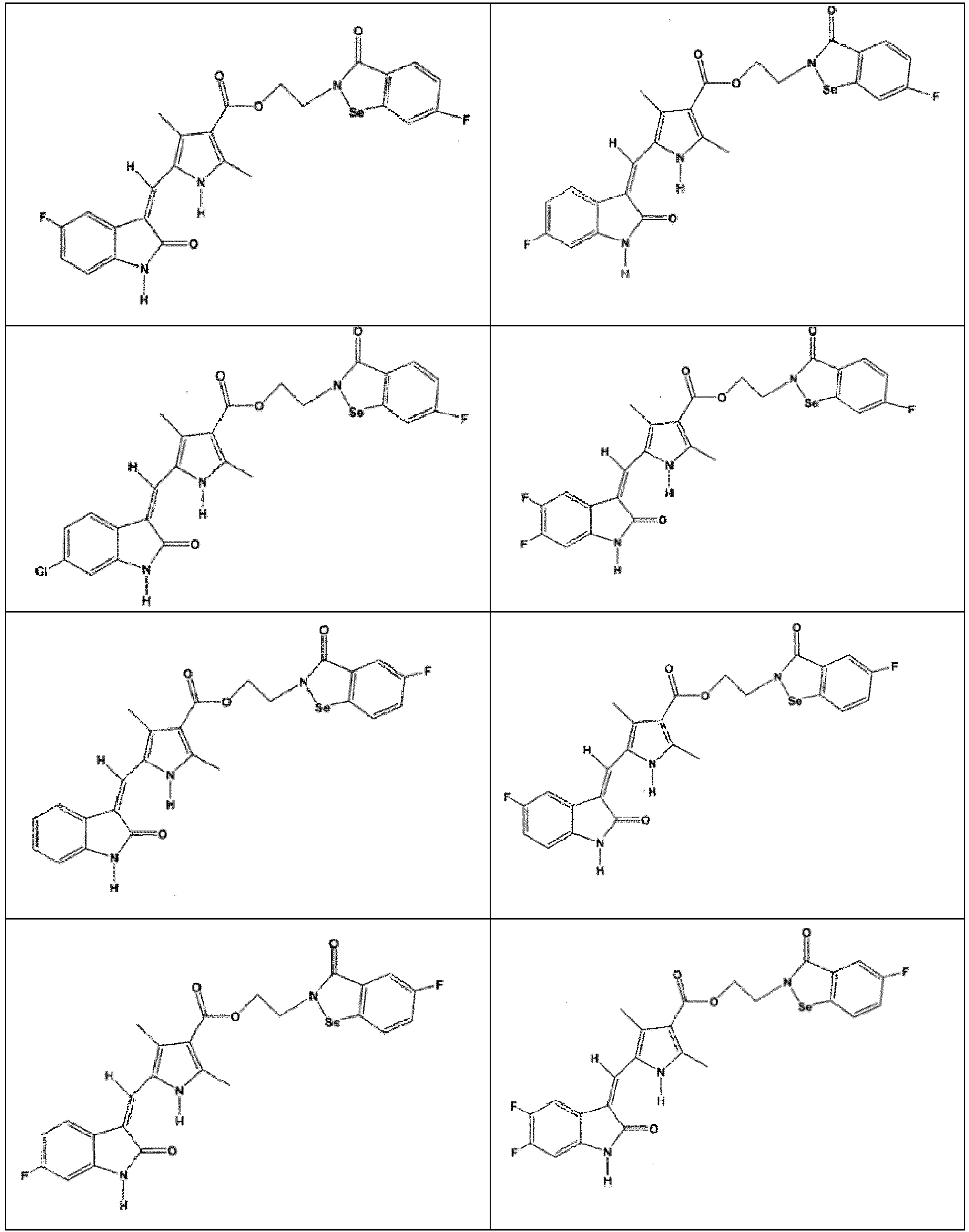

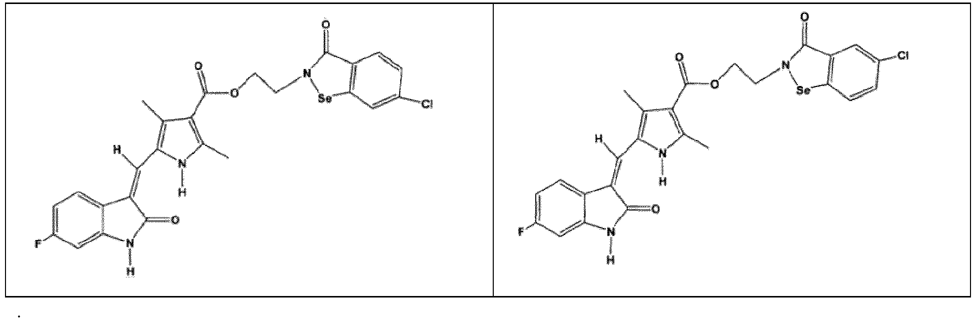

7. The use as claimed in claim 1 or 2, wherein the benzisoselenazole derivatives of formula (I) are compounds of the following formula (I-6): 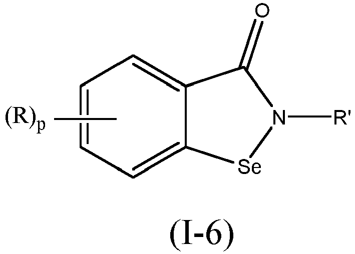 wherein Rand p each have the definitions given in the compounds of formula (I) as claimed in claim 1 or 2;
R' is selected from hydrogen, C₁₋₆ alkyl, C₃₋₇ cycloalkyl, C₁₋₆ alkyl-OH, R^{e}-NH-R^{f}, 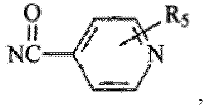 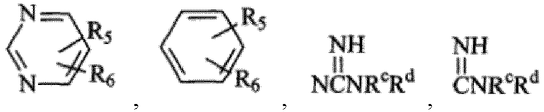 and saccharide residue;
R₅ and R₆ are each independently selected from hydrogen, halogen, cyano, C₁₋₆ alkyl, C₁₋₆ alkoxy, C₁₋₆ alkylthio, N(C₁₋₆ alkyl)₂, NH(C₁₋₆ alkyl), COOR^{b} and SO₃R^{b};
R^{b} is selected from hydrogen, C₁₋₆ alkyl and C₁₋₆ alkoxy;
R^{c} and R^{d} are each independently selected from hydrogen, C₁₋₆ alkyl and phenyl, or R^{c} and R^{d} together with a nitrogen atom to which they are linked form a nitrogen-containing heterocycle or nitrogen-containing heteroaryl ring;
R^{e} and R^{f} are each independently selected from C₁₋₆ alkyl and C₃₋₇ cycloalkyl;
preferably, the saccharide residue may be a *D*-glucopyranosyl group, such as 1,3,4,6-tetra-*O*-acetyl-2-deoxy-*D*-glucopyranosyl;
more preferably, the benzisoselenazole derivatives of formula (I-6) are selected from the compounds in Table 6 below:
**Table 6**
| | |
|---|---|
| 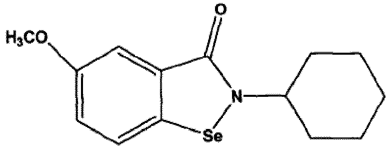 | 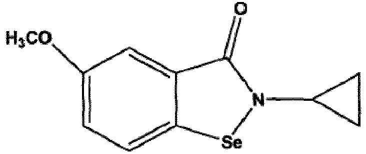 |
| 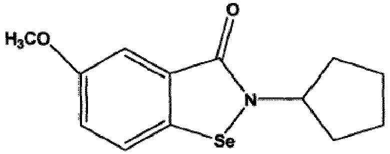 | 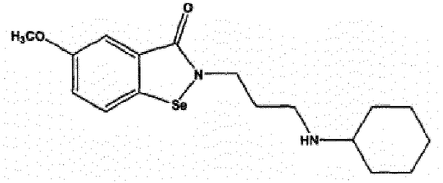 |
| 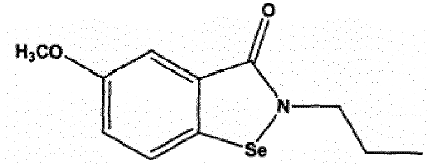 | 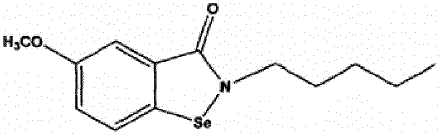 |
| 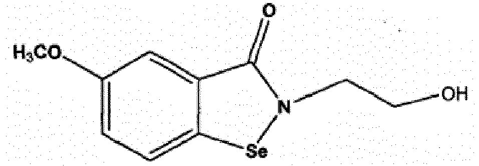 | 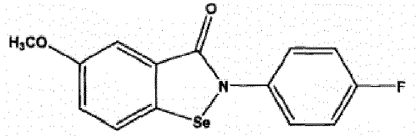 |
| 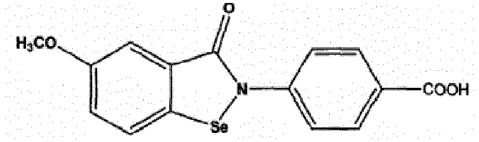 | |
| 4-{*N*-[1,2-Benzisoselenazol-3(2*H*)-one]formylhydrazine}-pyridine | 2-[1,2-Benzisoselenazol-3(2*H*)-one]-4,6-dimethylpyr imidine |
| 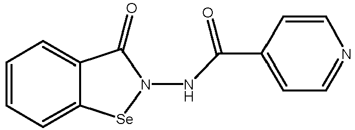 | 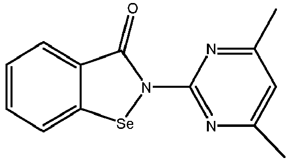 |
| 4-[1,2-Benzisoselenazol-3(2*H*)-one]-2-hydroxybenzoic acid | *N*-[1,2-Benzisoselenazol-3(2*H*)-one]-aminoguanidin e |
| 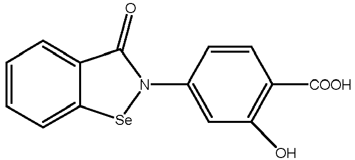 | 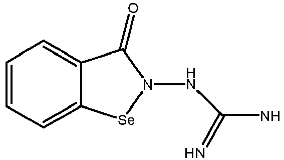 |
| *N*-[1,2-Benzisoselenazol-3(2*H*)-one]-guanidine | 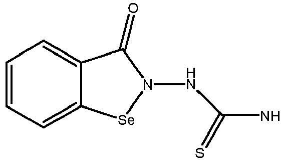 |
| 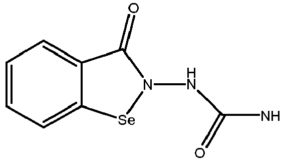 | |
| 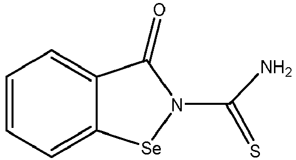 | 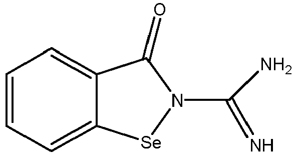 |
| 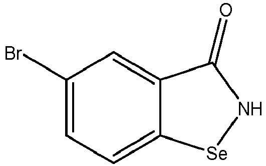 | 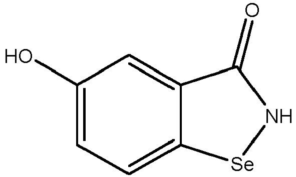 |
| 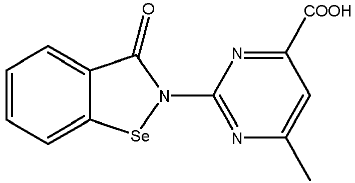 | |

8. Use of benzisoselenazole derivatives of formula (II) for the manufacturing of anti-coronavirus medicaments or medicaments for the treatment of diseases caused by coronaviruses: 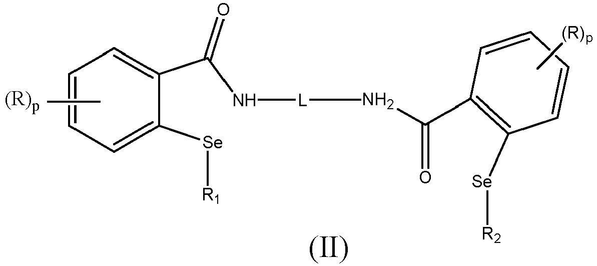 wherein R, p and L each have the definitions given in the compounds of formula (I) as claimed in claim 1 or 2; R₁ and R₂ are each independently selected from C₁₋₆ alkyl, C₁₋₆ alkoxy, C₁₋₆ alkylthio and wherein the S atom is linked to the Se atom by a single bond; 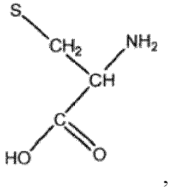 preferably, R₁ and R₂ are both 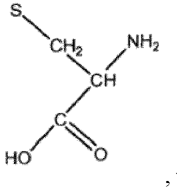 wherein the S atom is linked to the Se atom by a single bond; more preferably, the benzisoselenazole derivatives of formula (II) are selected from the compounds in Table 7 below:

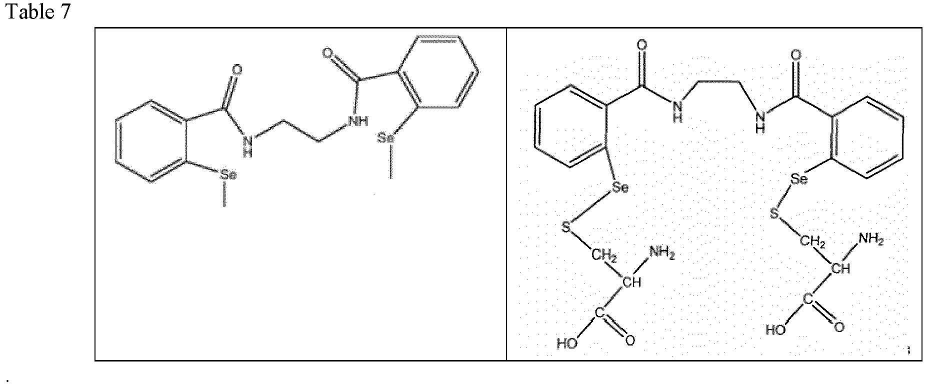

9. Use of benzisoselenazole derivatives of formula (III) for the manufacturing of anti-coronavirus medicaments or medicaments for the treatment of diseases caused by coronaviruses: 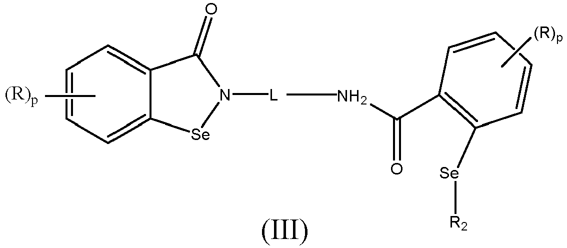 wherein R, p and L each have the definitions given in the compounds of formula (I) as claimed in claim 1 or 2; R₂ is selected from C₁₋₆ alkyl, C₁₋₆ alkoxy, C₁₋₆ alkylthio and 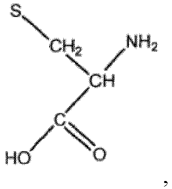 wherein the S atom is linked to the Se atom by a single bond;
preferably, the benzisoselenazole derivatives of formula (III) are the compounds in Table 8 below:

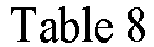

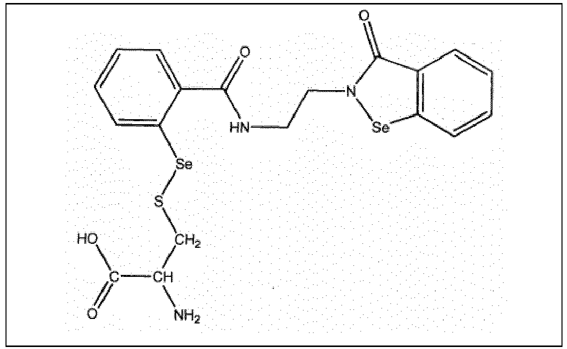

10. The use as claimed in any one of claims 1 to 9, wherein the diseases caused by coronaviruses include interstitial lung disease (ILD).
